(19) Europäisches Patentamt
European Patent Office
Office européen des brevets

(11) EP 0 842 174 B1

(12) FASCICULE DE BREVET EUROPEEN

(45) Date de publication et mention de la délivrance du brevet: **04.10.2001 Bulletin 2001/40**

(51) Int Cl.[7]: **C07D 417/12**, A61K 31/535, A61K 31/54, A61K 31/55, C07D 413/12

(21) Numéro de dépôt: **96927083.4**

(22) Date de dépôt: **25.07.1996**

(86) Numéro de dépôt international: **PCT/FR96/01176**

(87) Numéro de publication internationale: **WO 97/05134 (13.02.1997 Gazette 1997/08)**

(54) **COMPOSES HETEROCYCLIQUES POUR LE TRAITEMENT DE L'ISCHEMIE MYOCARDIQUE**

HETEROCYCLISCHE VERBINDUNGEN ZUR BEHANDLUNG VON MYOKARD-ISCHEMIA

HETEROCYCLIC COMPOUNDS FOR TREATING MYOCARDIAL ISCHAEMIA

(84) Etats contractants désignés: **AT BE CH DE DK ES FI FR GB GR IE IT LI LU MC NL PT SE**

(30) Priorité: **26.07.1995 FR 9509079**

(43) Date de publication de la demande: **20.05.1998 Bulletin 1998/21**

(73) Titulaire: **PIERRE FABRE MEDICAMENT 92100 Boulogne-Billancourt (FR)**

(72) Inventeurs:
- **RIEU, Jean-Pierre F-81100 Castres (FR)**
- **PATOISEAU, Jean-François F-81100 Castres (FR)**
- **JOHN, Gareth, W. F-81100 Castres (FR)**
- **LEGRAND, Bruno F-80440 Lautrec (FR)**
- **VALENTIN, Jean-Pierre F-31250 Revel (FR)**

(74) Mandataire: **Ahner, Francis et al Cabinet Régimbeau 20, rue de Chazelles 75847 Paris cedex 17 (FR)**

(56) Documents cités: **EP-A- 0 184 257**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

EP 0 842 174 B1

## Description

Les maladies coronariennes constituent la première cause dé décès dans le monde occidental. L'ischémie myocardique présente une éthiologie fort complexe et n'est traitée que par des médicaments agissant de manière indirecte. Les dérivés utilisés à l'heure actuelle dans l'angor et l'infarctus du myocarde sont les β-bloquants, les dérivés nitrés et les inhibiteurs calciques qui agissent tous indirectement par un phénomène hémodynamique.
Il est cependant admis que les métabolites produits très tôt dans l'ischémie (catabolisme phospholipidique et glycolyse) interfèrent sur l'inactivation du courant sodique. Celle-ci génère par l'intermédiaire de l'échangeur sodium-calcium dans le myocyte une surcharge calcique (Kohlhardt M. et al (1989) *FASEB* J. 3 p 1963 - 7 et Undrovinas A.I. et al (1992) *Circulation Research* 71 p 1231 - 41). Cette sucharge calcique finale induit la contracture du myocyte.
Récemment de nouveaux composés sans effet hémodynamique notable et agissant sur la surcharge sodique ont été revendiqués (Massingham R., John G. W. and Van Zwieten, *Drugs of Today* (1991) 27 (8), p 459 - 77) par les laboratoires Janssen (E. Boddeke et al, TIPS (1987) 10 p 397 - 400 ; Ver Donck L., Borgers M., Verdonk F., *Cardiovascular Research* (1993) 27 p 349 - 357, Brevet EP 0184257) et Syntex (Patmore L. et al. *Br J Pharmacol* (1991) 104 suppl.175 P ; Alps B. J., *Br J Clin Pharmacol* (1992) 34 199-206 et brevet US 4829065.

Dans le brevet EP 0184257 représentant l'état de la technique la plus proche les produits revendiqués sont constitués en partie de dérivés de N-(2-benzothiazolyl)-1-(phénoxyalcoyl)-4-pipéridinamine dont les produits leaders sont le R 56865 et le Sabeluzole de formule :

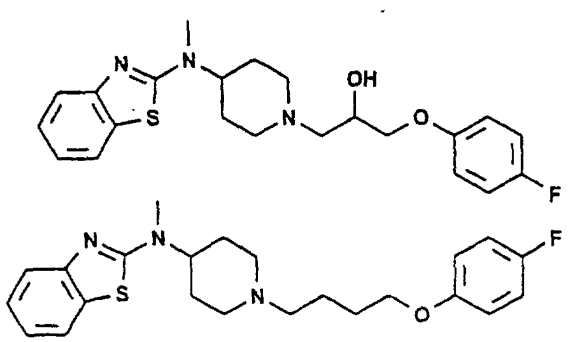

SABELUZOLE

R 56865

**MOLECULES REVENDIQUEES :**

Les molécules de la présente invention appartiennent à la classe des 1-(phénoxyalcoyl)-4-pipéridinamines N-substituées de **formule I :**

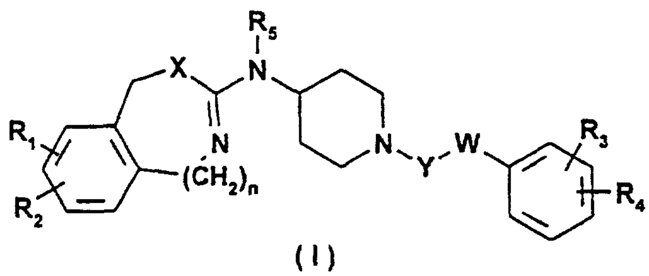

(I)

dans laquelle

$R_1$ à $R_4$ égaux ou différents représentent :

- un hydrogène
- un alcoyle ramifié ou non renfermant de 1 à 4 atomes de carbone
- un alcoyloxy ramifié ou non renfermant de 1 à 4 atomes de carbone
- un groupement halogène
- un groupement nitro
- un groupement hydroxy

- un groupement trifluorométhyle ou trifluorométhoxyle.

$R_5$ représente :

- un hydrogène
- un alcoyle ramifié ou non renfermant de 1 à 6 atomes de carbone
- un phénylalcoyle ramifié ou non renfermant de 7 à 12 carbones, pouvant être substitué sur l'aromatique par un ou deux radicaux définis comme $R_1$

W et X représentent :

- un oxygène ou un soufre

Y représente :

- un groupement $(CH_2)_m$ avec m est un entier compris entre 2 et 6 ou
- le radical - $CH_2$-CH(OH)-$CH_2$-

n peut prendre les valeurs 0 ou 1.

L'invention concerne aussi bien - lorsqu'ils existent - les isomères R ou S purs que leurs mélanges.
La présente invention inclut les sels minéraux ou organiques thérapeutiquement acceptables des composés de formule générale I et leurs hydrates éventuels.
L'invention concerne aussi le procédé de préparation des composés revendiqués ainsi que leur application comme médicaments.
La présente invention concerne encore un procédé de préparation des composés chimiques de formule la, c'est-à-dire les composés chimiques de formule I pour laquelle n=0, $R_5$=H et Y =$(CH_2)_m$,

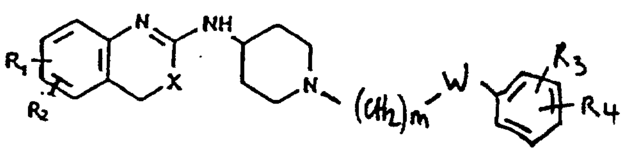

Ia

tel que l'on fait réagir un orthoaminobenzylalcool de formule VIII

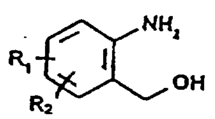

VIII

avec un 4-piperidinyl iso(thio)cyanate de formule IXa

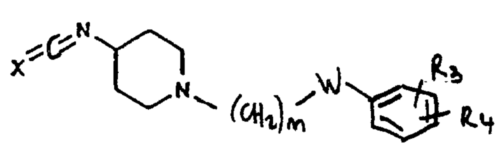

IXa

dans du THF ou du dioxane comme solvant à une température comprise entre 20 et 80° pour donner l'hydroxyméthyl (thio)urée intermédiaire Xa

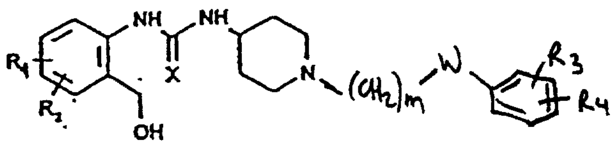

Xa

Xa est récupéré par filtration et cyclisé directement dans l'acide chlorhydrique concentré à une température comprise entre 20 et 80° pour donner le composé de formule générale la.

La présente invention concerne aussi un procédé de préparation des composés chimiques de formule Ib

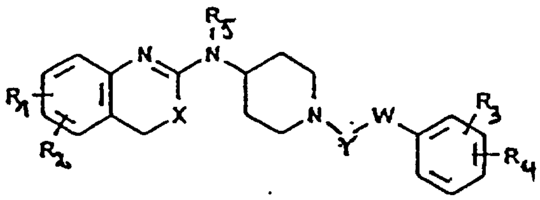

Ib

c'est-à-dire des composés chimiques de formule I où
n = 0, tel que l'on fait réagir un orthoaminoalcool de formule (VIII) avec un iso(thiocyanate) de formule (IXb)

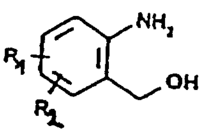

VIII

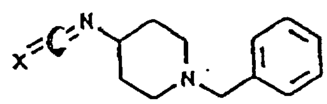

IXb

dans du THF ou du dioxane comme solvant à une température comprise entre 20 et 80° pour donner une hydroxyméthyl (thio)urée intermédiaire (Xb)

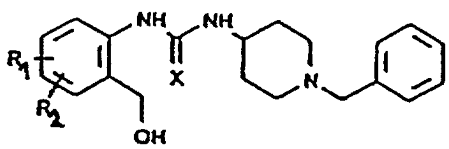

Xb

Xb qui peut ensuite être récupéré par filtration et qui est cyclisé directement par chauffage entre 20 et 70° dans l'acide chlorhydrique concentré pour donner le composé de formule XIb

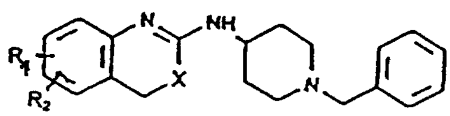

XIb

qui peut être alcoylé par un halogénure ($R_5$ - Br ou $R_5$ - I) ou un sulfate $(R_5)_2SO_4$ en présence de carbonate alcalin ou d'hydrure de sodium dans le DMF comme solvant pour donner le composé de formule (IVb),

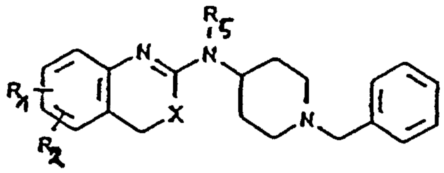

IVb

dont la N-débenzylation en présence de chloroformiate d'α-chloroéthyle dans le chlorure de méthylène suivi d'une hydrolyse au reflux du méthanol conduit à l'intermédiaire de formule (V)

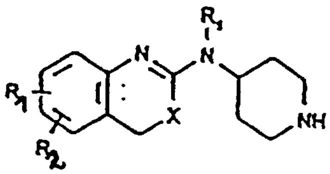

V

qui par N-alcoylation finale avec un halogénure de formule (VI) où Z représente un atome de brome, de chlore ou d'iode,

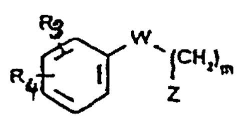

VI

dans le DMF en présence de $K_2CO_3$/KI 98/02 ou un glycidyléther de formule (VII) dans le méthanol donne le composé de formule générale Ib

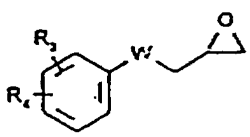

VII

En outre, l'invention a aussi trait à un procédé de préparation des composés chimiques de formule I où $R_5$ est différent de H tel que l'on fait agir un composé de formule I où $R_5$=H avec un halogénure d'(aryl)alcoyle $R_5$-Z $R_5$ et Z étant telsque définis ci-dessus, ou un sulfate d'alcoyle $(R_5)_2SO_4$, $R_5$ étant tel que défini ci-dessus, en présence d'une base comme NaH ou $Na_2CO_3$ pour donner un composé de formule I pour lequel R5 ≠ H est tel que défini précédemment.

Les molécules de la présente invention présentent des propriétés cytoprotectrices potentiellement remarquables au niveau cardiaque ou neuronal et généralement supérieures à celles des témoins le R 56865 et le sabeluzole précédemment cités.

**<u>SYNTHESE DES COMPOSES DE FORMULE I :</u>**

Selon la taille des cycles on peut envisager une ou plusieurs approches pour la synthèse des composés I.

**A) <u>Synthèse des dérivés à 6 chaînons</u> :**

Deux voies d'accès sont utilisées pour préparer les benzo(xa ou thia)zines : on peut partir en effet des 2-(halométhyl)phényliso(thio)cyanates substitués ou des 4-pipéridinyl iso(thio)cyanates - substitués en position 1.

1°) Préparation à partir des 2-(bromo ou chloro méthyl)phényliso(thio)cyanates : (cf. Schéma de synthèse **I**)

Ces composés **(II)** sont préparés par halogénation radicalaire substituante des o-tolyliso (thio)cyanates convenablement substitués (cf. J. Gonda et P. Kristian *Collect Czech Chem Commun* (1986) 51 p 2802-9 ; J. Gonda et M. Barnikol *Collect Czech Chem Commun* (1990) 55 p 752-60 et E. Klauke et L. Oehlmann, *Synthesis* (1978) p 376-7). Les 4-pipéridinamines **(III)** utilisées peuvent être substituées en 1 soit par le radical ($R_3$,$R_4$-$C_6H_3$-W-Y-) de la présente invention (cf. A.M. Ismaiel et col. *J Med Chem* (1993) 36 p 2519-25) soit protégées sous forme de dérivé benzylé (G. C. Caïn *Bioorg Med Chem Letters* (1994), 4, p 329-34) ou de carbamate d'éthyle. La condensation entre les 2 molécules **(II, III)** est réalisée selon Gonda et P. Kristian ; Gonda et Barnikol déjà cités ou W. Gauss et H.J. Kabbe, *Synthesis* (1978) p 377-9 et conduit :

- soit au dérivé attendu **I**
- soit à la pipéridine bloquée **(IV)** dont le déblocage est réalisé selon Bingwei V. Yang et col, *Synlett* (1993) p 195-6, ou par hydrolyse acide pour donner l'amine libre **(V).**

Celle-ci peut être condensée soit avec l'ω-bromo alcoyl phénylether **(IV)** (préparé selon A.M. Ismaiel et col déjà cité) ou sur un arylglycidyl éther **(VII)** (préparé selon W.S. Di Menna et col, *J Med Chem* (1978) 21 p 1073-1076 pour donner le dérivé I attendu convenablement substitué.

SCHEMA DE SYNTHESE I :

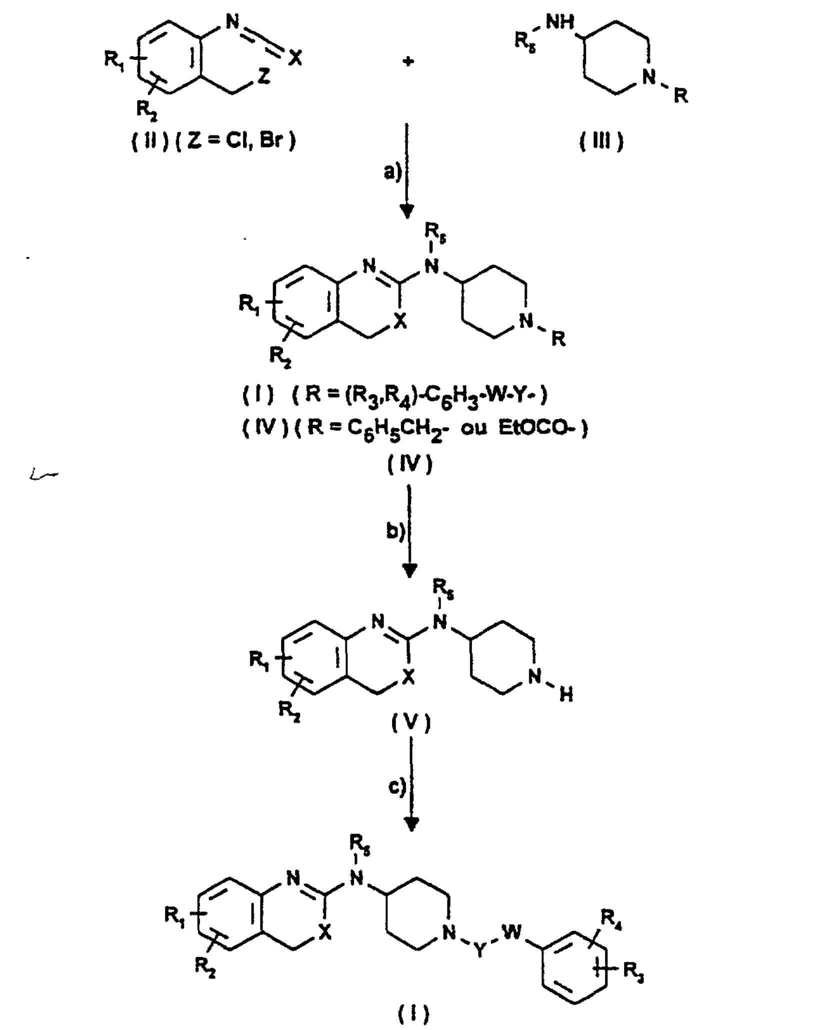


REACTIFS : a): $a_1$)X=S :$CH_2Cl_2$ , $Et_3N$ ou $a_2$)X=O :$CH_2Cl_2$ ; NaH-DMF - 0° ;
b): $b_1$) R = $C_6H_5CH_2-$ : $CH_3CH(Cl)COCl$ , $CH_2Cl_2$ ; MeOH reflux ou
$b_2$) R = COOEt : HCl 6N reflux ;
c): $c_1$) $(R_3R_4)-C_6H_3-W-(CH_2)_mBr$ ( VI ) , DMF , $K_2CO_3$/KI ou
$c_2$) $(R_3R_4)-C_6H_3-W-CH_2-CH-CH_2$ ( VII ) , MeOH.

2°) Préparation à partir des 4-pipéridinyliso(thio)cyanates-1-substitués **(IX)** : (Schéma de synthèse **II**).

Lorsque les Iso(thio)cyanates **(II)** sont difficiles à préparer, il est préférable de partir des 4-pipéridinyliso(thio) cyanates **(IX).** Ces iso(thio)cyanates IX utilisés sont préparés à partir des amines III précédemment décrites soit à l'aide du triphosgène (H. Eckert and B. Foster, *Angew Chem Int Ed English* (1987) 26 p 894-5) soit du di-2-pyridyl thiocarbonate (S. Kim, K.Y. Yi *Tetrahedron Letters* (1985) 26 p 1661-4) ou du N,N' thiocarbonyldiimidazole (Staab H. A. et G. Walther, *Ann* (1962) 657 104-107).

SCHEMA DE SYNTHESE II :

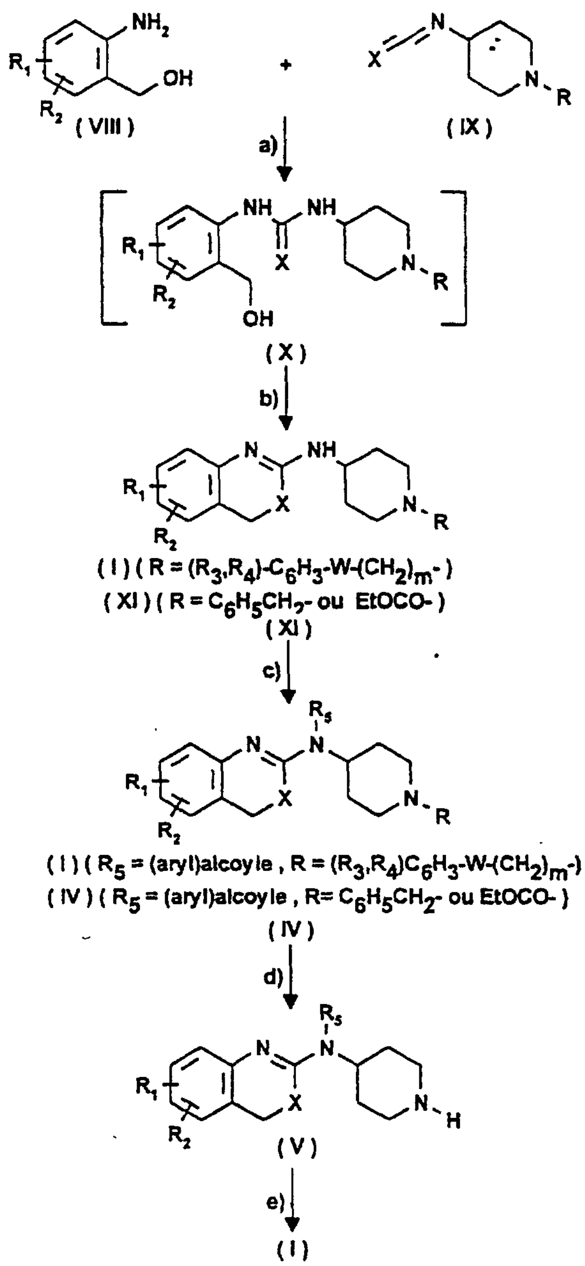


(VIII) + (IX)

(X)

(I) (R = $(R_3,R_4)$-$C_6H_3$-W-$(CH_2)_m$-)
(XI) (R = $C_6H_5CH_2$- ou EtOCO-)
(XI)

(I) ($R_5$ = (aryl)alcoyle , R = $(R_3,R_4)C_6H_3$-W-$(CH_2)_m$-)
(IV) ($R_5$ = (aryl)alcoyle , R= $C_6H_5CH_2$- ou EtOCO-)
(IV)

(V)

(I)

REACTIFS : a) : Dioxane ou THF ;
b) : HCl cé. ; ou Réaction de Mitsunobu ;
c) : DMF , NaH ou $K_2CO_3$ , $R_5$-I ou $R_5$-Br ;
d) : Cf. $b_1$ ou $b_2$ Schéma I ;
e) : Cf. $c_1$ ou $c_2$ Schéma I .

Ces iso(thio)cyanates sont condensés avec les alcools 2-aminobenzyliques **(VIII)** pour donner dans le dioxane au reflux des o-hydroxyméthylphénylurées intermédiaires **(X)** qui, sans être isolées, sont cyclisées dans l'acide chlorhydrique concentré en dérivé attendu **(I)** avec $R_5$ = H ou sous forme de dérivé **(XI)** N - protégé par des groupements benzyle ou éthyl carboxylate. La N-alcoylation après sodation par NaH ou $Na_2CO_3$ dans le DMF donne les composés **(I)** et **(IV)** avec $R_5$ = (ar)alcoyle. La déprotection de **(IV)** est ensuite réalisée comme dans le schéma I pour donner finalement après N - alcoylation les composés (I) de la présente invention convenablement substitués.

**B) <u>Synthèse des composés à 7 chaînons :</u>** (cf. schéma de synthèse III)

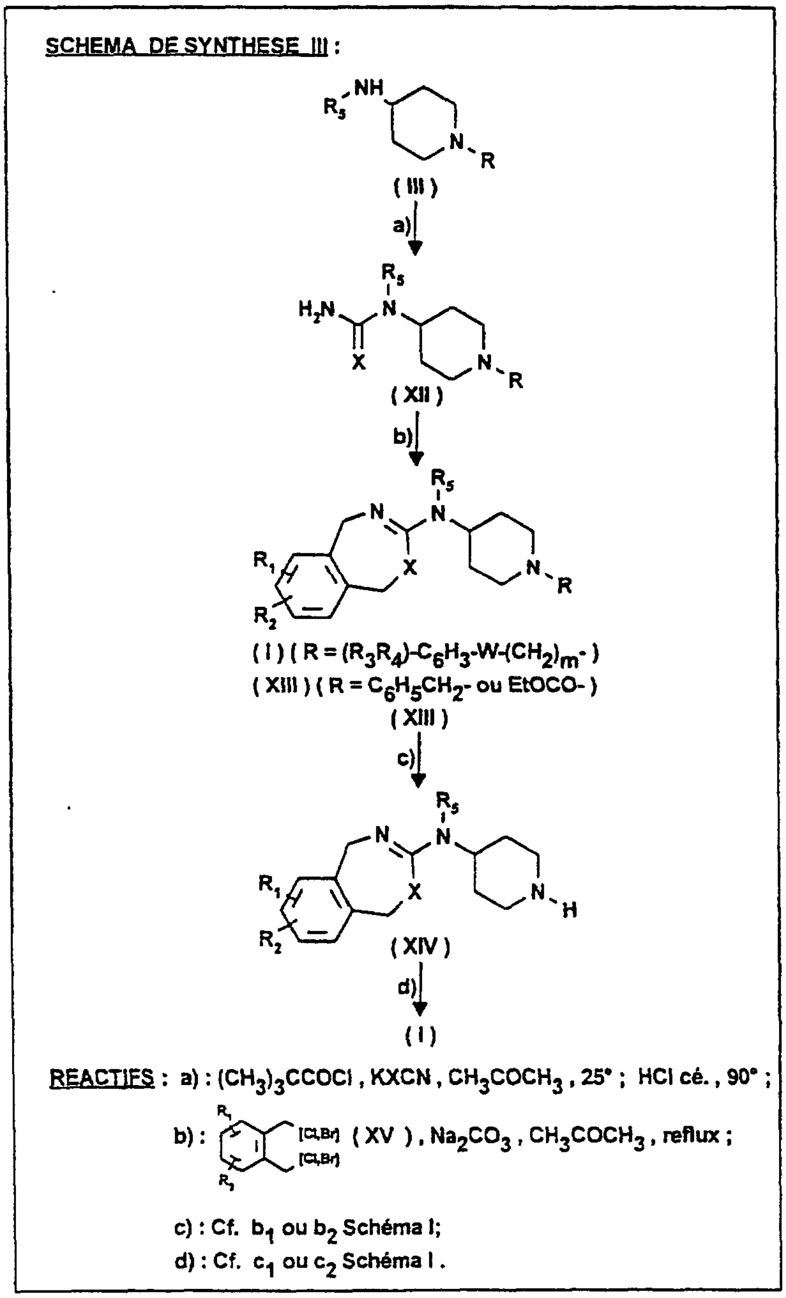


La diamine secondaire **(III)** est transformée en (thio)urée N-N-disubstituée **(XII)** par action de l'iso(thio)cyanate de sodium en présence de chlorure de pivaloyle selon le procédé décrit dans le brevet EP 126 934.

La pivaloylurée obtenue n'est pas isolée mais hydrolysée en présence de HCl au reflux, en urée **(XII).**

Cette (thio) urée est ensuite condensée avec un $\alpha,\alpha'$ dihalogénure d'o-xylène selon la méthode de D.N. Reinhoudt *(Recueil Trav Chim Pays Bas* (1973) <u>92</u> p 20-32) pour donner les 2-4-(thia ou oxa) zépines revendiquées (**I**) ou les composés N-protégés de formule **(XIII)** qui donnent par déprotection (selon le schéma I) le composé **(XIV)** qui est N-alcoylé en dérivé attendu **I** (n = 1).

**Exemple 1 :**

**Hydrogénomaléate de la N-méthyl-N-[1-[4-(4-fluorophénoxy)butyl]pipéridin-4-yl]-4H- 3,1-benzothiazin-2-amine (1).**

Une solution de 500 mg (2,19 mmoles) d'orthobromométhylphényl isothiocyanate (F = 42°), est traitée avec 900 mg (2,19 mmoles) de dichlorhydrate de 1-(4-(4-fluorophénoxy)butyl)-N-méthyl-4-pipéridinamine (F = 216°) et 1,2 ml (8,8 mmoles) de triéthylamine. Le mélange est ensuite porté à 80° sous courant d'azote pendant une heure. Après retour à 25° l'insoluble est éliminé, rincé avec du toluène et les eaux-mères sont évaporées à siccité au rotavapor sous vide. Le résidu est repris dans du chlorure de méthylène, lavé à l'eau, à l'eau salée puis la phase organique est séchée sur sulfate de sodium. L'huile jaune pâle obtenue après filtration est évaporée à siccité, purifiée par "flash chromatographie" en éluant avec un mélange $CH_2Cl_2$ - 97,5 / MeOH-2,25 / $NH_4OH$-0,25 pour donner après évaporation (m = 810 mg, Rdt : 86 %) d'huile crème qui cristallise à la longue.
Une solution de 220 mg d'acide maléique dans de l'acétate d'éthyle chaud est ajoutée à une solution de la base précédente dans le même solvant, puis on laisse cristalliser lentement à 25° et on filtre les cristaux de sel formé (m = 900 mg ; Rdt : 72 %). On recristallise dans l'alcool éthylique pour obtenir 675 mg (Rdt : 54 %) de cristaux blancs de **formule 1 :**

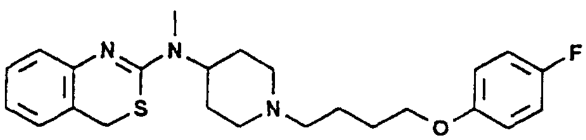 , 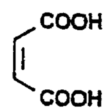 (1)

Formule brute : $C_{28}H_{34}FN_3O_5S$
Masse moléculaire : 543,63
Point de fusion : 139 - 140°
**IR** (KBr) : $\upsilon$ (C = N) : 1618 ; ($COO^-$) : 1586 $cm^{-1}$.
**RMN** (DMSO $d_6$) $\delta$ : 1.6 - 2.3 (m, 8H) ; 3,01 (s, 3H) ; 3,1 - 3,8 (m, 6H) ; 3,95 (s, 2H) ; 3,8 - 4,2 (m, 2H) ; 4,5 - 4,9 (m, 1H) ; 6,02 (s, 2H) ; 6,8- 7,05 (m, 4H) ; 7,05 - 7,3 (m, 4H) ; 8 - 11 (m, 2H).

**Exemple 2 :**

**Hydrogénomaléate de la N-méthyl-N-[1-[3-(4-fluorophénoxy) propyl] pipéridin-4-yl]-4H-3,1-benzothiazin-2-amine (2).**

En condensant 500 mg (2,19 mmoles) d'orthobromométhyl-phénylisothiocyanate sur 745 mg (2,19 mmoles) de dichlorhydrate de N-méthyl-1-[4-(4-fluorophénoxy)propyl]-4-pipéridinamine (F > 220°) selon le procédé décrit dans l'exemple 1 on obtient avec un rendement de 58 % des cristaux blancs de **formule 2 :**

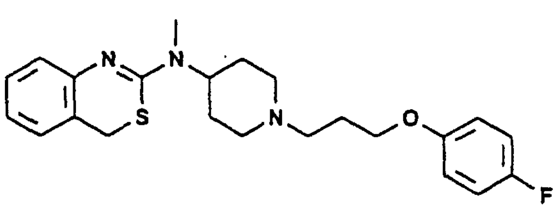 , 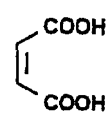 (2)

Formule brute : $C_{27}H_{32}FN_3O_5S$
Masse moléculaire : 529,606
Point de fusion : 160 - 161°
**IR** (KBr) : $\upsilon$ (N = C) : 1610 ; ($COO^-$) : 1540 $cm^{-1}$.
**RMN** (DMSO $d_6$) $\delta$ : 1,7 - 2,3 (m, 6H) ; 3,02 (s, 3H) ; 2,9 - 3,4 (m, 4H) ; 3,5 - 3,8 (m, 2H) ; 3,96 (s, 2H) ; 4,03 (t, 2H) ; 4,5 - 4,8 (m, 1H) ; 6,03 (s, 2H) ; 6,8 - 7,05 (m, 4H) ; 7,05 - 7,35 (m, 4H) ; 8,5 - 11 (m, 2H).

**Exemple 3 :**

**Hydrogénomaléate de la N-méthyl-N-[1-[2-(4-fluorophénoxy)éthyl]pipéridin-4-yl]-4H-3,1-benzothiazin-2-amine (3).**

En opérant comme décrit dans l'exemple 1 mais en partant de 856 mg de N-méthyl-[1-[2-(4-fluorophénoxy) éthyl)-4-pipéridinamine dichlorhydrate (F = 241°C) on obtient m = 910 mg (Rdt : 68 %) de cristaux blancs de **structure 3 :**

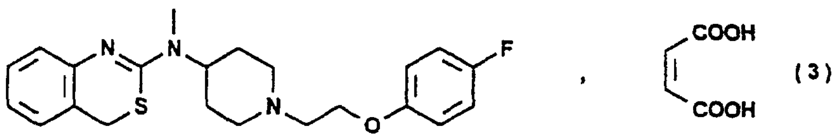

Formule brute : $C_{26}H_{30}FN_3O_5S$
Masse moléculaire : 515,586
Point de fusion : 114 - 115°
**IR** (KBr) : υ (N = C) : 1618 ; ($COO^-$) : 1562 $cm^{-1}$.
**RMN** ($CDCl_3$) δ : 2,01 (d, 2H) ; 2,3 (q.d, 2H) ; 2,85 - 3,2 (m, 2H) ; 3,11 (s, 3H) ; 3,44 (t, 2H) ; 3,45 -3,9 (m, 2H) ; 3,89 (s, 2H) ; 4,33 (t, 2H) ; 4,8 - 5,2 (m, 1H) ; 6,3 (s, 2H) ; 6,75 - 6,92 (m, 2H) ; 6,95 - 7,15 (m, 5H) ; 7,2 - 7,35 (m, 1H) ; 8,5 - 11,5 (m, 2H).

**Exemple 4 :**

**Hydrogénomaléate de la N-[1-[4-(4-fluorophénoxy)butyl]pipéridin-4-yl]-4H-3,1-benzothiazin-2-amine (4).**

En utilisant le mode opératoire décrit dans l'exemple 1 et en partant de 744 mg (2,19 mmoles) de dichlorhydrate de 1-[4-(4-fluorophénoxy)butyl]-4-pipéridinamine (F = 248°) on prépare 740 mg de base attendue (F = 134°) qui est ensuite salifiée avec l'acide maléique pour donner 880 mg (Rdt : 76 %) de cristaux blancs de **formule 4 :**

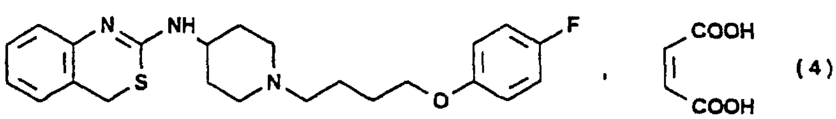

Formule brute : $C_{27}H_{32}FN_3O_5S$
Masse moléculaire : 529,61
Point de fusion : 131 - 132°
**IR** (KBr) : υ (N = H) : 3240 ; ($COO^-$) : 1560 $cm^{-1}$.
**RMN** (DMSO $d_6$) δ : 1,5 -1,9 (m, 6H) ; 1,9 - 2,4 (m, 2H) ; 2,9 - 3,75 (m, 6H) ; 3,94 (s, 2H) ; 3,99 (t, 2H) ; 3,9 - 4,5 (m, 1H) ; 6,05 (s, 2H) ; 6,8 - 7,05 (m, 4H) ; 7,05 - 7,3 (m, 4H) ; 7,3 - 7,7 (m, 1H) ; 8,7 - 10,8 (m, 2H).

**Exemple 5 :**

**Monohydrate de l'hydrogénomaléate de la N-[1-[3-(4-fluorophénoxy)propyl]pipéridin-4-yl]-4H-3,1-benzothiazin-2-amine (5).**

En utilisant 713 mg (2,19 mmoles) de dichlorhydrate de 1-[3-(4-fluorophénoxy)propyl]-4-pipéridinamine (F = 275 - 78°) et en les condensant sur 500 mg (2,19 mmoles) d'ortho bromométhyl phénylisothiocyanate selon le procédé décrit dans **l'exemple 1** on prépare 560 mg (Rdt 48 %) de cristaux blancs de **formule 5 :**

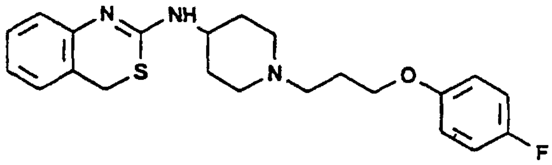 , 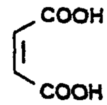 (5)

, $H_2O$

Formule brute : $C_{26}H_{32}FN_3O_6S$
Masse moléculaire : 533,602
Point de fusion : 98 - 99°
**IR** (KBr) : υ (OH) : 3429 ; (N-H) : 3256 ; ($COO^-$) : 1560 $cm^{-1}$.
**RMN** (DMSO $d_6$) δ : 1,4 - 2,4 (m, 6H) ; 2,7 - 3,2 (m, 9H) ; 3,91 (s, 2H) ; 4,02 (t, 2H) ; 3,9 - 4,5 (m, 1H) ; 6,02 (s, 2H) ; 6,7 - 7 (m, 4H) ; 7 - 7,2 (m, 4H) ; 7,2 - 7,7 (m, 1H) ; 8,6 - 9,6 (m, 1H).

**Exemple 6 :**

**Hydrogénofumarate de la N-éthyl-N-[1-[3-(4-fluoro-phénoxy)propyl]pipéridin-4-yl]-4H-3,1-benzothiazin-2-amine (6).**

En adaptant le procédé décrit dans l'exemple 1 au dichlorhydrate de N-éthyl-N-[1-[3-(4-fluorophénoxy) propyl]-4-pipéridinamine (F = 284°) on obtient 390 mg (Rdt : 33 %) de cristaux beiges de **formule 6 :**

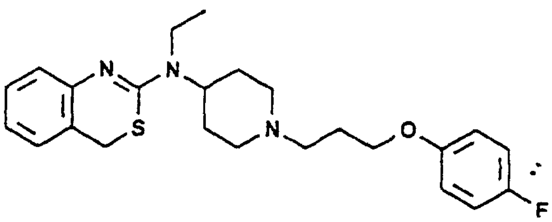 , 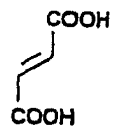 (6)

Formule brute : $C_{28}H_{34}FN_3O_5S$
Masse moléculaire : 543,636
Point de fusion : 140 - 141°
**IR** (KBr) : υ ($COO^-$) : 1552 $cm^{-1}$.
**RMN** (DMSO $d_6$) δ : 1,26 (t, 3H) ; 1,6 - 1,9 (m, 2H) ; 2 - 4 (m, 4H) ; 2,52 (t, 2H) ; 2,91 (t, 2H) ; 3,2 - 3,6 (m, 4H) ; 3,74 (s, 2H) ; 3,91 (t, 2H) ; 4,2 - 5,2 (m, 3H) ; 6,7 (s, 2H) ; 6,7 - 7,3 (m, 8H).

**Exemple 7 :**

**Hydrogénomaléate de la N-benzyl-N-[1-[3-(4-fluorophénoxy)propyl]pipéridin-4-yl]-4H-3,1-benzothiazin-2-amine (7).**

En utilisant le dichlorhydrate de N-benzyl-1-[3-(4-fluorophénoxy)propyl]-4-pipéridinamine (F = 280°) (910 mg, 2,19 mmoles) comme matière première et la faisant réagir selon le procédé de l'**exemple 1** on obtient 710 mg (Rdt : 53 %) de cristaux blancs de **structure 7** :

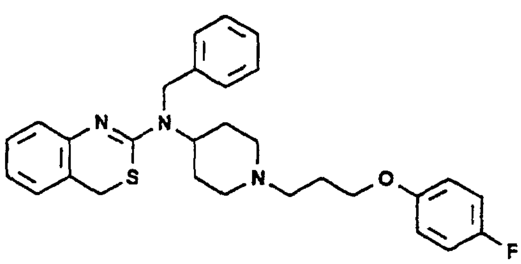 , 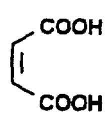 (7)

Formule brute : $C_{33}H_{36}FN_3O_5S$
Masse moléculaire : 605,706
Point de fusion : 114 - 116°
**IR** (KBr) : υ (N = C) : 1601 ; ($COO^-$): 1556 $cm^{-1}$.
**RMN** (DMSO $d_6$) δ : 1,8 - 2,1 (m, 2H) ; 2,1 - 2,6 (m, 4H) ; 2,83 (t, 2H) ; 3,18 (t, 2H) ; 3,4 - 3,7 (m, 2H) ; 3,85 (s, 2H) ; 3,98 (t, 2H) ; 4,80 (s, 2H) ; 4,8 - 5,2 (m, 1H) ; 6,25 (s, 2H) ; 6,65 - 6,85 (m, 2H) ; 6,85 - 7,15 (m, 5H) ; 7,15 - 7,4 (m, 6H) ; 11,5 - 13,5 (m, 2H).

**Exemple 8 :**

**Hydrogénomaléate de la 6-fluoro-N-méthyl-N-[1-[4-(4-fluorophénoxy)butyl]pipé ridin-4-yl]-4H-3,1-benzothiazin-2-amine (8).**

En partant du 4-fluoro-2-méthylphénylisothiocyanate et en utilisant le procédé de Gonda J. et Kristian P. (*Collect Czech Chem Commun* 1990, 55, 752-60) on prépare avec un rendement de 56 % le 4-fluoro-2-bromométhylphénylisothiocyanate (F < 40°). Ce dérivé condensé dans les mêmes conditions que décrit dans l'exemple 1 permet de préparer 650 mg (Rdt : 76 %) de base attendue (F = 113°) qui est salifiée pour donner 770 mg (Rdt : 72 %) de cristaux blancs de **formule 8 :**

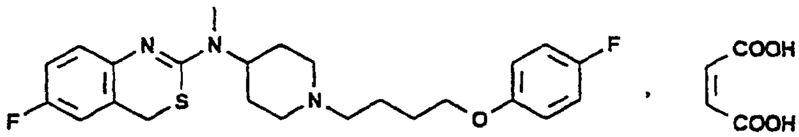

(8)

Formule brute : $C_{28}H_{33}F_2N_3O_5S$
Masse moléculaire : 561,63
Point de fusion: 148 - 149°
**IR** (KBr) : υ (N = C) : 1614 ; ($COO^-$): 1570 $cm^{-1}$.
**RMN** ($CDCl_3$) δ 1,7 - 2,15 (m, 6H) ; 2,34 (q.d., 2H) ; 2,3 - 3,4 (m, 4H) ; 3,07 (s, 3H) ; 3,5 - 3,8 (m, 2H) ; 3,86 (s, 2H) ; 3,91 (t, 2H) ; 4,8 - 5,2 (m, 1H) ; 6,26 (s, 2H) ; 6,45 - 6,8 (m, 2H) ; 6,9 - 7,15 (m, 4H) ; 7,15 - 7,3 (m, 1H) ; 10,5 -14 (m, 2H).

**Exemple 9 :**

**Hydrogénomaléate de la 6-chloro-N-méthyl-N-[1-[3-(4-fluorophénoxy)propyl]pipéridin-4-yl]-4H-3,1-benzothiazin-2-amine (9).**

Selon le procédé précisé dans **l'exemple 8** on prépare avec un rendement de 70 % le 4-chloro-2-bromométhyl-phényl-isothiocyanate (F = 65°) qui est condensé selon le mode opératoire décrit dans **l'exemple 1** pour donner 710 mg de base attendue (F = 125°) qui est salifiée par l'acide maléique pour donner 820 mg (Rdt : 81 %) de cristaux blanc cassé de **formule 9 :**

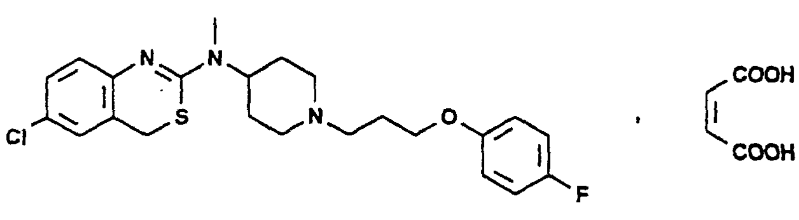

(9)

Formule brute : $C_{27}H_{31}ClFN_3O_5S$
Masse moléculaire : 564,06
Point de fusion : 155 - 156°
**IR** (KBr) : υ (N = C) : 1607 ; ($COO^-$): 1558 $cm^{-1}$.
**RMN** (DMSO $d_6$) δ : 1,7 - 2,3 (m, 6H) ; 3,01 (s, 3H) ; 2,9 - 3,5 (m, 4H) ; 3,5 - 3,75 (m, 2H) ; 3,96 (s, 2H) ; 4,01 (t,

2H) ; 4,45 - 4,8 (m, 1H) ; 6,01 (s, 2H) ; 6,8 - 7 (m, 3H) ; 7,05-7,4 (m, 4H) ; 8,5 - 11 (m, 2H).

**Exemple 10 ;**

**Dihydrogénomaléate de la 6-méthoxy-N-méthyl-N-[1-[3-(4-fluorophénoxy)propyl] pipéridin-4-yl]-4H-3,1-benzothiazin-2-amine (10).**

En condensant 568 mg (2,20 mmoles) de 4-méthoxy-2-bromométhylphényl isothiocyanate préparés selon **l'exemple 8** et en les condensant sur le dichlorhydrate de N-méthyl-1-[3-(4-fluorophénoxy)propyl]-4-pipéridinamine (748 mg) d'après le procédé décrit dans **l'exemple 1** mais en doublant la quantité d'acide maléïque on prépare 615 mg (Rdt : 41 %) de cristaux blancs de **formule 10 :**

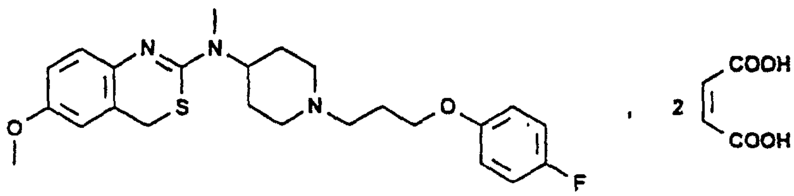

(10)

Formule brute : $C_{32}H_{38}FN_3O_{10}S$
Masse moléculaire : 675,708
Point de fusion : 136 - 137°
**IR** (KBr) : υ (N = C) : 1607 ; ($COO^-$) : 1577 $cm^{-1}$.
**RMN** ($CDCl_3$) δ : 1,7 - 2,3 (m, 6H) ; 3 (s, 3H) ; 2,9 - 3,4 (m, 4H) ; 3,5 - 3,8 (m, 2H) ; 3,71 (s, 3H) ; 3,96 (s, 2H) ; 4,04 (s, 2H) ; 4,45 - 4,75 (m, 1H) ; 6,14 (s, 4H) ; 6,75 - 6,85 (m, 2H) ; 6,85 - 7,05 (m, 3H) ; 7,1 - 7,3 (m, 2H) ; 8,5 - 12,5 (m, 4H).

**Exemple 11 :**

**Hydrogénomaléate de la N-méthyl-N-[1-[4-(3,4-difluorophénoxy)butyl]pipéridin-4yl]-4H-3,1-benzothiazin-2-amine (11).**

En partant de 654 mg (2,19 mmoles) de N-méthyl-1-[4-(3,4-difluorophénoxy)butyl]-4-pipéridinamine base préparée selon A.M. Ismaiel et col, *J Med Chem* (1993) 36 p 2519-2525 et en les condensant avec 500 mg (2,19 mmoles) d'orthobromométhyl phénylisothiocyanate selon le procédé décrit dans **l'exemple 1,** on prépare avec un rendement de 66 % des cristaux blancs de **formule 11 :**

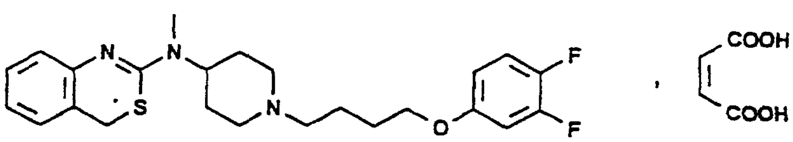

(11)

Formule brute : $C_{28}H_{33}F_2N_3O_5S$
Masse moléculaire : 561,626
Point de fusion : 130 - 131°
**IR** (KBr) : υ (C =N) : 1606 ; ($COO^-$) : 1564 $cm^{-1}$.
**RMN** (DMSO $d_6$) δ : 1,5 - 2,3 (m, 8H) ; 3,03 (s, 3H) ; 2,8 - 3,35 (m, 4H) ; 3,4 - 4,2 (m, 2H) ; 3,97 (s, 2H) ; 4,01 (t, 2H) ; 4,55 - 4,85 (m, 1H) ; 6,05 (s, 2H) ; 6,7 - 7,5 (m, 7H) ; 8,3 - 11,2 (m, 2H).

**Exemple 12 :**

**Hydrogéno maléate de la N-[1-[4-(3,4-difluorophénoxy)butyl]pipéridin-4-yl]-4H-3,1-benzothiazin-2-amine (12).**

1,1 g (3,07 mmoles) de dichlorhydrate de la 1-[4-(3,4-difluorophénoxy)butyl]-4-pipéridinamine (F = 240 - 3°) est condensé avec 700 mg (3,07 mmoles) d'orthobromométhylphénylisothiocyanate selon **l'exemple 1** et fournit 1,01

g de base attendue (F = 131°) qui est salifiée par l'acide maléïque pour donner 1,16 g (Rdt : 69 %) de cristaux blancs de **formule 12 :**

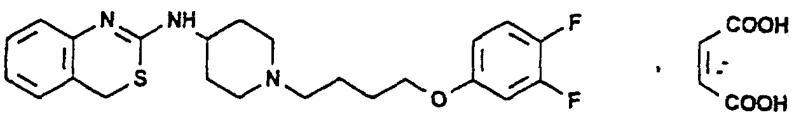

(12)

Formule brute : $C_{27}H_{31}F_2N_3O_5S$
Masse moléculaire : 547,606
Point de fusion : 103 - 104°
**IR** (KBr) : υ (N - H) : 3260 ; (C = N) ; 1606 ; ($COO^-$) : 1562 $cm^{-1}$.
**RMN** (DMSO $d_6$) δ : 1,4 - 2,1 (m, 6H) ; 2,1 - 2,5 (m, 2H) ; 2,5 - 3,1 (m, 4H) ; 3,1 - 3,6 (m, 2H) ; 3,7 - 4,5 (m, 5H) ; 6,22 (s, 2H) ; 6,4 - 6,9 (m, 2H) ; 6,9 - 7,7 (m, 5H) ; 10 - 12,5 (m, 2H).

**Exemple 13 :**

**Hydrogénomaléate de la N-[1-[4-(4-chlorophénoxy)butyl]pipéridin-4-yl]-4H-3,1-benzothiazin-2-amine (13).**

780 mg (2,19 mmoles) de dichlorohydrate de la 1-[4-(4-chlorophénoxy)butyl]-4-pipéridinamine (F = 224 - 6°) sont traités avec 500 mg (2,19 mmoles) d'orthobromo méthylisothiocyanate selon le procédé décrit dans **l'exemple 1** pour donner 860 mg (Rdt : 71 %) de poudre blanche de **formule 13 :**

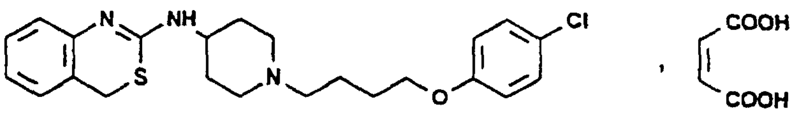

(13)

Formule brute : $C_{27}H_{32}ClN_3O_5S$
Masse moléculaire : 546,067
Point de fusion : 158 - 159°
**IR** (KBr) : υ (N - H) : 3230 ; (N = C) ; 1607 $cm^{-1}$.
**RMN** (DMSO $d_6$) δ : 1,5 - 1,9 (m, 6H) ; 1,9 - 2,4 (m, 2H) ; 2,8 - 3,7 (m, 6H) ; 3,91 (s, 2H) ; 3,98 (t, 2H) ; 3,8 - 4,4 (m, 1H) ; 6,01 (s, 2H) ; 6,8 - 7,05 (m, 4H) ; 7,05 - 7,2 (m, 2H) ; 7,2 - 7,65 (m, 3H) ; 8,5 - 10,5 (m, 2H).

**Exemple 14 :**

**Hémihydrate du dichlorhydrate de la N-[1-[2-hydroxy-3-(4-fluorophénoxy)propyl] pipéridin-4-yl]-4H-3,1-benzothiazin-2-amine (14).**

**14-1 :** 1-benzyl-4-terbutoxycarbonylamino pipéridine : dans un réacteur de 1 1, un mélange 40,8 ml (38 g, 0,2 mole) de 1-benzyl-4-pipéridinamine dans 220 ml de soude N et 110 ml de tertio-butanol est refroidi sur bain d'eau froide puis traité goutte à goutte avec une solution 43,7 g (0,2 mole) de ditertiobutyl dicarbonate dans 40 ml de tertio-butanol en maintenant la température vers 25°. L'agitation est poursuivie pendant 5 h à 25° puis le mélange est extrait plusieurs fois au chlorure de méthylène, lavé à l'eau séché sur sulfate de sodium, filtré puis évaporé à siccité. Le résidu est repris dans 250 ml d'éther isopropylique, agité, filtré et essoré pour donner 58,8 g (Rdt : 84 %) de poudre blanche de **formule 14-1 :**

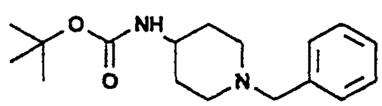

(14-1)

Formule brute : $C_{17}H_{26}N_2O_2$
Masse moléculaire : 190,29

Point de fusion : 121 - 122°

**14.2 :** Chlorhydrate de la 4-terbutoxycarbonylaminopipéridine : dans un réacteur de 1 l, une solution de 48 g (0,165 mole) de composé précédent dans 480 ml de chlorure de méthylène est refroidie à 0° puis traitée goutte à goutte avec 26 g (0,182 mole) de chlorure d'α-chloroéthoxy carbonyle, on laisse revenir lentement à 25° et on agite une nuit à cette température. Le mélange est évaporé à siccité pour donner une huile rosée qui cristallise à la longue. Le résidu est dilué avec 480 ml de méthanol et porté au reflux pendant 2 h puis le mélange est évaporé à siccité pour donner un solide beige qui est repris dans l'éther et agité pendant 1 h. L'insoluble est filtré, essoré et séché : pour donner 39,1 g de poudre blanche (Rdt : 97 %) de **formule 14-2 :**

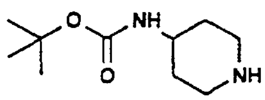

, HCl (14-2)

Formule brute : $C_{10}H_{21}ClN_2O_2$
Masse moléculaire : 236,72
Point de fusion : 151 - 152°
**IR** (KBr) υ (N - H) : 3296 ; (C=O): 1716 $cm^{-1}$.

**14-3 :** 4-terbutoxycarbonylamino-1-[2-hydroxy-3-(4-fluorophénoxy)propyl]pipéridine : dans un ballon de 250 ml, une solution de 10 g (49,8 mmoles) de base du composé principal précédent dans 100 ml de méthanol sec est traitée avec 10 g (59,62 mmoles) de 1-(4-fluorophénoxy)-2,3-époxypropane (préparé selon W.S. Di Menna et col, *J Med Chem* (1978), 21, p 1073-1076, Eb 94°/0,25 m bars, Rdt : 72 %) puis agitée à 25° pendant toute la nuit. Le mélange est évaporé à siccité, trituré dans l'éther isopropylique, filtré et essoré pour donner des cristaux blanc cassé (m = 15,1 g ; Rdt : 80 %) de **formule 14.3 :**

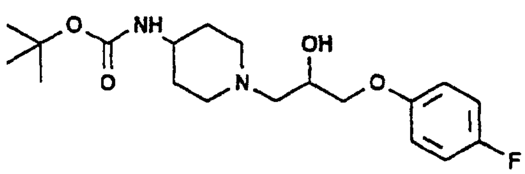

(14-3)

Formule brute : $C_{19}H_{29}FN_2O_4$
Masse moléculaire : 368,44
Point de fusion : 129 - 130°
**IR** (KRr) υ (N - H) : 3420 ; (O - H) : 3400 - 3200 ; (C = O) : 1709 $cm^{-1}$.

**14-4. :** Dichlorhydrate de la 1-[2-hydroxy-3-(4-fluorophénoxy)propyl]-4-pipéridin amine : dans un ballon de 250 ml, un mélange de 11 g (30 mmoles) de composé précédent dans 20 ml d'éthanol et 33 ml d'une solution 3 N d'acide chlorhydrique dans l'éthanol est agité pendant un week-end à 25°. L'insoluble est filtré, rincé avec de l'éther et séché pour donner 8,1 g (Rdt : 79 %) de poudre blanche de **formule 14.4 :**

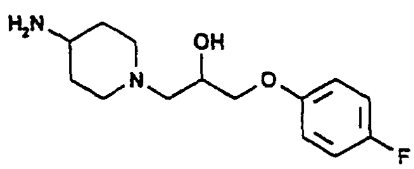

, 2 HCl (14-4)

Formule brute : $C_{14}H_{23}Cl_2FN_2O_2$
Masse moléculaire : 341,25
Point de fusion : 222 - 223°
**IR** (KBr) υ (OH, NH) : 3360 ; (C - O - C) : 1203 $cm^{-1}$.

**14-5 :** Hémihydrate du dichlorhydrate de la N-[1-[2-hydroxy-3-(4-fluorophénoxy) propyl]pipéridin-4-yl]-4H-3,1-benzothiazin-2-amine (14) : une solution de 500 mg (2,19 mmoles) d'orthobromométhylphénylisothiocyanate dans 7 ml de toluène est traitée par 748 mg (2,19 mmoles) de composé précédent en présence de 1,22 ml (8,8 mmoles) de triéthylamine. Puis on porte à 80° pendant 5h30. Après retour à 25° le mélange est filtré et l'insoluble rincé au toluène et le filtrat est évaporé à siccité pour donner un résidu qui est repris dans du chlorure de méthylène. La phase organique est lavée à l'eau, à l'eau salée, séchée sur sulfate et évaporée à siccité. L'huile brute résiduelle est purifiée par flash chromatographie en éluant avec un mélange $CH_2Cl_2$-95/MeOH-4,5/$NH_4OH$-0,5 pour donner après évaporation 720 mg (79 %) d'une huile jaune pâle qui cristallise (F = 117-118°).

Ce résidu est chlorhydraté de la manière habituelle avec une solution EtOH/HCl. Après recristallisation dans l'éthanol on obtient 600 mg (Rdt : 63 %) de cristaux blancs de **formule 14 :**

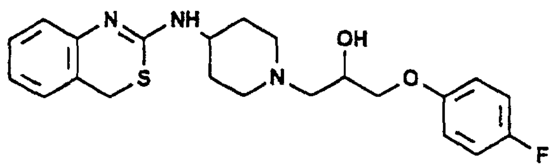

, 2 HCl , 1/2 H2O (14)

Formule brute : $C_{22}H_{26}N_3FO_2S$, 2HCl, 1/2$H_2O$
Masse moléculaire : 497,45
Point de fusion : 239 - 240°
(Point de fusion de la base : 117 - 118°)
**IR** (base) (KBr) υ (N - H) : 3422 ; (0 - H) : 3600 - 3300 ; (N = C) : 1608 $cm^{-1}$.
**RMN** (base ) (DMSO $d_6$) : 1,3 - 1,7 (m, 2H) ; 1,75 - 2 (m, 2H) ; 2 - 2,25 (m, 2H) ; 2,25-2,6 (m, 2H) ; 2,88 (t, 2H) ; 3,9 (s, 2H) ; 3,7 - 4,2 (m, 4H) ; 4,85 (s, 1H) ; 6,5 - 7,6 (m, 9H).

**Exemple 15 :**

**Dichlorhydrate de la N-méthyl-N-[1-[2-hydroxy-3-(4-fluorophénoxy)propyl]pipéridin-4-yl]-4H-3,1-benzothiazin-2-amine (15).**

**15-1 :** N-méthyl-1-benzyl-4-pipéridinamine : à un mélange de 37,85 g (0,2 mole) de 1-benzyl-4-pipéridinone dans 150 ml d'éther de pétrole et 40 ml d'acide acétique on ajoute 13,51 g (0,2 mole) de chlorhydrate de méthylamine et on agite le mélange pendant 3 h à 25°. On ajoute ensuite 90 ml de méthanol puis goutte à goutte 24 ml (18,6 g ou 0,2 mole) de borane pyridine 8 M et on maintient l'agitation pendant 5 h de plus à 25°. Le mélange est hydrolysé en versant goutte à goutte 100 ml d'acide chlorhydrique 6N sous vive agitation. La base est ensuite libérée par addition de lessive de soude jusqu'à pH12, extraite avec de l'éther puis lavée avec de l'eau salée, séchée sur sulfate et évaporée à siccité pour donner une huile verdâtre (m = 38 g). La purification sur 1000 g de silice par "flash chromatographie" donne après élution avec un mélange $CH_2Cl_2$ - 95 / MeOH - 4,5. / $NH_4OH$ - 0,5 et évaporation à siccité une huile incolore (m = 32,5 g ; Rdt : 79 %) de **formule 15.1 :**

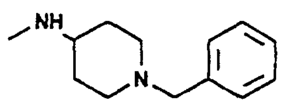

(15-1)

Formule brute : $C_{13}H_{20}N_2$
Masse moléculaire : 204,31
Huile incolore.

**15-2 :** N-tertbutoxycarbonyl-N-méthyl-1-benzyl-4-pipéridinamine: une solution de 18,4 g (90 mmoles) de composé précédent dans 120 ml de tertiobutanol est traitée avec 100 ml de soude aqueuse normale puis goutte à goutte avec 19,65 g (90 mmoles) de ditertiobutyldicarbonate et l'agitation est poursuivie toute la nuit à 25°. Le mélange est extrait 3 fois à l'éther puis la phase organique est lavée à l'eau, à l'eau salée, séchée sur sulfate de sodium et évaporée à siccité pour donner une huile ambrée (m = 25,8 g ; Rdt : 84 %) de **formule 15.2** qui est utilisée telle quelle dans l'étape suivante :

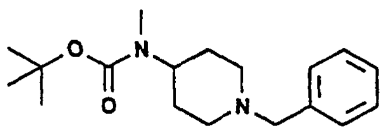

( 15-2 )

Formule brute : $C_{18}H_{28}N_2O_2$
Masse moléculaire : 304,42
Huile ambrée
**RMN** ($CDCl_3$) δ : 1,43 (s, 9H) ; 1,5 - 1,9 (m, 4H) ; 2 (t.d, 2H) ; 2,7 (s, 3H) ; 2,8 - 3 (m, 2H) ; 3,46 (s, 2H) ; 3,6 - 4,2 (m, 1H) ; 7,15 - 7,35 (m, 5H).

**15-3 :** Chlorhydrate de la N-méthyl-N-tertiobutoxycarbonyl-4-pipéridinamine : une solution de 24,35 g (80 mmoles) de dérivé précédent dans 200 ml de chlorure de méthylène est refroidie à 0° puis traitée goutte à goutte avec 9,50 ml (12,5 g ou 88 mmoles) d'α-chloroéthylchloroformiate puis on laisse revenir à 25° et on maintient l'agitation pendant 18 heures de plus. Le mélange est évaporé à siccité et le résidu repris dans 200 ml de méthanol et porté au reflux pendant 2 h. Après évaporation à sec sous vide, le résidu est trituré dans de l'éther, l'insoluble est filtré, rincé, essoré et séché pour donner une poudre blanche (m = 19,6 g ; Rdt : 98 %) de **formule 15.3 :**

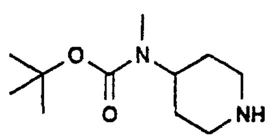

, HCl ( 15-3 )

Formule brute : $C_{11}H_{23}ClN_2O_2$
Masse moléculaire : 250,76
Point de fusion : > 250°

**15-4 :** Dichlorhydrate de la 1-[2-hydroxy-3-(4-fluorophénoxy)propyl]-N-méthyl-4-pipéridinamine : une solution de 3 g (14 mmoles) de base précédente dans 30 ml de méthanol sec est traitée avec 2,35 g (14 mmoles) de 1-(4-fluorophénoxy)-2,3-époxy propane et le mélange est agité pendant 24 h à 25° puis on évapore à siccité pour obtenir le composé de **formule 15.4.1.** qui est hydrolysé directement dans 30 ml d'acide chlorhyrique 3N en portant le mélange au reflux pendant 2h30.

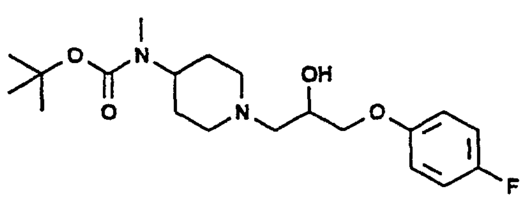

( 15-4-1 )

Le mélange est évaporé à siccité, repris dans 20 ml d'éthanol. Le dichlorhydrate de **formule 15.4** précipite par addition d'éther et est récupéré de la manière habituelle (m = 4,20 g ; Rdt : 84 %).

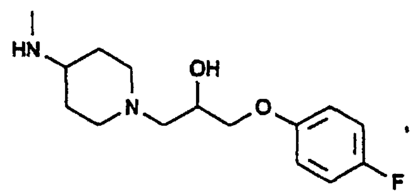

, 2 HCl ( 15-4 )

Formule brute : $C_{15}H_{25}Cl_2FN_2O_2$
Masse moléculaire : 355,28

Point de fusion : 198 - 200°
**RMN** (DMSO $d_6$) δ : 1,8 - 2,45 (m, 4H) ; 2,8 - 4,2 (m, 10H) ; 3,37 (s, 3H) ; 4,25 - 4,6 (m, 1H) ; 5,85 - 6,2 (m, 1H) ; 6,8 - 7,4 (m, 4H) ; 9,3 - 9,8 (m, 2H).

**15-5 :** Dichlorhydrate de la N-méthyl-N-[1-[2-hydroxy-3-(4-fluorophénoxy)propyl]pipéridin-4-yl]-4H-3,1-benzothiazin-2-amine: en opérant comme décrit dans **l'exemple 14.5** mais en partant de 860 mg (2,41 mmoles) de dérivé précédent et de 550 mg (2,41 mmoles) d'orthobromométhylphényl isothiocyanate, on prépare 820 mg (Rdt : 68 %) de cristaux blancs de **formule 15 :**

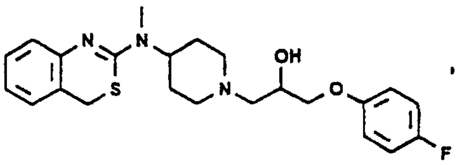

, 2 HCl (15)

Formule brute : $C_{23}H_{30}Cl_2FN_3O_2S$
Masse moléculaire : 502,46
Point de fusion : 249 - 251°
**IR** (KBr) ν (OH) : 3238 ; ($NH^+$) : 2473 ; (N = C) : 1622 $cm^{-1}$.
**RMN** (DMSO $d_6$) δ : 1,7 - 2,2 (m, 2H) ; 2,25 - 2,8 (m, 2H) ; 2,9 - 3,5 (m, 5H) ; 3,27 (s, 3H) ; 3,55 - 3,85 (m, 2H) ; 3,94 (d, 2H) ; 4,29 (s, 2H) ; 4,28 - 4,5 (m, 1H) ; 4,6 - 6,3 (m, 2H) ; 6,8 - 8 (m, 8H), 10,3 - 10,5 (m, 1H).

<u>**Exemple 16 :**</u>

**Hydrogénomaléate de la N-méthyl-N-[1-(4-phénoxybutyl)pipéridin-4-yl]-4H-3,1-benzothiazin-2-amine (16).**

**16-1** : N-méthyl-N-[1-benzylpipéridin-4-yl]-4H-3,1-benzothiazin-2-ylamine: un mélange de 6,32 g (22,8 mmoles) de dichlorhydrate de N-méthyl-1-benzyl-4-pipéridinamine (préparée selon décrit dans **l'exemple 15.1)** et de 5,2 (22,8 mmoles) de 2-bromométhyl phénylisothiocyanate et 12,7 ml de triéthylamine (91,2 mmoles) dans 60 ml de toluène est porté pendant 1 h à 80° sous agitation magnétique. Après retour à 40° le mélange est évaporé à siccité sous vide puis repris dans de l'eau et on extrait plusieurs fois au chlorure de méthylène, puis on lave à l'eau, à l'eau salée et on sèche sur sulfate de sodium. Après élimination du sel minéral, le mélange est évaporé à siccité sous vide puis purifié par chromatographie flash sur 450 ml de silice en éluant avec un mélange $CH_2Cl_2$-97,5 / MeON-2,5. Les fractions renfermant le composé attendu sont réunies et évaporées à sec pour donner 7,2 g (Rdt : 86 %) d'une huile jaune pâle de **formule 16.1 :**

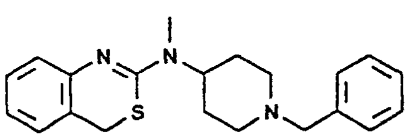

(16-1)

Formule brute : $C_{21}H_{25}N_3S$
Masse moléculaire : 351,49
**RMN** ($CDCl_3$) δ : 1,6 - 1,75 (m, 2H) ; 1,89 (q.d, 2H) ; 2,13 (t.d, 2H) ; 2,9 - 3,05 (m, 2H) ; 3,11 (s, 3H) ; 3,54 (s, 2H) ; 3,85 (s, 2H) ; 4,3 - 4,55 (m, 1H) ; 6,85 - 7,15 (m, 3H) ; 7,15 - 7,45 (m, 6H).

**16-2 :** Chlorhydrate de la N-méthyl-N(pipéridin-4-yl)-4H-3,1-benzothiazin-2-amine : une solution de 400 mg (1,14 mmole) de base précédente dans 4 ml de 1,2-dichloroéthane est refroidie sur bain de glace puis traitée goutte à goutte avec 0,135 ml (1,25 mmole) d'α-chloroéthyl chloroformiate puis on laisse revenir lentement à 25° et on agite 3 h de plus à cette température. Le mélange est évaporé à siccité sous vide, repris dans 4 ml de Méthanol et porté 1 h au reflux. Le mélange est évaporé à siccité pour donner une huile qui cristallise. On la reprend dans de l'éther et on filtre les cristaux blancs (m = 310 mg ; Rdt : 91 %) de **formule 16.2 :**

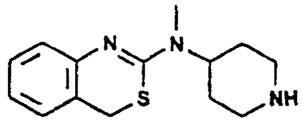

, HCl (16-2)

Formule brute : $C_{14}H_{20}ClN_3S$
Masse moléculaire : 297,84
Point de fusion : 200°
RMN (DMSO $d_6$) δ : 1,6 - 1,95 (m, 2H) ; 2 - 2,35 (m, 2H) ; 2,7 - 3,3 (m, 4H) ; 3,34 (d, 3H) ; 4,04 (s, 2H) ; 4,4 - 4,9 (m, 1H); 6,8 - 7,5 (m, 4H) ; 9,1 (s, 2H).

**16-3 :** Hydrogénomaléate de la N-méthyl-N-[1-(4-phénoxybutyl)pipéridin-4-yl]-4H-3,1-benzothiazin-2-amine: une suspension de 284 mg (0,95 mmole) de composé précédent dans 3 ml de DMF sec est traitée successivement par 182 mg (0,8 mmole) de 1-bromo-4-phénoxybutane et 210 mg (2 mmoles) de $Na_2CO_3$ puis chauffée à 80° pendant 2h30. Après retour à 25° le mélange est extrait plusieurs fois à l'acétate d'éthyle, lavé à l'eau, à l'eau salée et séché sur sulfate de sodium. Après élimination du sel minéral le mélange est évaporé à sec pour donner une huile qui est purifiée par flash chromatographie en éluant avec un mélange $CH_2Cl_2$-97,5 / MeOH - 2,25 / $NH_4OH$-0,25. Les fractions renfermant le dérivé attendu sont réunies, évaporées à siccité et la base obtenue est salifiée avec de l'acide maléïque de la manière habituelle : cristaux blancs (m = 290 mg ; Rdt : 58 %) de **formule 16 :**

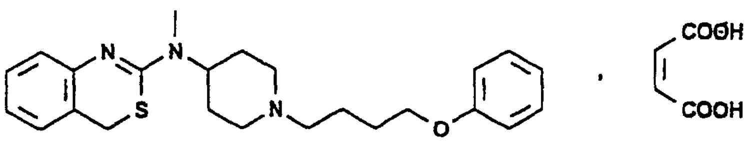

(16)

Formule brute : $C_{28}H_{35}N_3O_5S$
Masse moléculaire : 525,65
Point de fusion : 119 - 121°
**IR** (KBr) ν ($COO^-$) : 1566 $cm^{-1}$.
**RMN** ($CDCl_3$) δ : 1,75 - 2,2 (m, 6H) ; 2,25 - 2,6 (m, 2H) ; 2,85 - 3,2 (m, 4H) ; 3,14 (s, 3H) ; 3,6 - 3,8 (m, 2H) ; 3,92 (s, 2H) ; 4,01 (t, 2H) ; 4,95 - 5,25 (m, 1H) ; 6,3 (s, 2H) ; 6,8 - 7,2 (m, 6H) ; 7,2 - 7,4 (m, 3H) ; 11,5 - 13,5 (m, 2H).

**Exemple 17 :**

**N-[1-[4-(4-fluorophenoxy)butyl]pipéridin-4-yl]-4H-3,1-benzothiazin-2-amine (4).**

**17-1 :** 1-[4-(4-fluorophénoxy)butyl]pipéridin-4-ylisothiocyanate. Une solution de 470 mg (2 mmoles) de dipyridyl thiocarbonate dans 5,5 ml de chlorure de méthylène sec est traitée goutte à goutte à 25° sous atmosphère d'azote avec une solution de 515 mg de 1-[4-(4-fluorophénoxy)butyl]-4-pipéridinamine (1,95 mmoles) dans 3 ml de chlorure de méthylène. Après 20 minutes d'agitation la solution est diluée avec 30 ml de chlorure de méthylène, lavée à l'eau, à l'eau salée puis séchée sur sulfate de sodium. Par évaporation on récupère une huile légèrement orangée (m = 600 mg ; Rdt : 97 %) de **formule 17-1.**

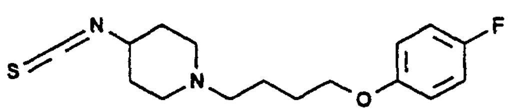

(17-1)

Formule brute : $C_{16}H_{21}FN_2OS$
Masse moléculaire : 308,408
**IR** (Lames NaCl) υ (N = C = S) : 2105 $cm^{-1}$.
**RMN** ($CDCl_3$) δ : 1,5 - 2,2 (m, 8H) ; 2,2 - 2,5 (m, 4H) ; 2,5 - 2,85 (m, 2H) ; 3,6 - 3,85 (m, 1H) ; 3,90 (t, 2H) ; 6,73 - 6,85 (m, 2H) ; 6,85 - 7,05 (m, 4H).

**17-2 :** 1-[1-[4-(4-fluorophénoxy)butyl]pipéridin-4yl]-3-(2-hydroxyméthylphényl)thiourée. A une solution de 800 mg (3 mmoles) d'isothiocyanate précédent dans 8 ml de dioxane sec chauffée à 60° on ajoute goutte à goutte une solution de 370 mg (3 mmoles) d'alcool 2-aminobenzylique dans 4 ml de dioxane puis on chauffe 2 heures de plus à cette température. On laisse revenir à 25° puis on filtre les cristaux blancs (m = 980 mg ; Rdt : 76 %) de thiourée ayant pour **formule 17-2.**

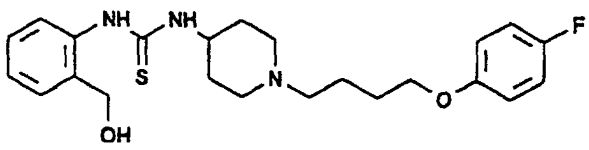

( 17-2 )

Formule brute : $C_{23}H_{30}FN_3O_2S$
Masse moléculaire : 431,55
Point de fusion : 116 - 117°
**RMN** ($CDCl_3$) δ : 1,25 - 1,9 (m, 6H) ; 1,95 - 2,25 (m, 4H) ; 2,36 (t, 2H) ; 2,7 - 2,95 (m, 2H) ; 2,95 - 3,5 (m, 1H) ; 3,90 (t, 2H) ; 4 - 4,4 (m, 1H) ; 4,63 (s, 2H) ; 6,12 (d, 1H) ; 6,7 - 6,85 (m, 2H) ; 6,85 - 7,05 (m, 2H) ; 7,15 - 7,5 (m, 4H) ; 8,31 (s, 1H).

**17-3 :** N-[1-[4-(4-fluorophénoxy)butyl]pipéridin-4-yl]-4H-3,1-benzothiazin-2-amine (4'). Un solution de 610 mg (1,4 mmole) de thiourée précédente dans 5 ml d'HCl concentré est chauffée au reflux pendant 10 minutes. Après retour à 25° on ajoute de la glace, pilée et on basifie jusqu'à pH de 12 avec une solution concentrée de soude. La base organique libérée est extraite à l'acétate d'éthyle et séchée sur sulfate puis évaporée à siccité. Le résidu solide est ensuite recristallisé de l'alcool bouillant pour donner des cristaux blancs (m = 470 mg ; Rdt : 81 %) de **formule 4'** fondant à 133° dont les caractéristiques spectrales sont identiques à celles de la base du dérivé préparé dans **l'exemple 4 :**

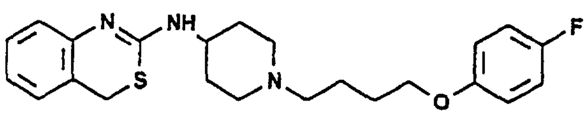

( 4' )

__Exemple 18 :__

**Dihydrogénomaléate de la 6,7-diméthoxy-N-[1-[3-(3,4-difluorophénoxy)propyl]pipéridin-4-yl]-4H-3,1-benzothiazin-2-amine(18).**

**18-1 :** Alccol 2-amino-4,5-diméthoxy benzylique : A une suspension de 1,90 g (50 mmoles) de $LiAlH_4$ dans 150 ml de THF sec refroidie à 0°, on ajoute goutte à goutte en 1 heure 10,50 g (50 mmoles) de 2-amino-4,5-diméthoxybenzoate de méthyle dissous dans 150 ml de THF. On agite 1 h à cette température puis on laisse revenir à 25° et on agite une heure de plus à 25°. Le mélange est refroidi à 0° et traité goutte à goutte avec 30 ml d'acétate d'éthyle. L'insoluble est filtré sur filtre de silice, rincé avec le même solvant et le filtrat est évaporé à siccité. Le résidu est repris dans du chlorure de méthylène et rincé avec un peu d'eau salée saturée, séché et évaporé, puis purifié par flash chromatographie. Le résidu est trituré dans un peu d'éther isopropylique pour donner des cristaux roses de **formule 18.1** qui s'assombrissent à la longue (m = 6,5 g ; Rdt : 65 %).

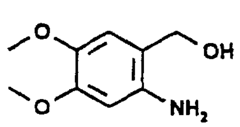

( 18-1 )

Formule brute : $C_9H_{13}NO_3$
Masse moléculaire : 183,30
Point de fusion : 76° - 77°
**RMN** ($CDCl_3$) δ : 3,1 (s, 3H) ; 3,80 (s, 3H) ; 3,87 (s, 3H) ; 4,6 (s, 2H) ; 6,3 (s, 1H) ; 6,65 (s, 1H).

**18-2 :** 1-[3-(3,4-difluorophénoxy)propyl]pipéridin-4-ylisothiocyanate. En opérant comme décrit dans l'exemple **17-1** mais en partant de 22,3 mmoles de 1-[3-(3,4-difluorophénoxy)propyl]-4-pipéridinamine et en les condensant sur 6,14 g (23 mmoles) de dipyridylthiocarbonate, on prépare avec un rendement de 62 % l'isothiocyanate de **formule 18.2 :**

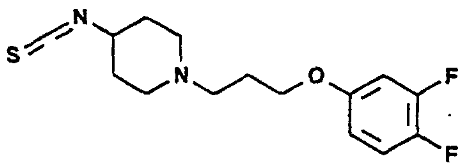

(18-2)

Formule brute : $C_{17}H_{22}F_2N_2OS$
Masse moléculaire : 313,38
Huile ambrée.
**IR** (Lames NaCl) : ν (N = C = S) : 2106 $cm^{-1}$
**RMN** ($CDCl_3$) δ : 1,18 - 2,1 (m, 6H) ; 2,2 - 2,45 (m, 2H) ; 2,49 (t, 2H) ; 2,5 -2,75 (m, 2H) ; 3,65 - 3,85 (m, 1H) ; 3,93 (t, 2H) ; 6,5 - 6,8 (m, 2H) ; 7,03 (q, 1H).

**18-3 :** 1-[1-[3-(3,4-difluorophénoxy)propyl]pipéridin-4-yl]-3-(2-hydroxyméthyl-4,5-dimé thoxyphenyl)thiourée. En utilisant le procédé décrit dans l'exemple **17.2** mais en partant de 760 mg (4,15 mmoles) de dérivé **18-1** et en le condensant sur 1,40 g (5,6 mmoles) de composé précédent on prépare 1,62 g (Rdt : 79 %) de cristaux blancs de **formule 18.3 :**

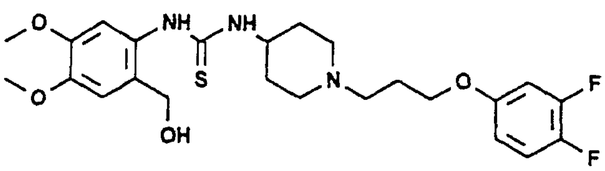

(18-3)

Formule brute : $C_{24}H_{31}F_2N_3O_4S$
Masse moléculaire : 495,57
Point de fusion : 168°
**RMN** (DMSO $d_6$) : δ : 1,3 -1,6 (m, 2H) ; 1,75 - 2,1 (m, 6H) ; 2,39 (t, 2H) ; 2,7 - 2,9 (m, 2H) ; 3,72 (s, 3H) ; 3,77 (s, 3H) ; 3,98 (t, 2H) ; 3,9 - 4,2 (m, 1H) ; 4,36 (d, 2H) ; 5,09 (t, 1H) ; 6,7 - 6,83 (m, 1H) ; 6,85 (s, 1H) ; 7 (s, 1H) ; 7 - 7,2 (m, 1H) ; 7,2 - 7,5 (m, 2H) ; 8,84 (s, 1H).

**18-4 :** Dihydrogénomaléate de la 6,7-diméthoxy-N-[1-[3-(3,4-difluorophénoxy)propyl] pipéridin-4-yl]-4H-3,1-benzothiazin-2-amine (18). En opérant selon le protocole de l'exemple 173 on prépare avec un rendement de 76 % le dérivé de **formule 18** à partir de 2,86 mmoles de **composé 18.3 :**

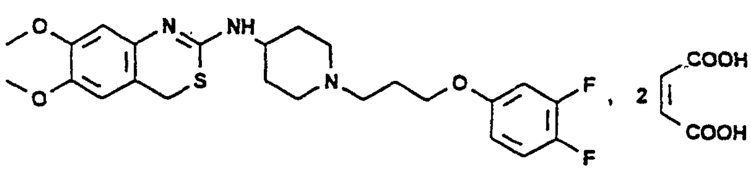

(18)

Formule brute : $C_{32}H_{37}F_2N_3O_{11}S$
Masse moléculaire: 709,69
Point de Fusion : 180 - 182°C
**RMN** (DMSO $d_6$) δ : 1,5 - 1,9 (m, 2H) ; 1,9 - 2,4 (m, 4H) ; 2,9 - 3,4 (m, 4H) ; 3,4 - 3,7 (m, 2H) ; 3,72 (s, 6H) ; 3,9 (s, 2H) ; 4,05 (t, 2H) ; 3,9 - 4,4 (m, 1H) ; 6,13 (s, 4H) ; 6,53 (s, 1H) ; 6,7 - 6,85 (m, 1H) ; 6,79 (s, 1H) ; 7 - 7,2 (m, 1H) ; 7,38 (q, 1H) ; 7,3-7,7 (m, 1H) ; 8,6 - 9,8 (m, 4H).

**Exemple 19 :**

**Dihydrogénomaléate de la 6,7-diméthoxy-N-méthyl-N-[1-[3-(3,4-difluorophénoxy) propyl]pipéridin-4-yl]-4H-3,1-benzothiazin-2-amine (19).**

Une solution de 890 mg (1,86 mmole) de 6,7-diméthoxy-N-[1-[3-(3,4-difluorophénoxy)propyl]pipéridin-4-yl]-4H-3,1-benzothiazine-2-amine base brute préparée dans l'exemple **18** dans 9 ml de DMF sec est traitée à 0° avec 100 mg (2,5 mmoles) de NaH à 60 % et agitée 30 minutes, puis 290 mg (0,22 ml ou 2,05 mmoles) de MeI sont ajoutés et on laisse revenir lentement à 25°. Après 1h15 d'agitation à cette température le DMF est éliminé sous vide et le résidu est repris dans du chlorure de méthylène, lavé à l'eau, à l'eau salée, puis séché sur sulfate de sodium. Après concentration à sec l'huile résiduelle est purifiée par "flash chromatographie" sur colonne de silice en éluant avec un mélange $CH_2Cl_2$-95/MeOH-4,5/$NH_4OH$-0,5. On obtient 560 mg d'huile (Rdt : 62 %) qui est salifiée avec l'acide maléïque de la manière habituelle pour donner 710 mg (Rdt : 52 %) de cristaux blancs de **formule 19 :**

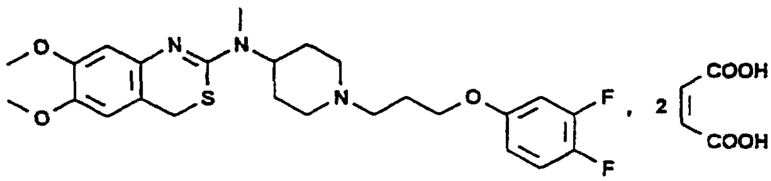

(19)

Formule brute : $C_{33}H_{39}F_2N_3O_{11}S$
Masse moléculaire : 723,72
Point de fusion 134°
**RMN** (DMSO $d_6$) δ : 1,75 - 2,3 (m, 6H) ; 3,03 (s, 3H) ; 3,1 - 3,4 (m, 4H) ; 3,55 - 3,78 (m, 2H) ; 3,73 (s, 3H) ; 3,74 (s, 3H) ; 3,94 (s, 2H) ; 4,08 (t, 2H) ; 4,45 - 4,85 (m, 1H) ; 6,16 (s, 4H) ; 6,57 (s, 1H) ; 6,81 (s, 1H) ; 6,75 - 6,85 (m, 1H) ; 7,05 - 7,2 (m, 1H) ; 7,41 (q, 1H) ; 8,2 - 10,5 (m, 4H).

**Exemple 20 :**

**Hémifumarate de la 6-méthyl-N-[1-[3-(3,4-difluorophénoxy)propyl]pipéridin-4-yl-3,1-benzothiazin-2-amine (20).**

**20-1 :** Alcool 2-amino-5-méthyl-benzylique : ce composé de **formule 20.1** est préparé avrec un rendement de 68 % en adaptant le mode opératoire de l'exemple **18.1 :**

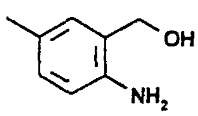

(20-1)

Formule brute : $C_8H_{11}NO$
Masse moléculaire : 137,18
Point de fusion : 124 - 125°
**RMN** (DMSOd6) δ : 2,13 (s, 3H) ; 4,33 (d, 2H) ; 4,68 (s, 2H) ; 4,95 (t, 1H) ; 6,51 (d, 1H) ; 6,77 (dd, 1H) ; 6,86 (s, 1H).

**20-2 :** 1-[1-[3-(3,4-difluorophénoxy)propyl]pipéridin-4-yl]-3-(2-hydroxyméthyl-4-méthyl phényl)thiourée : la condensation de 410 mg (3 mmoles) de composé précédent sur 1,05 g (3,3 mmoles) de 1-[3-(3,4-difluorophénoxy) propyl]pipéridin-4-yl isothiocyanate **(18.2)** selon l'**exemple 183** donne 1,18 g (Rdt 87 %) de résidu gommeux de **formule 20.2** utilisé tel quel dans l'étape suivante :

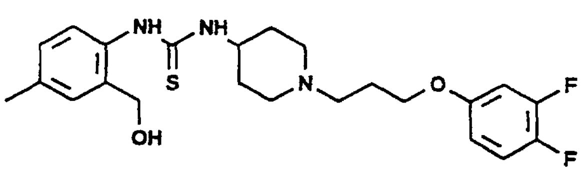

(20-2)

**20-3 :** Hémifumarate de la 6-méthyl-N-[1-[3-(3,4-difluorophénoxy)propyl]pipéridin-4-yl] -4H-3,1-benzothiazin-2-amine: la cyclisation de 1,08 g (2,4 mmoles) de base brute précédente selon le procédé décrit dans l'**exemple 18.4** donne avec un rendement de 63 % des cristaux blancs de **formule 20 :**

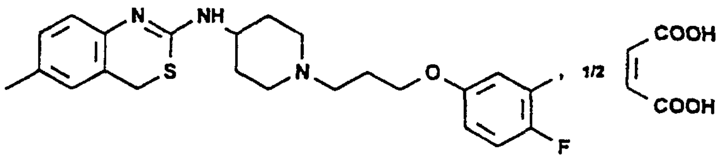

( 20 )

Formule brute : $C_{25}H_{29}F_2N_3O_3S$.
Masse moléculaire : 489,57
Point de fusion : 183°
**RMN** (DMSO $d_6$) δ : 1,4 - 1,75 (m, 2H) ; 1,75 - 2,05 (m, 4H) ; 2,05 - 2,45 (m, 2H) ; 2,24 (s, 3H) ; 2,4 - 2,75 (m, 2H) ; 2,8 - 3,2 (m, 2H) ; 3,86 (s, 2H) ; 3,8 - 4,2 (m, 3H) ; 6,56 (s, 1H) ; 6,65 - 7,5 (m, 7H) ; 8,5 - 10 (m, 1H).

<u>**Exemple 21 :**</u>

**Hydrogénomaléate de la N-méthyl-N-[1-[4-(4-méthylphénoxy)butyl]pipéridin-4-yl]-4H-3,1-benzothiazin-2-amine (21).**

La condensation de 700 mg (2,35 mmoles) de N-méthyl-N-[4-pipéridinyl]-4H-3,1-benzothiazin-2-amine obtenue dans l'exemple **16.2** sur 467 mg (2,35 mmoles) de 1-(4-méthyl-phénoxy)-4-chlorobutane dans 8 ml de DMF sec en présence de 630 mg (5,86 mmoles) de $K_2CO_3$-98/KI-02 selon le procédé décrit dans l'exemple **16.3** donne avec un rendement de 49 % des cristaux blancs de **formule 21 :**

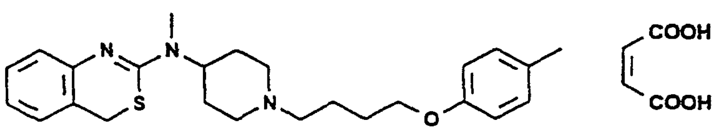

( 21 )

Formule brute : $C_{29}H_{37}N_3O_5S$
Masse moléculaire : 539,66
Point de fusion : 144-6°
**RMN** ($CDCl_3$) δ : 1,7 - 2,15 (m, 6H) ; 2,28 (s, 3H) ; 2,2 - 2,5 (m, 2H) ; 2,87 (t, 2H) ; 3,07 (s, 3H) ; 3 - 3,3 (m, 2H) ; 3,55 - 3,75 (m, 2H) ; 3,86 (s, 2H) ; 3,97 (t, 2H) ; 4,8 - 5,15 (m, 1H) ; 6,28 (s, 2H) ; 6,76 (d, 2H) ; 6,9 - 7,15 (m, 5H) ; 7,15 - 7,35 (m, 1H) ; 10,5 - 13,5 (m, 2H).

<u>**Exemple 22 :**</u>

**Hydrogénomaléate de la N-méthyl-N-[1-[4-(4-méthoxyphénoxy)butyl]pipéridin-4-yl]-4H-3,1-benzothiazin-2-amine (22).**

La condensation du 1,4-dibromobutane sur le 4-méthoxyphénol selon A.M. Ismaiel et col (*J Med Chem* (1993) <u>36</u>, 2519-25) donne avec un rendement de 82 % le 1-(4-méthoxyphénoxy)-4-bromobutane. En condensant 670 mg (2,58 mmoles) de ce dérivé sur 700 mg (2,35 mmoles) de N-méthyl-N-[4-pipéridinyl]-4H-3,1-Benzothiazin-2-amine obtenue dans l'exemple **16.2** dans 8 ml de DMF sec en présence de 630 mg (5,8 mmoles) de $Na_2CO_3$-98/KI-02 selon le mode opératoire de l'exemple **16.3** on prépare avec un rendement de 58 % le composé de **formule 22 :**

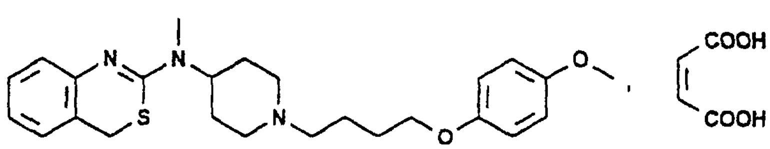

( 22 )

Formule brute : $C_{29}H_{37}N_3O_6S$
Masse moléculaire : 555,67
Point de fusion : 121-122°
**RMN** ($CDCl_3$) δ : 1,7 - 2,15 (m, 6H) ; 2,15 - 2,55 (m, 2H) ; 2,7 - 3 (m, 2H) ; 3,07 (s, 3H) ; 3 - 3,3 (m, 2H) ; 3,55 - 3,82 (m, 2H) ; 3,76 (s, 3H) ; 3,86 (s, 2H) ; 3,94 (t, 2H) ; 4,85 - 5,2 (m, 1H) ; 6,29 (s, 2H) ; 6,82 (s, 4H) ; 6,94 - 7,17 (m, 3H) ; 7,2 - 7,4 (m, 1H) ; 11-13,5 (m, 2H).

<u>**Exemple 23 :**</u>

**Dichlorhydrate de la (dl) N-[1-[2-hydroxy-3-(3,4-difluorophénoxy)propyl]pipéridin-4-yl]-4H-3,1-benzothiazin-2-amine (23).**

**23-1 :** 1-(3,4 difluorophénoxy)-2,3-époxypropane. La condensation de 25 g (192 mmoles) de 3,4-difluorophénol sur 45,2 ml (576 mmoles) d'épichlorhydrine dans 96 ml de soude 2N en présence de 2 g de $Bu_4NHSO_4$ selon le procédé de W.S. Di Menna et col *(J Med Chem* (1978) <u>21</u>, 1073-6) donne 28,4 g (Rdt : 80 %) d'huile de **formule 23.1 :**

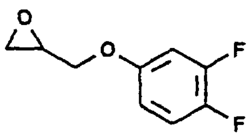

( 23-1 )

Formule brute : $C_9H_8F_2O_2$
Masse moléculaire : 186,154
Point d'ébullition : 104-105°/1 mbar

**23-2 :** 4-terbutoxycarbonylamino-1-[2-hydroxy-3-(3,4-difluorophénoxy)propyl]pipéridine. En partant de 7,5 g (37,4 mmoles) de 4-terbutoxycarbonylaminopipéridine préparée dans l'exemple **14.2** et en les condensant avec 8,35 g (45 mmoles) d'époxyde précédent dans 75 ml de méthanol selon le procédé décrit dans l'exemple **14.3** on obtient 16 g (Rdt : 83 %) de composé de **formule 23.2 :**

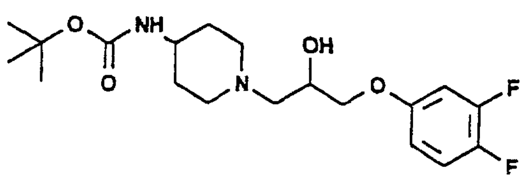

( 23-2 )

Formule brute : $C_{19}H_{28}F_2N_2O_4$
Masse moléculaire : 386,43
Point de fusion : 99 - 100°

**23-3 :** 1-[2-hydroxy-3-(3,4-difluorophénoxy)propyl]-4-pipéridinamine. 10 g (26 mmoles) de dérivé précédent sont hydrolysés par 75 ml d'acide chlorhydrique 3N selon le protocole de l'exemple **14.4** pour donner 5,9 g (Rdt : 79 %) de base de formule **23.3** qui est utilisée telle quelle dans l'étape suivante :

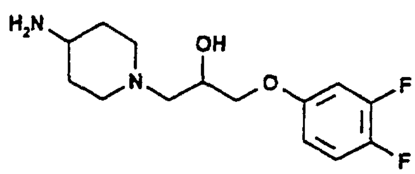

( 23-3 )

Formule brute : $C_{14}H_{20}F_2N_2O_2$
Masse moléculaire : 286,32

Huile jaune clair.

**23-4 :** Dichlorhydrate de la (dl) N-[1-[2-hydroxy-3-(3,4-difluorophénoxy)propyl] pipéridin-4-yl]-4H-3,1-benzothiazin-2-amine : la condensation de 530 mg (2,32 mmoles) de 2-bromo- méthylphénylisothiocyanate sur 665 mg (2,32 mmoles) de base **23.3** selon le procédé de l'exemple **14.5** fournit 675 mg (Rdt : 58 %) de cristaux blancs de **formule 23 :**

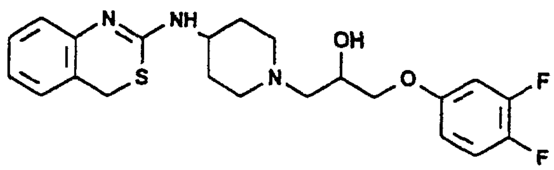
, 2 HCl (23)

Formule brute : $C_{22}H_{27}F_2Cl_2N_3O_2S$
Masse moléculaire : 506,43
Point de fusion : 250 - 251°
(Point de fusion de la base : 117 - 118°)
**RMN** (DMSO $d_6$) $\delta$ : 1,9 - 2,4 (m, 4H) ; 2,9 - 3,85 (m, 4H) ; 3,85 - 4,1 (m, 3H) ; 4,25 (s, 2H) ; 4,3 - 4,55 (m, 2H) ; 4,6 - 5 (m, 1H) ; 5,8 - 6,3 (m, 1H) ; 6,75 - 6,9 (m, 1H) ; 7,05-7,55 (m, 5H) ; 7,8 - 8,1 (m, 1H) ; 10,7 - 11,2 (m, 2H) ; 12,5 - 13,5 (m, 1H).

**Exemple 24 :**

**Hydrate du dihydrogénofumarate de la N-méthyl-N-[1-[4-(4-fluorophénoxy)butyl] pipéridin-4-yl]-1,5-dihydro-2,4-benzothiazepin-3-amine (24).**

**24.1 :** 1-Pivaloyl-3-[1-[4-(4-fluorophénoxy)butyl)pipéridin-4-yl]-3-méthylthiourée. Une suspension de 1,73 g (17,8 mmoles) de thiocyanate de potassium dans 100 ml d'acétone sèche est agitée sur bain de glace puis traitée goutte à goutte vers 4° avec 2,2 ml (17,8 mmoles) de chlorure de pivaloyle et l'agitation est poursuivie pendant 3 h à la même température. 5 g (17,8 mmoles) de N-méthyl-N-[1-[4-(4-fluorophénoxy)butyl]pipéridine-4-amine base sont ensuite introduits puis on laisse revenir lentement à 25° pendant une nuit. Le sel minéral est éliminé par filtration, on rince avec un peu d'acétone et le filtrat est évaporé à siccité pour donner une huile jaune qui est reprise dans du dichlorométhane, lavée à l'eau, séchée sur sulfate de sodium et évaporée à siccité. L'huile résiduelle triturée dans l'hexane donne 7,3 g (Rdt : 95 %) de cristaux blanc cassé de **formule 24.1 :**

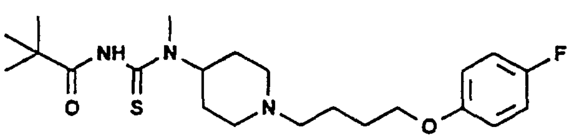
(24-1)

Formule brute : $C_{22}H_{34}FN_3O_2S$
Masse moléculaire : 423,574
Point de fusion : 115°
**RMN** ($CDCl_3$) $\delta$ : 1,26 (s, 9H) ; 1,6 - 2,1 (m, 8H) ; 2,1 - 2,3 (m, 2H) ; 2,48 (t, 2H) ; 3 (s, 3H) ; 3 - 3,2 (m, 2H) ; 3,94 (t, 2H) ; 4,8 - 5,1 (m, 1H) ; 6,71 - 7 (m, 2H) ; 7 - 7,1 (m, 2H) ; 7,85 (s, 1H).

**24-2 :** 1-méthyl-1-[1-[4-(4-fluorophénoxy)butyl]pipéridin-4-yl]thiourée: 5 g (11,32 mmoles) de composé précédent dans 100 ml d'acide chlorhydrique concentré sont portés une heure au reflux puis le mélange est refroidi à 5° et alcalinisé sur bain de glace avec de la lessive de soude à 50 %. La base libérée est extraite au dichlorométhane, lavée à l'eau, à l'eau salée, séchée sur sulfate de sodium. Le filtrat est évaporé à sec pour donner une huile ambrée qui est triturée dans l'hexane pour donner des cristaux beiges (m = 3,1 g ; Rdt : 77 %) de **formule 24.2 :**

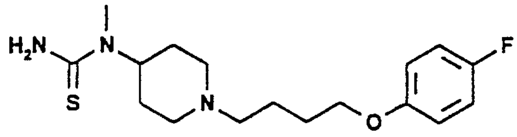

( 24-2 )

Formule brute : $C_{17}H_{26}FN_3OS$
Masse moléculaire : 339,477
Point de fusion : 106 - 107°
**RMN** ($CDCl_3$) δ : 1,5 - 1,9 (m, 8H) ; 2,08 (td, 2H) ; 2,39 (t, 2H) ; 2,94 (s, 3H) ; 2,9 - 3,1 (m, 2H) ; 3,91 (t, 2H) ; 4,8 - 5,25 (m, 1H) ; 5,71 (s, 2H) ; 6,7 - 6,85 (m, 2H) ; 6,85 - 7,95 (m, 2H).

**24-3 :** Hydrate du dihydrogénofumarate de la N-méthyl-N-[1-[4-(4-fluorophénoxy)butyl] pipéridin-4-yl]-1,5-dihydro-2,4-benzothiazépin-3-amine : Sous courant d'azote, une suspension de 1,56 g (14,7 mmoles) de carbonate de sodium dans 100 ml d'acétone est traitée avec 2,5 g (7,36 mmoles) de composé précédent puis avec 1,95 g (7,36 mmoles) d' α,α'-dibromo-o-xylène. Le mélange est porté au reflux pendant 4 h. Après retour à 25°, le sel minéral est filtré, et le filtrat est évaporé à siccité. L'huile orangée résiduelle est purifiée par "flash chromatographie" pour donner 2,06 g d'huile orangée (Rdt : 64 %) qui est salifiée de la manière habituelle par deux équivalents d'acide fumarique pour donner le **composé 24** de formule :

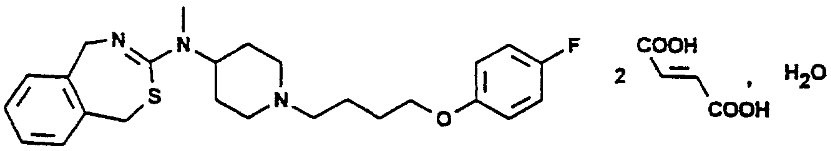

( 24 )

Formule brute : $C_{33}H_{40}FN_3O_9S$, $H_2O$
Masse moléculaire : 691,75
Point de fusion : 141 - 143°
**RMN** (base) ($CDCl_3$) δ : 1,5 - 1,85 (m, 8H) ; 1,99 (td, 2H) ; 2,35 (t, 2H) ; 2,75 (s, 3H) ; 2,75 - 3,1 (m, 2H) ; 3,9 (t, 2H) ; 3,9 - 4,1 (m, 1H) ; 4,22 (s, 2H) ; 4,76 (s, 2H) ; 6,8 - 6,87 (m, 2H) ; 6,87 - 7,05 (m, 2H) ; 7,1 - 74 (m, 4H).

**Exemple 25 :**

**Hydrate de dihydrogénofumarate de la N-méthyl-N-[1-[4-(3,4-difluorophénoxy) butyl]pipéridin-4-yl]-1,5-dihydro-2,4-benzothiazépin-3-amine (25).**

**25-1 :** 1-Pivaloyl-3-[1-[4-(3,4-difluorophénoxy)butyl]pipéridin-4-yl]-3-méthylthiourée : En opérant comme décrit dans **l'exemple 24.1,** mais en partant de 5 g (16,7 mmoles) de N-méthyl-1-[4-(3,4 difluorophénoxy)butyl]-4-pipéridinamine on prépare 6,3 g (Rdt : 85 %) de composé de **formule 25.1 :**

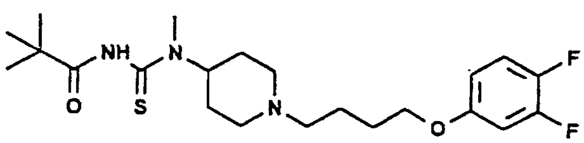

( 25-1 )

Formule brute : $C_{22}H_{33}F_2N_3O_2S$
Masse moléculaire : 441,564
Point de fusion : 87°
**RMN** ($CDCl_3$) δ : 1,25 (s, 9H) ; 1,5 - 2,2 (m, 10H) ; 2,4 (t, 2H) ; 2,97 (s, 3H) ; 2,95 - 3,1 (m, 2H) ; 3,90 (t, 2H) ; 4,75 - 5,05 (m, 1H) ; 6,5 - 6,63 (m, 1H) ; 6,69 (qd, 1H) ; 7,03 (q, 1H). 7,83 (s, 1H).

**25-2 :** 1-Méthyl-1-[1-[4-(3,4-difluorophénoxy)butyl]pipéridin-4-yl]thiourée : En hydrolysant le composé précédent selon le procédé de l'**exemple 24.2** on prépare avec un rendement de 89 % le composé de **formule 25.2 :**

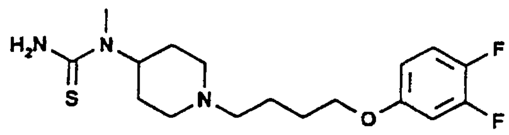

( 25-2 )

Formule brute : $C_{17}H_{25}F_2N_3OS$
Masse moléculaire : 357,454
Point de fusion : 81°C
**RMN** ($CDCl_3$) δ : 1,5 - 1,9 (m, 8H) ; 2,1 (qd, 2H) ; 2,39 (t, 2H) ; 2,95 (s, 3H) ; 2,9 - 3,1 (m, 2H) ; 3,9 (t, 2H) ; 4,85 - 5,3 (m, 1H) ; 5,72 (s, 2H) ; 6,48 - 6,61 (m, 1H) ; 6,68 (qd, 1H) ; 7,03 (q, 1H).

**25-3 :** Hydrate du dihydrogénofumarate de la N-méthyl-N-[1-[4-(3,4-difluorophénoxy) butyl]pipéridin-4-yl]-1,5-dihydro-2,4-benzothiazepin-3-amine (25) : la condensation de 3 g (8,4 mmoles) de composé précédent sur 2,21 (8,4 mmoles) d'α, α'-dibromo-o-xylène selon le descriptif de l'**exemple 24.3** donne avec un rendement de 52 % le composé de **formule 25 :**

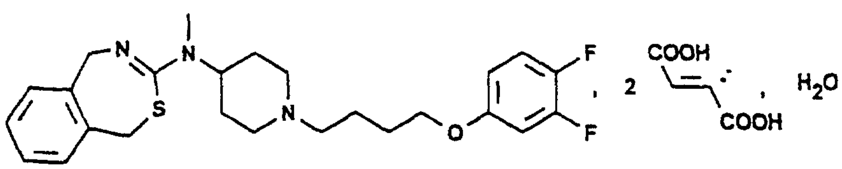

( 25 )

Formule brute : $C_{33}H_{39}F_2N_3O_9S$, $H_2O$
Masse moléculaire : 709,763
Point de fusion: 122 - 124°
**RMN** de la base ($CDCl_3$) δ : 1,5 - 1,85 (m, 8H) ; 1,96 (td, 2H) ; 2,34 (t, 2H) ; 2,73 (s, 3H) ; 2,85 - 3,05 (m, 2H) ; 3,88 (t, 2H) ; 3,9 - 4,15 (m, 1H) ; 4,21 (s, 2H) ; 4,75 (s, 2H) ; 6,45 - 6,6 (m, 1H) ; 6,67 (qd, 1H) ; 7,02 (q, 1H) ; 7,12 - 7,35 (m, 4H).

<u>**Exemple 26 :**</u>

**Hémifumarate de la 6,7-diméthoxy-N-[1-[3-(4-fluorophénoxy)propyl]pipéridin-4-yl]-4H-3,1-benzothiazin-2-amine (26).**

**26-1 :** 1-[3-(4-fluorophénoxy)propyl]pipéridin-4-yl isothiocynate : une solution de 6 g (23,7 mmoles) de 1-[3-[4-fluorophénoxy)propyl]-4-pipéridinamine base (cf. exemple 5) dans 60 ml de DMF sec est ajoutée goutte à goutte sous courant d'azote à une solution de 4,31 g (24,2 mmoles) de thiocarbonyl diimidazole dans 40 ml de DMF sec refroidie sur bain de glace. Après agitation 30 minutes à 0°, on dilue le mélange dans 750 ml d'eau froide et on extrait à l'acétate d'éthyle. La phase organique est lavée à l'eau salée, séchée sur sulfate de sodium et évaporée à siccité pour donner 6,65 g (Rdt : 94 %) d'une huile ambrée de **formule 26.1 :**

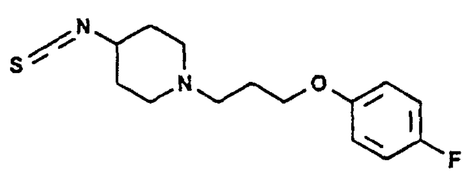

( 26-1 )

Formule brute : $C_{15}H_{19}FN_2OS$
Masse moléculaire : 294,38
Huile ambrée :
**IR** (lames NaCl) ν : (N = C = S) 2107 $cm^{-1}$.

**26-2 :** 1-[1-[3-[4-fluorophénoxy)propyl]pipéridin-4-yl]-3-(2-hydroxyméthyl-4,5-diméthoxyphényl)thiourée : en partant de 2,59 g (8,8 mmoles) de dérivé précédent et en les condensant sur 1,47 g d'alcool 2-amino-4,5-diméthoxybenzylique (préparé dans l'exemple **18.1**) selon le procédé décrit dans l'exemple **17.2** on prépare avec un rendement de 84 % des cristaux beiges de **structure 26.2 :**

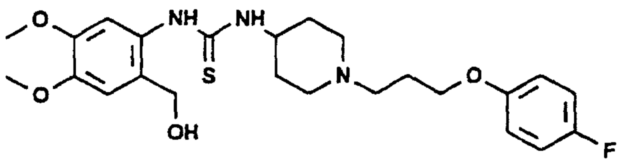

( 26-2 )

Formule brute : $C_{24}H_{32}FN_3O_4S$
Masse moléculaire : 477,58
Point de fusion : 146°
**RMN** (DMSO $d_6$) δ : 1,3 - 1,65 (m, 2H) ; 1,7 - 2,1 (m, 6H) ; 2,4 (t, 2H) 2,7 - 2,95 (m, 2H) ; 3,71 (s, 3H) ; 3,77 (s, 3H) ; 3,96 (t, 2H) ; 3,9 - 4,3 (m, 1H) ; 4,36 (d, 2H) ; 5,08 (t, 1H) ; 6,8 - 7,2 (m, 6H) ; 7,3 - 7,6 (m, 1H) ; 8,82 (s, 1H).

**26-3 :** Hémifumarate de la 6,7-diméthoxy-N-[1-[3-(4-fluorophénoxy)propyl]pipéridin-4-yl]-4H-3,1-benzothiazin-2-amine : par cyclisation de 3 g (6,38 mmoles) de composé **26.2** précédent selon le procédé de l'exemple **17.3** on obtient en salifiant avec de l'acide fumarique le composé de **formule 26 :**

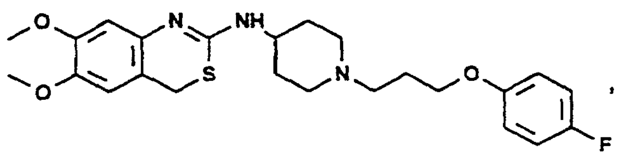 , 1/2 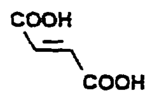

( 26 )

Formule brute : $C_{26}H_{32}FN_3O_5S$
Masse moléculaire : 517,60
Point de fusion : 190 - 191°
**RMN** (DMSO $d_6$) δ : 1,35 - 1,7 (m, 2H) ; 1,7 - 2,05 (m, 4H) ; 2,1 - 2,35 (m, 2H) ; 2,56 (t, 2H) ; 2,8 - 3,1 (m, 2H) ; 3,7 (s, 3H) ; 3,72 (s, 3H) ; 3,84 (s, 2H) ; 3,98 (t, 2H) ; 3,7 - 4,5 (m, 2H) ; 6,50 (s, 1H) ; 6,55 (s, 1H) ; 6,75 (s, 1H) ; 6,85 - 7,05 (m, 2H) ; 7,05 - 7,25 (m, 2H) ; 9,5 - 12 (m, 1H).

**Exemple 27 :**

**Dichlorhydrate de la N-[1-[4-(4-fluorophénoxy)butyl]pipéridin-4-yl]-4H-3,1-benzoxazin-2-amine (27).**

**27-1 :** 1-[1-[4-(4-fluorophénoxy)butyl]pipéridin-4-yl]-3-(2-hydroxyméthylphényl)urée : une solution de 754 mg (2,54 mmoles) de triphosgène dans 50 ml de $CH_2Cl_2$ sec est refroidie à 0° sous courant d'azote et traitée goutte à goutte avec une solution de 1,88 g (7,6 mmoles) de 1-[4-(4-fluorophénoxy)butyl]-4-pipéridinamine et de 1,08 ml (7,76 mmoles de triéthylamine dans 25 ml de $CH_2Cl_2$. Après 5 minutes d'agitation à 0° on laisse revenir à 25° pendant 2 h. L'intermédaire réactionnel formé n'est pas isolé et la solution précédente est traitée goutte à goutte par une solution de 869 mg (7,06 mmoles) d'alcool 2-aminobenzylique et 0,98 ml (7,06 mmoles) de triéthylamine dans 25 ml de $CH_2Cl_2$. Après agitation une nuit à 25°, on dilue avec du $CH_2Cl_2$ et on lave à l'eau, à l'eau salée et on sèche sur sulfate de sodium. Après élimination du sel minéral et évaporation à sec, on récupère une huile qui, triturée dans l'éther isopropylique, donne 2,1 g (Rdt : 72 %) de cristaux beiges de **formule 27.1 :**

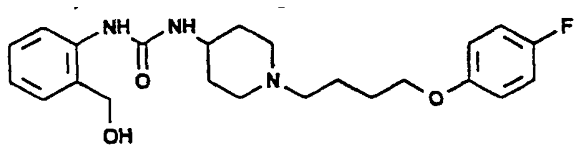

( 27-1 )

Formule brute : $C_{23}H_{30}FN_3O_3$
Masse moléculaire : 415,49
Point de fusion : 145 - 146°

**RMN** (DMSO, $d_6$) δ : 1,2 - 2,15 (m, 10H) ; 2,33 (t, 2H) ; 2,6 - 2,9 (m, 2H) ; 3,3 - 3,55 (m, 1H) ; 3,96 (t, 2H) ; 4,45 (d, 2H) ; 5,28 (t, 1H) ; 6,7 - 7 (m, 4H) ; 7 - 7,4 (m, 4H) ; 7,77 (s, 1H) ; 7,86 (d, 1H).

**27-2 :** Dichlorhydrate de la N-[1-[4-(4-fluorophénoxy)butyl]pipéridin-4-yl]-4H-3,1-benzoxazin-2-amine : un mélange de 1,4 g (3,37 mmoles) de composé précédent dans 7 ml d'acide chlorhydrique concentré est chauffé à 40 - 50° pendant 10 minutes puis on abandonne une nuit à 25°. Le mélange est évaporé à siccité, repris dans de l'alcool isopropylique, évaporé à siccité, trituré dans l'alcool isopropylique, filtré et recristallisé de l'isopropanol aqueux pour donner 910 mg (Rdt : 58 %) de cristaux blancs de **formule 27 :**

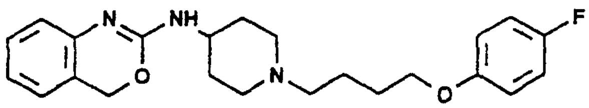
, 2 HCl (27)

Formule brute : $C_{23}H_{30}Cl_2FN_3O_2$
Masse moléculaire : 470,396
Point de fusion : 208° (dec.)
**RMN** ($D_2O$) : δ : 1,7 - 2,1 (m, 6H) ; 2,1 - 2,5 (m, 2H) ; 3 - 3,45 (m, 4H) ; 3,45 - 3,8 (m, 2H) ; 4,08 (t, 2H) ; 4 - 4,4 (m, 1H) ; 5,61 (s, 2H) ; 6,9- 7,25 (m, 5H) ; 7,25 - 7,5 (m, 3H).

__Exemple 28 :__

**Hémifumarate de la 6,7-diméthoxy-N-[1-[4-(4-fluorophénoxy)butyl]pipéridin-4-yl]-4H-3,1-benzothiazin-2-amine (28).**

**28-1 :** 1-[1-[4-(4-fluorophénoxy)butyl]pipéridin-4-yl]-3-[2-hydroxyméthyl-4,5-diméthoxy phényl)thiourée. La condensation de 2,40 g (6,6 mmoles) de 1-[4-(4-fluorophénoxy)butyl]pipéridin-4-yl isothiocyanate préparé dans l'exemple **17.1,** sur 1,10 g de 4,5-diméthoxy-2-aminobenzylalcool préparé dans l'exemple **18.1** dans 30 ml de dioxane selon le procédé décrit dans l'exemple **17.2** donne 2,37 g (Rdt : 80 %) de composé de **formule 28.1 :**

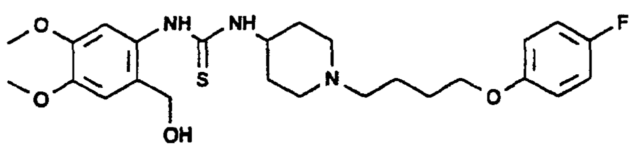
(28-1)

Formule brute : $C_{25}H_{34}FN_3O_4S$
Masse moléculaire : 491,60
Point de fusion : 160°
**RMN** (DMSO $d_6$) δ : 1,25 - 2,15 (m, 10H) ; 2,27 (t, 2H) ; 2,65 - 2,9 (m, 2H) ; 3,68 (s, 3H) ; 3,73 (s, 3H) ; 3,91 (t, 2H) ; 3,9 - 4,2 (m, 1H) ; 4,32 (d, 2H) ; 5,05 (t, 1H) ; 6,81 (s, 1H) ; 6,8 - 7,2 (m, 5H) ; 7,2 - 7,6 (m, 1H) ; 8,79 (s, 1H).

**28-2 :** Hémifumarate de la 6,7-diméthoxy-N-[1-[4-(4-fluorophénoxy)butyl]pipéridin-4-yl]-4H-3,1-benzothiazin-2-amine : la cyclisation de 2,2 g (4,47 mmoles) de composé précédent dans 10 ml d'HCl 12N à 60° selon l'exemple **17.3** donne 2,01 g (Rdt : 95 %) de cristaux beiges, qui par salification avec l'acide fumarique donnent avec un rendement de 79% le composé de **formule 28 :**

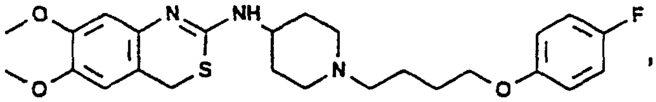
, 1/2
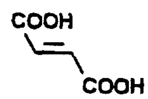
(28)

Formule brute : $C_{27}H_{34}FN_3O_5S$
Masse moléculaire : 531,63

Point de fusion : 175°
(Point de fusion de la base : 126°)
**RMN** (DMSO $d_6$) δ : 1,4 - 1,85 (m, 6); 1,85 - 2,1 (m, 2H); 2,23 (t, 2H); 2,9 - 3,15 (m, 2H) ; 3,71 (s, 3H) ; 3,73 (s, 3H) ; 3,85 (s, 2H) ; 3,96 (t, 2H) ; 6,51 (s, 1H) ; 6,55 (s, 1H) ; 6,76 (s, 1H); 6,85 - 7 (m, 2H); 7 - 7,3 (m, 2H); 9 - 13 (m, 2H).

**Exemple 29 :**

**Hémihydrate du dichlorhydrate de la 6-méthyl-N-[1-[4-(4-fluorophénoxy)butyl]pipé- ridin-4-yl]-4H-3,1-benzothiazin-2-amine (29).**

**29-1 :** 1-[1-[4-(4-fluorophénoxy)butyl]pipéridin-4-yl]-3-[2-hydroxyméthyl-4-méthyl phényl]thiourée : la réaction de 2,40 g (6,6 mmoles) de 1-[4-(4-fluorophénoxy)butyl] pipéridin4-yl isothiocyanate préparé dans l'exemple **17.1** sur 0,82 g (6 mmoles) de 5-méthyl-2-aminobenzylalcool préparé dans l'exemple **20.1** selon le procédé **17.2** donne 2,21 g (Rdt : 82 %) de cristaux beiges de **formule 29.1 :**

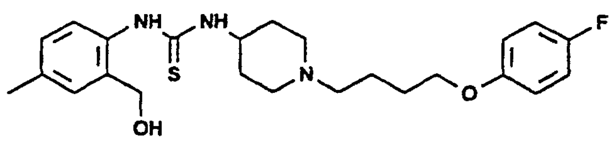

(29-1)

Formule brute : $C_{24}H_{32}FN_3O_2S$
Masse moléculaire : 445,60
Point de fusion : 130°
**RMN** (DMSO $d_6$) δ : 1,3 - 2,1 (m, 10H) ; 2,27 (s, 3H) ; 2,2 - 2,4 (m, 2H) ; 2,65 - 2,9 (m, 2H) ; 3,92 (t, 2H) ; 3,8 - 4,2 (m, 1H) ; 4,38 (d, 2H) ; 5,13 (t, 1H) ; 6,8 - 7,3 (m, 7H) ; 7,4 - 7,7 (m, 1H) ; 8,78 (s, 1H).

**29-2 :** Hémihydrate du dichlorhyrate de la 6-méthyl-N-[1-[4-(4-fluorophénoxy)butyl] pipéridin-4-yl]-4H-3,1-benzothiazin-2-amine : par cyclisation de 2,10 g (4,71 mmoles) de composé précédent dans 15 ml d'acide chlorhydrique concentré on prépare avec un rendement de 83 % le composé de **formule 29 :**

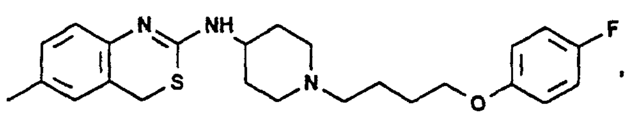

, 2 HCl , 1/2 $H_2O$ (29)

Formule brute : $C_{24}H_{32}Cl_2FN_3OS$, 1/2 $H_2O$
Masse moléculaire: 509,49
Point de fusion : 242 - 244°
**RMN** (DMSO $d_6$) δ : 1,6 - 2,4 (m, 8H) ; 2,31 (s, 3H) ; 2,9 - 3,25 (m, 4H) ; 3,3 - 3,8 (m, 3H) ; 3,98 (t, 2H) ; 4,22 (s, 2H) ; 4,5 - 4,9 (s, 1H) ; 6,9 - 7,3 (m, 6H) ; 7,6 - 7,9 (m, 1H) ; 10,3 - 11,2 (m, 2H) ; 12,7 - 13,3 (m, 1H).

**Exemple 30 :**

**Hydrogénomaléate de la N,6-diméthyl-N-[1-[4-(4-fluorophénoxy)butyl]pipéridin-4-yl]-4H-3,1-benzothiazin-2-amine (30).**

Une solution de 1,44 g (3,37 mmoles) de 6-méthyl-N-[1-[4-(4-fluorophénoxy)butyl] pipéridin-4-yl]-4H-3,1-benzothiazin-2-amine (exemple **29)** dans 10 ml de DMF sec est refroidie à 0° sous courant d'azote puis on ajoute 140 mg (3,54 mmoles) de NaH (à 60 %) par fraction ; lorsque le dégagement gazeux a cessé on ajoute 480 mg (210 µl ou 3,37 mmoles) d'iodure de méthyle. Après 30 minutes d'agitation le DMF est évaporé sous vide et le résidu est repris dans 50 ml de $CH_2Cl_2$, lavé à l'eau, à l'eau salée et séché sur sulfate de sodium. Après élimination du sel minéral et évaporation à siccité l'huile résiduelle est purifiée par "flash chromatographie" en éluant avec un mélange $CH_2Cl_2$-95/MeOH-4,5/$NH_4OH$-0,5 pour donner 1,31 g (Rdt : 88 %) de cristaux beiges. Puis on salifie avec l'acide maléique pour obtenir 1,32 g (Rdt : 70 %) de cristaux blancs de **formule 30 :**

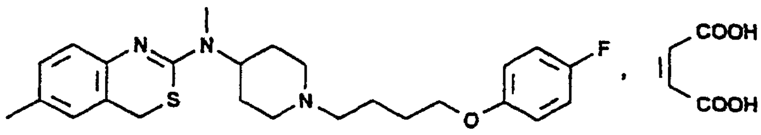

( 30 )

Formule brute : $C_{29}H_{36}FN_3O_5S$
Masse moléculaire : 557,66
Point de fusion : 162°
(Point de fusion de la base : 83°)
**RMN** (DMSO $d_6$) δ : 1,6 - 2,15 (m, 8H) ; 2,21 (s, 3H) ; 2,97 (s, 3H) ; 2,9 - 3,3 (m, 4H) ; 3,4 - 3,7 (m, 2H) ; 3,89 (s, 2H) ; 3,96 (t, 2H) ; 4,5 - 4,75 (m, 1H) ; 6,00 (s, 2H) ; 6,79 (d, 1H) ; 6,85 - 7,05 (m, 4H) ; 7,10 (t, 2H) ; *8,5 - 9,7* (m, 2H).

**Exemple 31 :**

**Dichlorhydrate de la (dl) N-méthyl-N-[1-[2-hydroxy-3-(3,4-difluorophénoxy)propyl] pipéridin-4-yl-4H-3,1-benzothiazin-2-amine (31).**

Une solution de 572 mg (3,07 mmoles) de 1-(3,4-difluorophénoxy)-2,3-époxypropane (préparé dans l'exemple **23.1)** et de 670 mg (2,56 mmoles) de N-méthyl-N-(pipéridin-4-yl)-4H-3,1-benzothiazine-2 amine base (préparée dans l'exemple **16.2)** dans 6 ml de méthanol est agitée pendant une nuit à 25°. Le méthanol est éliminé sous vide puis le résidu huileux est purifié par "flash chromatographie" en éluant avec un mélange $CH_2Cl_2$-97,4/MeOH-2,3/$NH_4OH$-0,3 pour donner 1,58 g (Rdt : 64 %) d'huile jaune pâle. La base précédente est chlorhydratée de la manière habituelle dans l'éthanol pour donner des cristaux blancs (m = 1,18 g ; Rdt : 42 %) de **formule 31 :**

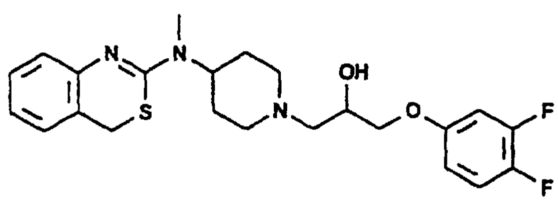

2 HCl ( 31 )

Formule brute : $C_{23}H_{29}Cl_2F_2N_3O_2S$
Masse moléculaire : 520,45
Point de fusion : 196 - 198°
**RMN** (DMSO $d_6$) δ : 1,8 - 2,3 (m, 2H) ; 2,3 - 2,75 (m, 2H) ; 3 - 3,8 (m, 10H) ; 3,99 (d, 2H) ; 4,31 (s, 2H) ; 4,28 - 5,2 (m, 2H) ; 6,7 - 6,9 (m, 1H) ; 6,9 - 7,45 (m, 5H) ; 7,5 - 7,9 (m, 1H) ; 10,4 - 11 (m, 1H) ; 11,3 - 14 (m, 1H).

**Exemple 32 :**

**Dichlorhydrate de la (S)-N-méthyl-N-[1-[2-hydroxy-3-(3,4-difluorophénoxy)propyl] pipéridin-4-yl]-4H-3,1-benzothiazin-2-amine (32)**

**32-1 :** (S)-1-(3,4-difluorophénoxy)-2,3-époxypropane: pour préparer ce glycidyl éther chiral, on adapte le procédé de J.M. Klunder et col. (*J. Med Chem* 1989, 54, 1295-1304). Une solution de 10 g (76,8 mmoles) de 3,4-difluorophénol dans 100 ml de DMF sec est refroidie à 0° puis traitée sous courant d'azote par 6,14 g (153,7 mmoles) d'hydrure de sodium à 60 %. L'agitation est poursuivie pendant 20 minutes puis le mélange est traité avec 17,5 g (76,8 mmoles) de (S) tosylate de glycidyle et l'agitation est poursuivie pendant 6 h à 25°. Le mélange réactionnel est hydrolysé dans 600 ml d'eau glacée et est extrait au toluène, lavé à l'eau, à l'eau salée et séché sur sulfate: Après élimination du sel minéral et évaporation à siccité, l'huile jaune résiduelle (m = 13,7 g ; Rdt : 96 %) est purifiée par « flash chromatographie » en éluant avec de l'éther isopropylique. On obtient 10,1 g (Rdt : 70 %) de composé de **formule 32-1** qui est utilisé tel quel dans l'étape suivante :

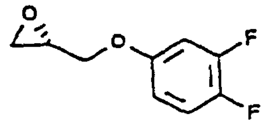

(32-1)

Formule brute : $C_9H_8F_2O_2$
Masse moléculaire : 186,16
Huile incolore.
$\alpha_D^{24°}$ = + 10,9° (c = 1 % MeOH)
**RMN** ($CDCl_3$) δ : 2,72 (dd, 1H) ; 2,89 (t, 1H) ; 3,24 - 3,45 (m, 1H) ; 3,84 (dd, 1H) ; 4,19 (dd, 1H) ; 6,5 - 6,9 (m, 2H) ; 7,03 (q, 1H)

**32-2 :** Dichlorhydrate de la (S)-N-méthyl-N-[1-[2-hydroxy-3-(3,4-difluorophénoxy)propyl]pipéridin-4-yl]-4H-3,1-benzothiazin-2-amine : une solution de 890 mg (3,4 mmoles) de N-méthyl-N(4-pipéridinyl)-4H-3,1-benzothiazin-2-amine (cf : exemple **16-2**) dans 8 ml de méthanol RP est traitée avec 698 mg (3,75 mmoles) d'époxyde chiral **32-1** et agitée pendant une nuit à 25°. La solution est évaporée à siccité et l'huile jaune résiduelle est purifiée par « flash chromatographie » en éluant avec un mélange $CH_2Cl_2$-97,5 / MeOH-2,25 / $NH_4OH$ - 0,25 pour donner après évaporation une huile jaune pâle (m = 960 mg). Cette huile est chlorhydratée avec une solution d'acide chlorhydrique dans l'éthanol, décolorée avec du noir animal et évaporée à quasi-siccité. Le chlorhydrate précipite par addition d'éther et est recristallisé dans l'alcool pour donner 610 mg (Rdt : 35 %) de cristaux crème de **formule 32 :**

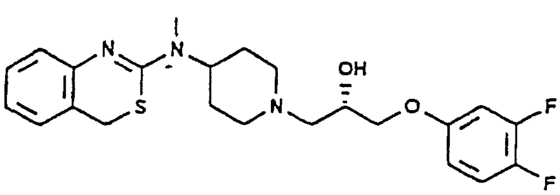

. 2 HCl (32)

Formule brute : $C_{23}H_{29}Cl_2F_2N_3O_2S$
Masse moléculaire : 520,46
α D = - 10,8 ° (C = 1 %, MeOH)

## EXPERIMENTATIONS BIOLOGIQUES

Les composés de la présente invention de formule I et leurs sels thérapeutiquement acceptables présentent d'intéressantes propriétés pharmacologiques.
Ces dérivés sont actifs au niveau du cardiomyocyte en inhibant la contracture diastolique induite par la vératrine au niveau de l'oreillette gauche isolée du rat.
Ces composés sont aussi actifs *in vivo* lors de l'ischémie reperfusion chez le lapin anesthésié : ils inhibent les perturbations électriques de l'ECG provoquées par l'ischémie reperfusion sans effet hémodynamique notable et ne sont pas dépresseurs cardiaques.
De tels composés sont utiles à titre préventif ou curatif dans le traitement des coronaropathies, de l'ischémie cardiaque et cérébrale sous toutes leurs formes et dans le traitement de l'athérosclérose.

### 1°) Etude pharmacologique:

Les expérimentations auxquelles ont été soumises les molécules chimiques objet de la présente invention ont permis de mettre en évidence une intéressante activité sur le système cardiovasculaire à la fois sur des tests **"in vitro" et "in vivo".**

### a) Action "in vitro"

L'inhibition de la contracture à la vératrine de l'oreillette gauche isolée de rat a été réalisée selon la technique de Le **Grand et coll.** (*Naunyn - Schmiedeberg's Arch Pharmacol* (1993) **348** p 184 - 190). Les résultats sont portés dans le tableau ci-après où les $CI_{50}$ sont exprimées en micromoles pour certains composés à titre d'exemples non

limitatifs :

| Composé | Exemple n° 1 | R 56865* | Exemple n° 15 | Sabeluzole** |
|---|---|---|---|---|
| $CI_{50}$ mmoles | 0,15 | 0,25 | 0,32 | 5,1 |

correspondent à la *N-(1-(4-(4-fluorophénoxy)butyl)pipéridin-4-yl)-N-méthyl-2-benzothiazolamine et à la **N-(1-(2-hydroxy-3-(4-fluorophénoxy)propyl)pipéridin-4-yl)-N-méthyl-2-benzothiazolamine cités dans le brevet EP 0 184 257 et en développement.
Les composés de la présente invention ne sont pas inotropes négatifs à la dose de 10 µM.

**b) Activité "in vivo"**

Les composés de la présente invention sont aussi actifs par voie veineuse dans le test de **l'ischémie reperfusion chez le lapin anesthésié** selon la méthode de **Verscheure et coll.** (*Fundam Clin Pharmacol* (1993) 7, 385). Les résultats pour les composés des **exemples 1, 2, 11, 15** sont donnés dans le tableau ci-dessous à titre d'exemple non limitatif :

| n° produit ou témoin | Dose mg/kg iv | % Inhibition segment ST | Nombre d'arythmies sous reperfusion | % variation fréquence cardiaque | % variation pression artérielle |
|---|---|---|---|---|---|
| 1<br>2<br>11 | 0,16<br>0,16<br>0,16 | 76<br>76<br>71 | 3/5<br>1/5<br>1/5 | + 3<br>- 3<br>- 1 | +6<br>+ 4<br>+ 3 |
| Atenolol | 0,16 | 64 | 4/6 | -13 | - 9 |
| Diltiazem | 0,16 | 61 | 0/6 | - 5 | -27 |
| 15 | 0,16 | 90 | 1/5 | 0 | + 5 |
| Sabeluzole | 0,16 | 84 | 1/5 | - 5 | 2 |

**2°) Applications thérapeutiques :**

Les composés de la présente invention et leurs sels thérapeutiquement acceptables sont utiles comme médicaments.
Ces composés sont plus particulièrement adaptés en cardiologie dans le traitement prophylactique des maladies cardiovasculaires comme :

* l'ischémie du myocarde et les coronaropathies et plus particulièrement dans les crises :
  - d'angor stable chronique,
  - d'angor instable et de Prinzmetal,
* l'ischémie silencieuse, et dans la prévention des réocclusions, resténoses et le réinfarctus.
* l'ischémie cérébrale et plus précisément dans :
  - l'accident vasculaire cérébral,
  - l'attaque ischémique transitoire,
  - les maladies neurodégénératives,
* et enfin dans l'athérosclérose.

L'administration de ces composés peut être réalisée par voie orale, parentérale ou rectale ; chaque dose est constituée d'un adjuvant inerte favorisant la préparation, l'absorption du médicament et du principe actif pouvant être aussi associés à un autre. Ces médicaments peuvent se présenter sous forme solide (comprimés ou gélules) ou liquide à réaliser au moment de l'emploi (suspensions, émulsions, sirops, solutions ou autres) ou suppositoires. L'administration

du principe actif se fait à la dose moyenne comprise entre 0,1 et 10 mg/kg du poids du corps.
Deux préparations sont données à titre d'exemple non limitatifs. Les ingrédients ainsi que d'autres thérapeutiquement acceptables peuvent être introduits en d'autres proportions sans modifier la portée de l'invention.

**Exemple 33 :** Solution injectable à réaliser au moment de l'emploi.

| | |
|---|---|
| 1°) Un flacon stérile pour préparation injectable en verre inactinique renfermant : | |
| Hydrogénomaléate de la N-méthyl-N-[1-[4-(3,4-difluorophénoxy)butyl] pipéridin-4-yl]-4H-3,1-Benzothiazin-2-amine | 10 mg |
| 2°) Une ampoule de solvant en verre, stérile renfermant : | |
| Propylène glycol | 100 mg |
| Dextrose anhydre | 50 mg |
| Eau distillée stérile q.s.p. | 2 ml |

**Exemple 34 :** Comprimés.

| | |
|---|---|
| Hydrogénomaléate de la N-méthyl-N-[1-[4-(3,4-difluorophénoxy)butyl]pipéridin-4-yl]-4H-3,1-Benzothiazin-2-amine | 40 mg |
| Lactose hydrate | 100 mg |
| Cellulose microcristalline | 25 mg |
| Carboxyméthyl cellulose Sel de Na | 3 mg |
| Stéarate de magnésium | 2 mg |
| Amidon de maïs | 20 mg |
| Talc | 3 mg |
| Polyvinyl pyrrolidone | 7 mg |
| Poids total | 200 mg |

Comprimés sécables à conserver à l'abri de la chaleur et de l'humidité.

<u>**Exemple 35 :**</u>

**Hydrogénomaléate de la (S)-N-méthyl-N-[1-[2-hydroxy-3-(3,4-difluorophénoxy) propyl] piperidin-4-yl]-4H-3,1-benzothiazin-2-amine (35).**

En opérant comme décrit dans l'exemple 32 mais en salifiant avec un équivalent d'acide maléique, on prépare avec un rendement de 55 % des cristaux blanc cassé de **formule 35 :**

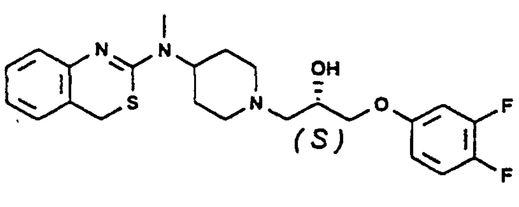

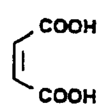

**( 35 )**

Formule brute : $C_{27} H_{31} F_2 N_3 D_6 S$
Masse moléculaire : 563,61
Point de fusion : 125-127°
(Point de fusion de la base : 89-90°)
$\alpha_D^{26°}$ = - 10,82° (c = 1 % MeOH)
**RMN** ($CDCl_3$) δ : 1,9-2,1 (m, 2H) ; 2,2-2,5 (m, 2H) ; 2,9-3,4 (m, 4H) ; 3,1 (s, 3H) ; 3,7-4,2 (m, 6H) ; 4,4-4,6 (m, 1H) ;

4,9-5,1 (m, 1H) ; 6,3 (s, 2H) ; 6,6-6,9 (m, 2H) ; 7-7,4 (m, 5H) ; 8-13 (m, 3H).

**Exemple 36 :**

**Hydrogénomaléate de la N-méthyl-N-[1-[4-fluorophénylthio) butyl] pipéridin-4-yl]-4H-3,1-benzothiazin-2-amine (36).**

Par condensation de 551 mg (2,09 m moles) de 4-fluoro-1-(4-bromobutylthio) benzène (préparé selon **l'exemple 22** avec un rendement de 41 %) sur 547 mg de N-méthyl-N-(4-piperidinyl)-4H-3,1-benzothiazin-2-amine obtenue dans **l'exemple 16.2** selon le protocole décrit dans **l'exemple 16.3** on prépare avec un rendement de 51 % le composé suivant de **formule 36 :**

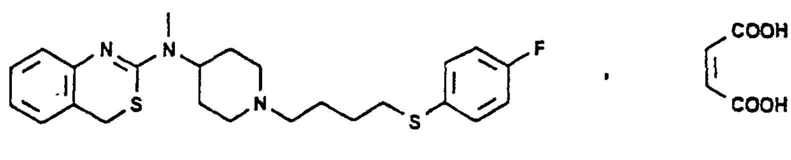

(36)

Fomule brute : $C_{28}H_{34}FN_3O_4S_2$
Masse moléculaire : 554,69
Point de fusion : 126-128°
**RMN** (DMSO $d_6$) $\delta$ : 1,5-1,8 (m, 2H) ; 1,8-2,1 (m, 4H) ; 2,2-2,5 (m, 2H) ; 2,5-3,2 (m, 6H) ; 3,08 (s, 3H) ; 2,65 (d, 2H) ; 3,88 (s, 2H) ; 4,8-5,1 (m, 1H) ; 6,3 (s, 2H) ; 6,9-7,2 (m, 5H) ; 7,2-7,5 (m, 3H) ; 9-12 (m, 2H).

**Exemple 37 :**

**Hydrogénofumarate de la N-méthyl-N-[1-[5-(4-fluorophénoxyl) pentyl] pipéridin-4-yl]-4H-3,1-benzothiazin-2-amine (37).**

En opérant comme décrit dans **l'exemple 16.3** mais en partant de 520 mg de 4-fluoro-4-(5-bromopentyl) benzène et de 520 mg de N-méthyl-N-[4-pipéridinyl]-4H-3,1-benzothiazin-2-amine on obtient après salification avec de l'acide fumarique des cristaux blancs (Rdt : 69 %) de **formule 37 :**

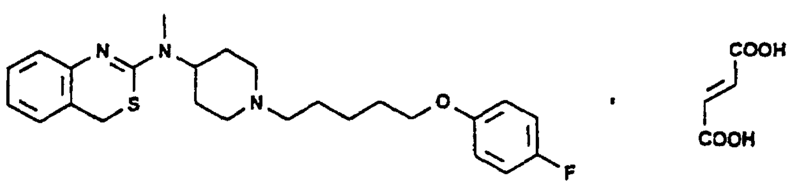

(37)

Formule brute : $C_{29}H_{36}FN_3O_5S$
Masse moléculaire : 557,66
Point de fusion: 170-172°
**RMN :** (DMSO $d_6$) $\delta$ : 1,3-1,70 (m, 6H) ; 1,75-2 (m, 2H) ; 2,3 (t, 2H) ; 2,4-2,6 (m, 2H) ; 2,99 (s, 3H) ; 3,11 (d, 2H) ; 3,8-4 (m, 2H) ; 3,92 (s, 2H) ; 4,2-4,5 (m, 1H) ; 6,55 (s, 2H) ; 6,8-7 (m, 4H) ; 7-7,2 (m, 4H) ; 8,5-12,5 (m, 2H).

**Exemple 38 :**

**Hydrogénofumarate de la N-méthyl-N-[1-[3-(4-fluorophénoxy) propyl] pipéridin-4-yl]-4H-3,1-benzothiazin-2-amine (38).**

La condensation de 600 mg (2,63 m moles) de 1-bromométhyl phénylisothiocyanate sur 940 mg (2,63 m moles) de N-méthyl-1-[3-(3,4-difluorophénoxy) propyl] pipéridin-4-yl amine selon le procédé de **l'exemple 1** conduit au composé de **formule 38** avec un redement de 61 % :

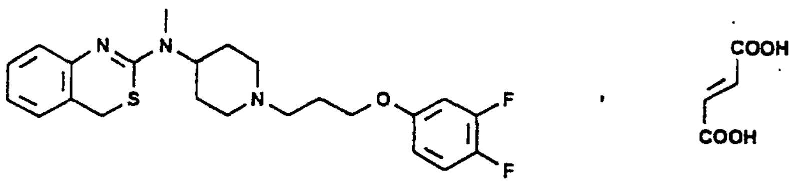

( 38 )

Formule brute : $C_{27}\ H_{31}\ F_2\ N_3\ O_5\ S$
Masse moléculaire : 547,61
Point de fusion : 171-173°
**RMN** (DMSO $d_6$) δ : 1,5-1,7 (m, 2H) ; 1,7-2,1 (m, 4H) ; 2,26 (t, 2H) ; 2,6 (t, 2H) ; 3,01 (s, 3H) ; 3-3,3 (m, 2H) ; 3,92 (s, 2H) ; 3,99 (t, 2H) ; 4,2-4,5 (m, 1H) ; 6,57 (m, 2H); 6,65-7,5 (m, 1H) ; 6,92 (t, 2H) ; 6,95-7,2 (m, 3H ) ; 3,32 (q, 1H) ; 8,5-12 (m, 2H).

<u>**Exemple 39 :**</u>

**Hydrogénofumarate de la N-méthyl-N-[1-[6-(4-fluorophénoxy) hexyl] pipéridin-4-yl]-4H-3,1-benzothiazin-2-amine (39).**

En utilisant le procédé de **l'exemle 16.3** mais en partant de 550 mg de 4-fluoro-1- (6-bromohexyloxy) benzène et de 520 mg (2 m moles) de N-méthyl-N-[4-pipéridinyl]-4H-3,1-benzothiazin-2-amine on obtient avec un rendement de 68 % le composé de **formule 39 :**

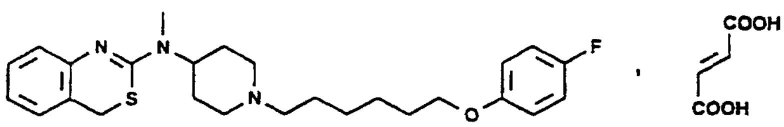

( 39 )

Formule brute : $C_{30}\ H_{36}\ F\ N_3\ O_5\ S$
Masse moléculaire : 571,68
Point de fusion : 176-178°
**RMN** (DMSO $d_6$) δ : 1,2-2,1 (m, 10H) ; 2,34 (t, 2H) ; 2,45-2,55 (m, 2H) ; 3,03 (s, 3H) ; 3,05-3,3 (s, 2H) ; 3,8-4,1 (m, 2H) ; 3,95 (s, 2H) ; 4,25-4,6 (m, 1H) ; 6,58 (s, 2H) ; 6,85-7,05 (m, 4H) ; 7,1-7,3 (m, 4H) ; 9-12 (m, 2H).

<u>**Exemple 40 :**</u>

**Hydrogénofumarate de la N-méthyl-N-[1-[4-(3,4-diméthoxyphénoxy) butyl] pipéridin-4-yl]-4H-3,1-benzothiazin-2-amine (40).**

Par condensation de 720 mg (2,5 m moles) de 3,4 -diméthoxy-1-(4-bromobutoxy) benzène sur 650 mg (2,5 m moles) de N-méthyl-N-[4-pipéridinyl]-4H-3,1-benzothiazin-2-amine selon **l'exemple 16.3** on obtient avec un rendement de 75 % le composé de **formule 40 :**

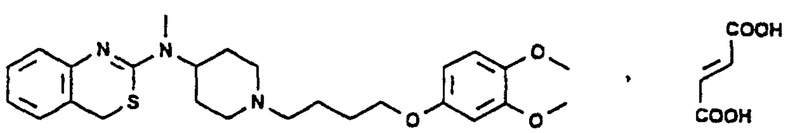

( 40 )

Formule brute : $C_{30}\ H_{39}\ N_3\ O_7\ S$
Masse moléculaire : 585,69
Point de fusion : 172-175°

**RMN** (DMSO $d_6$) δ : 1,5-1,7 (m, 6H) ; 1,7-2,1 (m, 2H) ; 2,26 (t, 2H) ; 2,4-2,65 (m, 2H) ; 3 (s, 3H) ; 3,05-3,2( m, 2H) ; 3,66 (s, 3H) ; 3,72 (s, 3H) ; 3,8-4 (m, 2H) ; 3,92 (s, 2H) ; 4,2-4,5 (m, 1H); 6,4 (dd, 1H) ; 6,54 (d, 1H) ; 6,57 (s, 2H) ; 6,8-7 (m, 3H) ; 7,05-7,25 (m, 2H); 8,5-12 (m, 2H).

**Exemple 41 :**

**Hydrogénofumarate de la N-méthyl-N-[1-[4-(2-méthoxyphénoxy) butyl)] pipéridin-4-yl]-4H-3,1-benzothiazin-2-amine (41).**

La réaction de 650 mg (2,5 m moles) de 2-méthoxy-1-(4-bromobutoxy) benzène avec 656 mg (2,5 m moles) de N-méthyl-N-[4-pipéridinyl]-4H-3,1-benzothiazin-2-amine selon le procédé de **l'exemple 16.3** fournit avec un rendement de 69 % des cristaux beiges de **formule 41 :**

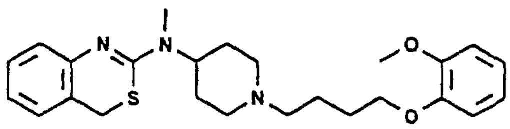 , 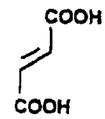 ( 41 )

Formule brute : $C_{29} H_{37} N_3 O_6 S$
Masse moléculaire : 555,66
Point de fusion : 172°
**RMN** (DMSO $d_6$) δ : 1,5-2,1 (m, 8H) ; 2,31 (t, 2H) ; 2,58 (t, 2H) ; 3,02 (s, 3H) ; 3-3,3 (m, 2H) ; 3,75 (s, 3H) ; 3,94 (s, 2H) ; 3,9-4,05 (m, 2H); 4,25-4,5 (m, 1H) ; 6,58 (s, 2H) ; 6,8-7,05 (m, 6H) ; 7,05-7,25 (m, 2H) ; 8-12 (m, 2H).

**Exemple 42 :**

**Hydrogénofumarate de la N-méthyl-N-[1-[4-(2,3-diméthoxyphénoxy)butyl)] pipéri-din-4-yl]-4H-3,1-benzothiazin-2-amine (42).**

La N-alcoylation de 520 mg (2 m moles) de N-méthyl-N-[4-pipéridinyl]-4H-3,1-benzothiazin-2-amine par 580 mg (2 m moles) de 2,3-diméthoxy-1-(4-bromobutoxy) benzène selon le protocole de **l'exemple 16.3** conduit au composé de **formule 42** avec un rendement de 61 %.

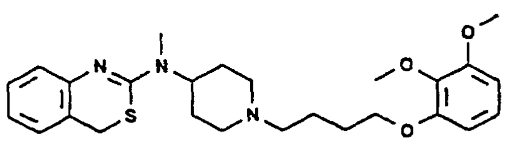 , 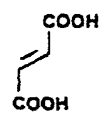 ( 42 )

Formule brute : $C_{30} H_{39} N_3 O_7 S$
Masse moléculaire : 585,70
Point de fusion : 146°
**RMN** (DMSO $d_6$) δ : 1,5-2,05 (m, 8H) ; 2,27 (t, 2H) ; 2,56 (t, 2H) ; 3 (s, 3H) ; 3-3,2 (m, 2H) ; 3,65 (s, 3H) ; 3,74 (s, 3H) ; 3,92 (s, 2H) ; 3,97 (t, 2H) ; 4,25-4,5 (m, 1H) ; 6,56 (s, 2H) ; 6,57-6,7 (m, 2H) ; 6,8-7 (m, 3H) ; 7,02-7,2 (m, 2H) ; 8,5-12 (m, 2H).

**Exemple 43 :**

**Hydrogénofumarate de la N-méthyl-N-[1[4-(3,5-diméthoxyphénoxy) butyl] pipéridin-4-yl]-4H-3,1-benzothiazin-2-amine (43).**

En condensant 775 mg (2,7 m moles) de 3,5-diméthoxy-1-(4-bromobutoxy) benzène sur 700 mg (2,7 m moles) de N-méthyl-N-[4-pipéridinyl]-4H-3,1-benzothiazin-2-amine en utilisant le mode opératoire décrit dans **l'exemple 16.3** on prépare avec un rendement de 58 % de **formule 43 :**

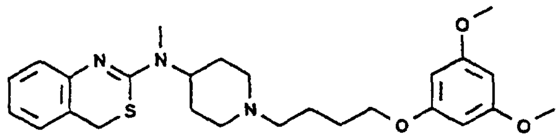 , 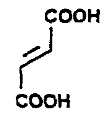 **( 43 )**

Formule brute : $C_{30}$ $H_{39}$ $N_3$ $O_7$ S
Masse moléculaire : 585,70
Point de fusion : 173-174°
**RMN** (DMSO $d_6$) δ : 1,5-2,1 (m, 8H) ; 2,71 (t, 2H) ; 2,53 (t, 2H) ; 2,99 (s, 3H) ; 3-3,2 (m, 2H) ; 3,68 (s, 6H) ; 3,92 (s, 2H) ; 3,8-4 (m, 2H) ; 4,25-4,5 (m, 1H) ; 6,06 (s, 3H) ; 6,55 (s, 2H) ; 6,90 (t, 2H) ; 7,07-7,2 (m, 2H) ; 8-12 (m, 2H).

<u>**Exemple 44 :**</u>

**Hydrogénofumarate de la N-méthyl-N-[1-[4-(2,6-diméthoxyphénoxy) butyl] pipéridin-4-yl]-4H-3,1-benzothiazin-2-amine (44).**

La condensation de 700 mg (2,7 m moles) de N-méthyl-N-[4-pipéridinyl]-4H-3,1-benzothiazin-2-amine sur 775 mg (2,7 m moles) de 2,6-diméthoxy-1-(4-bromobutyl) benzène selon le protocole de **l'exemple 16.3** donne des cristaux blanc cassé (Rdt: 63 %) de **formule 44 :**

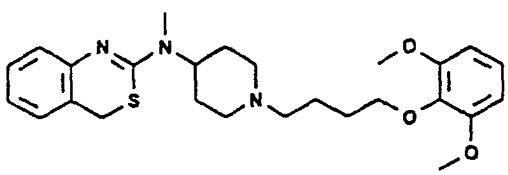 , 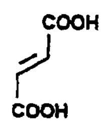 **( 44 )**

Formule brute : $C_{30}$ $H_{39}$ $N_3$ $O_3$ S
Masse moléculaire : 585,70
Point de fusion : 155-156°
**RMN** (DMSO $d_6$) δ : 1,5-1,8 (m, 6H) ; 1,8-2,1 (m, 2H) ; 2,35 (t, 2H) ; 2,6 (t, 2H) ; 3,02 (s, 3H) ; 3,07-3,25 (m, 2H) ; 3,76 (s, 6H) ; 3,9 (t, 2H) ; 3,95 (s, 2H) ; 4,27-4,55 (m, 1H) ; 6,57 (s, 2H) ; 6,66 (d, 2H) ; 6,85-7,05 (m, 3H) ; 7,05-7,23 (m, 2H) ; 8-12 (m, 2H).

<u>**Exemple 45 :**</u>

**Hydrogénofumarate de la N-méthyl-N-[1-[4-(2,6-difluorophénoxy) butyl] pipéridin-4-yl]-4H-3,1-benzothiazin-2-amine (45).**

Par réaction de 811 mg (3,06 m moles) de 2,6-difluoro-1-(4-bromobutoxy) benzène avec 800 mg (3,06 m moles) de N-méthyl-N-[4-pipéridinyl]-4H-3,1-benzothiazin-2-amine comme décrit dans **l'exemple 16.3** on obtient des cristaux beiges (Rdt : 59 %) de **formule 45 :**

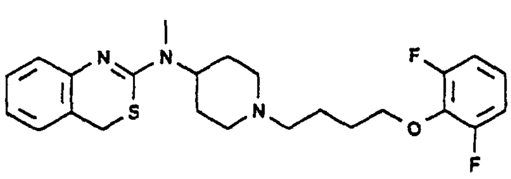 , 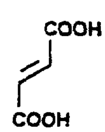 **( 45 )**

Formule brute : $C_{28}$ $H_{33}$ $F_2$ $N_3$ $O_5$ S
Masse moléculaire : 561,63
Point de fusion : 172-173°
**RMN** (DMSO $d_6$) δ : 1,5-2,05 (m, 8H) ; 2,27 (t, 2H) ; 2,54 (t, 2H) ; 3,01(s, 3H) ; 3-3,2 (m, 2H) ; 3,94 (s, 2H) ; 4,11

(t, 2H) ; 4,2-4,5 (m, 1H) ; 6,58 (s, 2H) ; 6,93 (t, 2H); 7-7,25 (m, 5H) ; 8-12 (m, 2H).

**Exemple 46 :**

**Hydrogénofumarate de la N-méthyl-N-[1-[5-(3,4-difluorophénoxy) pentyl] pipéridin-4-yl]-4H-3,1-benzothiazin-2-amine (46).**

En utilisant le mode opératoire de l'**exemple 16.3** mais en partant de 800 mg de 3,4-difluoro-1-(5-bromopentyloxy) benzène et de 800 mg de N-méthyl-N-[4-pipéridinyl]-4H-3,1-benzothiazine on prépare après salification avec l'acide furnarique le composé de **formule 46** (Rdt : 48 %) :

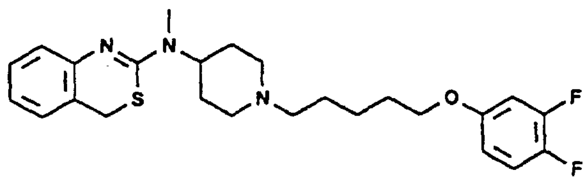
,
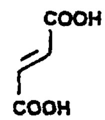
**( 46 )**

Formule brute : $C_{29} H_{35} F_2 N_3 O_5 S$
Masse moléculaire : 575,66
Point de fusion : 174-175°
**RMN** (DMSO $d_6$) δ : 1,3-2,05 (m, 10H) ; 2,32 (t, 2H) ; 2,4-2,62 (m, 2H) ; 3,02 (s, 3H) ; 3,05-3,22 (m, 2H) ; 3,94 (s, 2H) ; 3,96 (t, 2H) ; 4,25-4,5 (m, 1H) ; 6,58 (s, 2H) ; 6,65-6,85 (m, 1H) ; 6,85-7,45 (m, 6H) ; 8,5-12 (m, 2H).

**Exemple 47 :**

**Hydrogénofumarate de la N-méthyl-N-[1-[4-(3,5-difluorophénoxy) butyl] pipéridin-4-yl]-4H-3,1-benzothiazin-2-amine (47).**

La condensation de 800 mg (~ 3 m moles) de N-méthyl-N-[4-pipéridinyl]-4H-3,1-benzothiazin-2-amine sur 820 mg (~ 3 m moles) de 3,5-difluoro-1-(4-bromobutoxy) benzène selon le protocole de **l'exemple 16.3** fournit 1,1 g de cristaux blancs (Rdt : 66 %) de **formule 47 :**

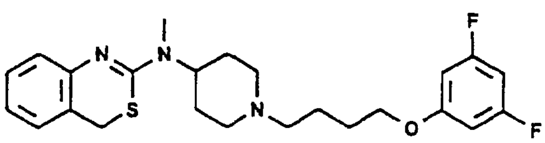
,
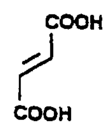
**( 47 )**

Formule brute : $C_{28} H_{33} F_2 N_3 O_5 S$
Masse moléculaire : 561,63
Point de fusion: 187-188°
**RMN** (DMSO $d_6$) δ : 1,5-2,05 (m, 8H) ; 2,32 (t, 2H) ; 2,58 (t, 2H) ; 3,02 (s, 3H) ; 3,03-3,23 (m, 2H) ; 3,94 (s, 2H) ; 4,02 (t, 2H) ; 4,25-4,5 (m, 1H) ; 6,6 (s, 2H) ; 6,63-6,85 (m, 3H) ; 6,94 (t, 2H) ; 7,05-7,25 (m, 2H) ; 8,5-12 (m, 2H).

**Exemple 48 :**

**Hydrogénofumarate de la N-méthyl-N-[1-[4-(2,4-difluorophénoxy) butyl] pipéridin-4-yl]-4H-3,1-benzothiazin-2-amine (48).**

La réaction de 1 g de 2,4-difluoro-1-(4-bromobutoxy) benzène avec 990 mg de N-méthyl-N-[4-pipéridinyl]-4H-3,1-benzothiazin-2-amine dans les conditions de **l'exemple 16.3** donne 1,3 g (Rdt : 62 %) de cristaux blancs de **formule 48 :**

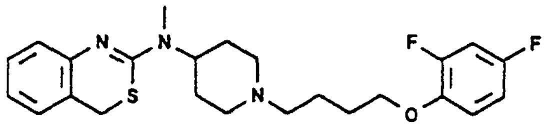 , 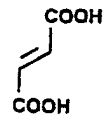 ( 48 )

Formule brute : $C_{28}H_{33}F_2N_3O_5S$
Masse moléculaire : 561,63
Point de fusion : 186°
**RMN** (DMSO $d_6$) δ : 1,5-2,1 (m, 8H) ; 2,34 (t, 2H) ; 2,58 (t, 2H) ; 2,99 (s, 3H) ; 3,02-3,24 (m, 2H) ; 3,92 (s, 2H) ; 4,02 (t, 2H) ; 4,25-4,5 (m, 1H) ; 6,55 (s, 2H) ; 6,75-7,05 (m, 3H) ; 7,05-7,35 (m, 4H) ; 8,5-12 (m, 2H)

**Exemple 49 :**

**Hydrogénofumarate de la N-méthyl-N-[1-[4-(2,5-difluorophénoxy) butyl] pipéridin-4-yl]-4H-3,1-benzothiazin-2-amine (49).**

En opérant comme décrit dans **l'exemple 16.3** mais en partant de 436 mg (1,64 m moles) de 2,5-difluoro-1-(4-bromobutoxy) benzène on prépare avec un rendement de 55 % le composé de **formule 49 :**

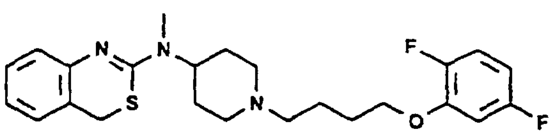 , 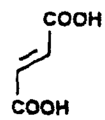 ( 49 )

Formule brute : $C_{28}H_{33}F_2N_3O_5S$
Masse moléculaire : 561,63
Point de fusion : 179°
**RMN** (DMSO $d_6$) δ : 1,5-2 (m, 8H) ; 2,14 (t, 2H); 2,35-2,55 (m, 2H) ; 3,02 (s, 3H) ; 3-3,15 (m, 2H) ; 3,94 (s, 2H) ; 4,08 (t, 2H) ; 4,15-4,45 (m, 1H) ; 6,58 (s, 2H) ; 6,65-6,82 (m, 1H) ; 6,82-7 (m, 2H) ; 7-7,35 (m, 4H) ; 8,5-12 (m, 2H).

**Exemple 50 :**

**Hydrogénofumarate de la N-méthyl-N-[1-[4-(3-fluoro-4-chlorophénoxy) butyl] pipéridin-4-yl]-4H-3,1-benzothiazin-2-amine (50).**

L'action de 540 mg (1,9 m moles) de 3-fluoro-4-chloro-1-(4-bromobutoxy) benzène sur 500 mg (1,9 m moles) de N-méthyl-N-[4-pipéridinyl]-4H-3,1-benzothiazin-2-amine selon le descriptif de l'**exemple 16.3** permet d'accéder avec un rendement de 53 % au composé de **formule 50 :**

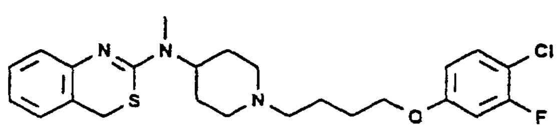 , 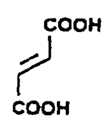 ( 50 )

Formule brute : $C_{28}H_{33}ClFN_3O_5S$
Masse moléculaire : 578,08
Point de fusion : 184°
**RMN** (DMSO $d_6$) δ : 1,5-2 (m, 8H) ; 2,16 (t, 2H) ; 2,3-2,52 (m, 2H) ; 3,01 (s, 3H) ; 2,95-3,15 (m, 2H) ; 3,93 (s, 2H) ; 4 (t, 2H) ; 4,2-4,45 (m, 1H) ; 6,59 (s, 2H) ; 6,65-7,25 (m, 6H) ; 7,44 (t, 1H) ; 8-13 (m, 2H).

**Exemple 51 :**

**Hydrogénofumarate de la N-méthyl-N-[1-[4-(2,3-difluorophénoxy) butyl] pipéridin-4-yl]-4H-3,1-benzothiazin-2-amine (51).**

La condensation de 610 mg (2,3 m moles) de 2,3-difluoro-1-(4-bromobutoxy) benzène sur 600 mg (2,3 m moles) de N-méthyl-N-[4-pipéridinyl]-4H-3,1-benzothiazin-2-amine dans les conditions de **l'exemple 16.3** donne 970 mg (Rdt : 58 %) de cristaux blancs de **formule 51 :**

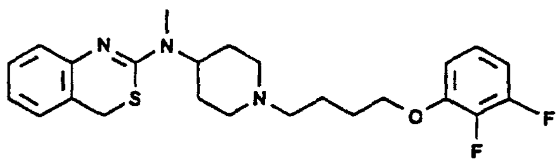 , 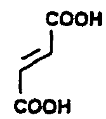 **( 51 )**

Formule brute : $C_{28} H_{33} F_2 N_3 O_5 S$
Masse moléculaire : 561,63
Point de fusion : 174°
**RMN** (DMSO $d_6$) $\delta$ : 1,45-2 (m, 8H) ; 2,15 (t, 2H) ; 2,3-2,55 (m, 2H) ; 3,01 (s, 3H) ; 3-3,15 (m, 2H) ; 3,94 (s, 2H) ; 4,11 (t, 2H) ; 4,2-4,45 (m, 1H); 6,58 (s, 2H) ; 6,8-7,25 (m, 7H) ; 8-12 (m, 3H).

**Exemple 52 :**

**Hydrogénofumarate de la N-méthyl-N-[1-[4-(3-chloro-4-fluorophénoxy) butyl] pipé-ridin-4-yl]-4H-3,1-benzothiazin-2-amine (52).**

La N-alcoyaltion de 754 mg (2,68 m moles) de N-méthyl-N-(4-pipéridinyl)-4H-3,1-benzothiazin-2-amine par 700 mg (2,68 m moles) de 3-chloro-4-fluoro-1-(4-bromobutoxy) benzène selon le procédé de l'**exemple 16.3** donne 1,21 g (Rdt : 78 %) de cristaux blancs de **formule 52 :**

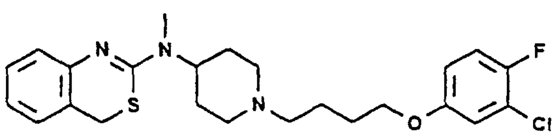 , 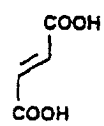 **( 52 )**

Formule brute : $C_{28} H_{33} Cl F N_3 O_5 S$
Masse moléculaire : 578,08
Point de fusion : 176°
**RMN** (DMSO $d_6$) $\delta$ : 1,5-2,1 (m, 8H) ; 2,3 (t, 2H) ; 2,56 (t, 2H) ; 3 (s, 3H) ; 3,05-3,2 (m, 2H) ; 3,92 (s, 2H) ; 3,98 (t, 2H) ; 4,25-4,5 (t, 1H) ; 6,56 (s, 2H) ; 6,82-7,02 (m, 3H); 7,05-7,21 (m, 3H) ; 7,31 (t, 1H) ; 8-13 (m, 2H).

**Exemple 53 :**

**Hydrogénofumarate de la N-méthyl-N-[1-[4-(3-fluorophénoxy) butyl] pipéridin-4-yl]-4H-3,1-benzothiazin-2-amine (53).**

La condensation de 600 mg (2,3 m moles) de N-méthyl-N-(4-pipéridinyl)4H-3,1-benzothiazin-2-amine sur 570 mg (2,3 m moles) de 3-fluoro-1-(4-bromobutoxy) benzène dans les conditions de **l'exemple 16.3** donne avec un rendement de 58 % le composé de **formule 53 :**

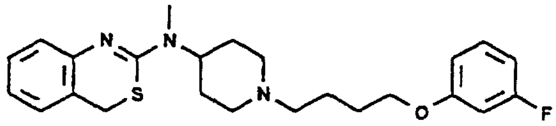 , 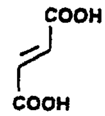 ( 53 )

Formule brute : $C_{28}H_{34}FN_3O_5S$
Masse moléculaire : 543,63
Point de fusion : 184°
**RMN** (DMSO $d_6$) $\delta$ : 1,5-2,05 (m, 8H) ; 2,26 (t, 2H) ; 2,53 (t, 2H) ; 3 (s, 3H) ; 3-3,22 (m, 2H) ; 3,92 (s, 2H) ; 3,98 (t, 2H) ; 4,25-4,5 (m, 1H) ; 6,56 (s, 2H) ; 6,65-7 (m, 4H); 7-7,4 (m, 4H); 8-13 (m, 2H).

**<u>Exemple 54 :</u>**

**Hydrogénofumarate de la N-méthyl-N-[1-[4-(3-méthoxyphénoxy) butyl] pipéridin-4-yl]-4H-3,1-benzothiazin-2-amine (54).**

En utilisant le protocole de l'**exemple 16.3** mais en partant de 655 mg (2,5 m moles) de 3-méthoxy-1-(4-bromobutoxy) benzène on prépare 980 mg (Rdt : 76 %) de poudre blanche de **formule 54 :**

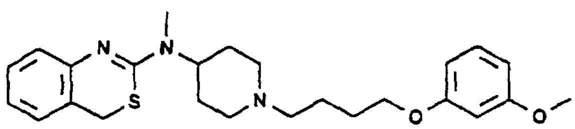 , 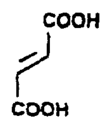 ( 54 )

Formule brute : $C_{29}H_{37}N_3O_6S$
Masse moléculaire : 555,67
Point de fusion : 165-166°
**RMN** (DMSO $d_6$) $\delta$ : 1,5-1,75 (m, 6H) ; 1,75-2,1 (m, 2H) ; 2,33 (t, 2H) ; 2,58 (t, 2H) ; 3 (s, 3H) ; 3,05-3,22 (m, 2H) ; 3,71 (s, 3H) ; 3,93 (s, 2H) ; 3,9-4,02 (m, 2H) ; 4,25-4,5 (m, 1H); 6,4-6,6 (m, 5H) ; 6,8-7 (m, 2H) ; 7-7,25 (m, 3H) ; 8-13 (m, 2H).

**<u>Exemple 55 :</u>**

**Hydrogénofumarate de la N-méthyl-N-[1-[4-(2-fluorophénoxy) butyl] pipéridin-4-yl]-4H-3,1-benzothiazin-2-amine (55).**

L'application du **protocole 16.3** au 2-fluoro-1-(4-bromobutoxy) benzène (570 mg ; 2,3 m moles) permet de préparer 810 mg (Rdt : 65 %) de composé blanc de **formule 55 :**

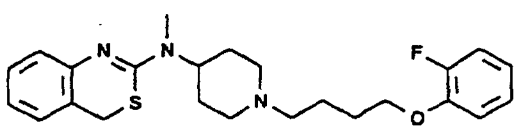 , 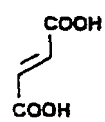 ( 55 )

Formule brute : $C_{28}H_{34}FN_3O_5S$
Masse moléculaire : 543,63
Point de fusion : 187°
**RMN** (DMSO $d_6$) $\delta$ : 1,5-2 (m, 8H) ; 2,18 (t, 2H) ; 2,35-2,6 (m, 2H) ; 2,9-3,15 (m, 2H) ; 2,99 (s, 3H) ; 3,92 (s, 2H) ; 4,05 (t, 2H) ; 4,25-4,5 (m, 1H) ; 6,58 (s, 2H) ; 6,82-7 (m, 3H) ; 7-7,3 (m, 5H) ; 8-13 (m, 2H).

**Exemple 56 :**

**Hydrogénofumarate de la N-méthyl-N-[1-[4-(2-méthoxy-4-chlorophénoxy) butyl] pipéridin-4-yl]-4H-3,1-benzothiazin-2-amine (56).**

En partant de 600 mg (2,3 m moles) de N-méthyl-N-(4-pipéridinyl)-4H-1,3-benzothiazin-2-amine et en les condensant sur 675 mg (2,3 m moles) de 2-méthoxy-4-chloro-1-(4-bromobutoxy) benzène selon le procédé décrit dans **l'exemple 16.3** on prépare avec un rendement de 58 % le composé de **formule 56 :**

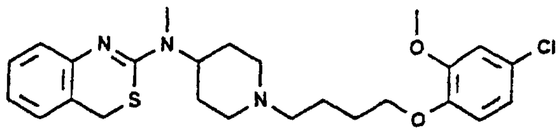
,
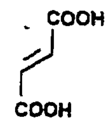
**( 56 )**

Formule brute : $C_{29}$ $H_{36}$ Cl $N_3$ $O_6$ S
Masse moléculaire : 590,14
Point de fusion : 184°s
**RMN** (DMSO $d_6$) δ : 1,45-2,05 (m, 8H) ; 2,28 (t, 2H) ; 2,55 (t, 2H) ; 2,99 (s, 3H) ; 3-3,2 (m, 2H) ; 3,75 (s, 3H) ; 3,92 (s, 2H) ; 3,94 (t, 2H) ; 4,25-4,5 (m, 1H) ; 6,55 (s, 2H) ; 6,75-7,02 (m, 5H) ; 7,03-7,22 (m, 2H); 8-13 (m, 2H).

**Exemple 57 :**

**Hydrogénofumarate de la N-méthyl-N-[1-[4-(4-chlorophénoxy) butyl] pipéridin-4-yl]-4H-3,1-benzothiazin-2-amine (57).**

La condensation de 606 mg (2,3 m moles) de 4-chloro-1-(4-bromobutoxy) benzène sur 600 mg (2,3 m moles) de N-méthyl-N-(4-pipéridinyl)-4H-3,1-benzothiazinamine selon **l'exemple 16.3** fournit 1,02 g (Rdt : 79 %) de poudre blanche de **formule 57 :**

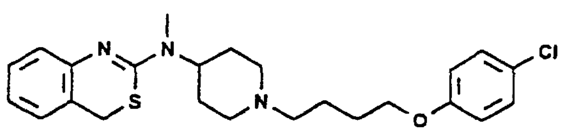
,
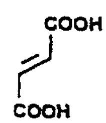
**( 57 )**

Formule brute : $C_{28}$ $H_{34}$ Cl $N_3$ $O_5$ S
Masse moléculaire : 560,12
Point de fusion : 188°
**RMN** (DMSO $d_6$) δ : 1,5-2,1 (m, 8H) ; 2,32 (t, 2H) ; 2,58 (t, 2H) ; 3,02 (s, 3H); 3-3,25 (m, 2H) ; 3,96 (s, 2H) ; 3,99 (t, 2H) ; 4,25-4,5 (m, 1H) ; 6,59 (s, 2H) ; 6,85-7,02 (m, 4H) ; 7,05-7,25 (m, 2H) ; 7,25-7,4 (m, 2H) ; 8-13 (m, 2H).

**Exemple 58 :**

**Hydrogénofumarate de la N-méthyl-N-[1-[5-(4-méthoxyphénoxy) pentyl] pipéridin-4-yl]-4H-3,1-benzothiazin-2-amine (58).**

L'adaptation du procédé de l'**exemple 16.3** au 4-méthoxy-1-(5-bromopentyloxy) benzène (630 mg ; 2,3 m moles) permet de préparer avec un rendement de 51 % le composé de **formule 58:**

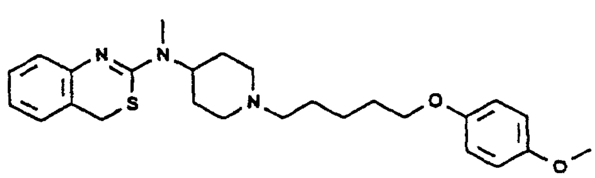
,
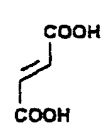
**( 58 )**

Formule brute : $C_{30}H_{39}N_3O_6S$
Masse moléculaire : 569,72
Point de fusion : 187°
**RMN** (DMSO $d_6$) δ : 1,3-1,8 (m, 8H) ; 1,8-2,1 (m, 2H) ; 2,25-2,7 (m, 4H) ; 3,03 (s, 3H) ; 3,05-3,3 (m, 2H) ; 3,69 (s, 3H) ; 3,90 (t, 2H), 3,95 (s, 2H) ; 4,25-4,55 (m, 1H); 6,60 (s, 4H) ; 6,75-7,3 (m, 6H) ; 8-13 (m, 2H).

**Exemple 59 :**

**Dihydrogénofumarate de la N-[1-[5-(3,4-difluorophénoxy) pentyl] pipéridin-4-yl]-4H-3,1-benzothiazin-2-amine (59).**

Par condensation de 732 mg (2,7 m moles) de 3,4-difluoro-1-(5-bromopentyloxy) benzène sur 636 mg (2,6 m moles) de N-(4-pipéridinyl)-4H-3,1-benzothiazin-2-amine selon le mode opératoire 16.3 on obtient avec un rendement de 51 % le composé de **formule 59 :**

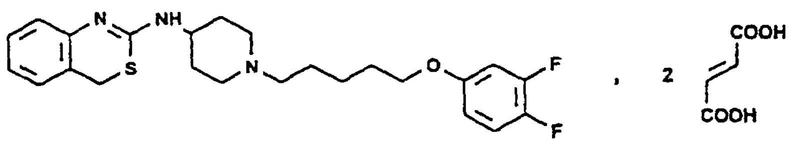

( 59 )

Formule brute : $C_{32}H_{37}F_2N_3O_9S$
Masse moléculaire : 677,70
Point de fusion: 185-186°
**RMN** (DMSO $d_6$) δ : 1,27-1,82 (m, 8H) ; 1,9-2,15 (m, 2H) ; 2,55-2,85 (m, 4H) ; 3,15-3,32 (m, 2H) ; 3,92 (s, 2H) ; 3,96 (t, 2H) ; 3,95-4,15 (m, 1H) ; 6,57 (s, 4H) ; 6,65-7,45 (m, 7H) ; 8-14 (m, 5H).

**Exemple 60 :**

**Dihydrogénofumarate de la N-[1-[5-(4-fluorophénoxy) pentyl] pipéridin-4-yl]-4H-3,1-benzothiazin-2-amine (60).**

L'application du protocole de **l'exemple 16.3** à 700 mg de N-(4-pipéridinyl)-4H-3,1-benzothiazin-2-amine et 776 mg (2,97 m moles) de 4-fluoro-1-(5-bromopentyloxy) benzène permet de préparer avec un rendement de 45 % le composé de **formule 60 :**

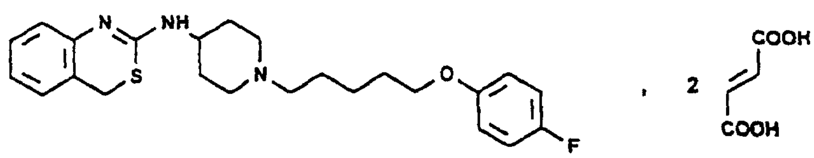

( 60 )

Formule brute : $C_{32}H_{38}FN_3O_9S$
Masse moléculaire : 659,71
Point de fusion : 185-186°
**RMN** (DMSO $d_6$) δ : 1,25-1,8 (m, 8H) ; 1,85-2,1 (m, 2H) ; 2,5-2,85 (m, 4H) ; 3,1-3,3 (m, 2H) ; 3,88 (s, 2H) ; 3,90 (t, 2H) ; 3,95-4,15 (m, 1H) ; 6,54 (s, 4H) ; 6,78-7 (m, 4H) ; 7-7,22 (m, 4H) ; 7-8 (m, 1H) ; 8-14 (m, 4H).

**Exemple 61 :**

**Hydrogénofumarate de la (R)-N-méthyl-N-[1-[2-hydroxy-3-(3,4-difluorophénoxy) propyl] pipéridin-4-yl]-4H-3,1-benzothiazin-2-amine (61).**

**61.1 :** (R)-1-(3,4-difluorophénoxy-2,3-époxypropane

En utilisant le mode opératoire de l'**exemple 32.1** mais en partant de 3,2 g (12,34 m moles) de R-3-nitrobenzène sulfonate de glycidyle et en les condensant sur 1,69 g (13 m moles) de 3-4-difluorophénol on récupère une huile

jaune paille (Rdt : 82 %) de **formule 61-1 :**

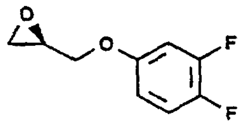

**( 61-1 )**

Formule brute : $C_9\ H_8\ F_2\ O_2$
Masse moléculaire : 186,15
$\alpha_D^{24°}$ = - 9,79° (c : 2,3 %, MeOH)

**61.2 :** Hydrogénofumarate de la (R)-N-méthyl-N-[1-(2-hydroxy-3-(3,4-difluorophénoxy) propyl] pipéridin-4-yl]-4H-3,1-benzothiazin-2-amine.

En opérant comme décrit dans l'**exemple 32.2** mais en condensant 1,2 g (4,6 m moles) d'époxyde (R) précédent sur 900 mg (4,83 m moles) de N-méthyl-N-[4-pipéridinyl]-4H-3,1-benzothiazin-2-amine, on prépare (Rdt : 48 %) le composé de **formule 61 :**

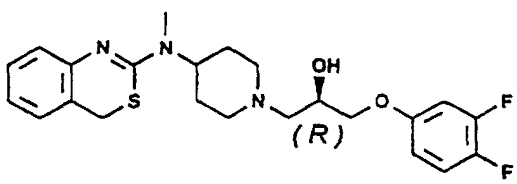

,

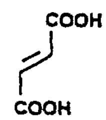

**( 61 )**

Formule brute : $C_{27}\ H_{31}\ F_2\ N_3\ O_6\ S$
Masse moléculaire : 563,60
Point de fusion : 153-4°
$\alpha_D^{24°}$ = + 8,05° (c : 1 %, MeOH) ; e/e ≥ 98 % (HPLC)
**RMN** (DMSO $d_6$) δ : 1,5-1,7 (m, 2H) ; 1,7-2,05 (m, 2H) ; 2,35 (t, 2H) ; 2,5-2,7 (m, 2H) ; 3,01 (s, 3H) ; 2,95-3,2 (m, 2H) ; 3,92 (s, 2H) ; 3,75-4,05 (m, 3H) ; 4,2-4,5 (m, 1H) ; 6,58 (s, 2H) ; 6,65-6,8 (m, 1H) ; 6,8-7 (m, 2H) ; 7-7,2 (m, 3H) ; 7,2-7,5 (m, 1H) ; 8-13 (m, 2H).

__Exemple 62 :__

**Hydrogénofumarate de la N-méthyl-N-[1-[5-(2-métoxyphénoxy) pentyl] pipéridin-4-yl]-4H-3,1-benzothiazin-2-amine (62).**

En partant de 600 mg (2,3 m moles) de N-méthyl-N-[4-pipéridinyl]-4H-3,1-benzothiazin-2-amine et en les condensant sur 630 mg (2,3 m moles) de 2-méthoxy-1-(5-bromopentyloxy) benzène selon le protocole de **l'exemple 16.3** on prépare avec un rendement de 58 % le composé de **formule 62 :**

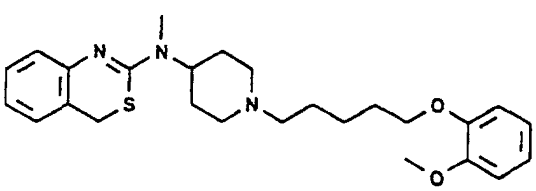

,

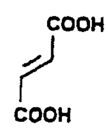

**( 62 )**

Formule brute : $C_{30}\ H_{39}\ N_3\ O_6\ S$
Masse moléculaire : 569,72
Point de fusion : 175°
**RMN** (DMSO $d_6$) δ : 1,3-2,3 (m, 8H) ; 1,8-2,1 (m, 2H) ; 2,3-2,7 (m, 4H) ; 2,99 (s, 3H) ; 3,1-3,3 (m, 2H) ; 3,72 (s,

3H) ; 3,8-4 (m, 2H) ; 3,92 (s, 2H) ; 4,3-4,6 (m, 1H) ; 6,55 (s, 2H) ; 6,8-7 (m, 6H) ; 7-7,2 (m, 2H) ; 8-13 (m, 2H).

**Exemple 63 :**

**Hydrogénofumarate de la N-méthyl-N-[1-[2-(2-méthoxyphénoxy) éthyl] pipéridin-4-yl]-4H-3,1-benzothiazin-2-amine (63).**

En opérant comme décrit dans **l'exemple 16.3** à partir de 600 mg (2,3 m moles) de N-méthyl-N-[4-pipéridinyl]-4H-3,1-benzothiazin-2-amine et de 504 mg (2,3 m moles) de 2-méthoxy-[1-(2-bromoéthoxy) benzène on obtient avec un rendement de 63 % le composé de **formule 63 :**

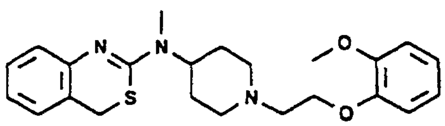 , 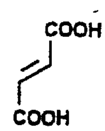 **( 63 )**

Formule brute : $C_{27} H_{33} N_3 O_6 S$
Masse moléculaire : 527,64
Point de fusion : 178°
**RMN** (DMSO $d_6$) $\delta$ : 1,5-1,7 (m, 2H) ; 1,7-2 (m, 2H) ; 2,25 (t, 2H) ; 2,79 (t, 2H) ; 3,02 (s, 3H) ; 3,05-3,2 (m, 2H) ; 3,75 (s, 3H) ; 3,94 (s, 2H) ; 4,07 (t, 2H) ; 4,25-4,5 (m, 1 H) ; 6,6 (s, 2H) ; 7,3-7 (m, 6H) ; 7,05-7,25 (m, 2H) ; 8-12 (m, 2H).

## Revendications

**1.** N-hétérocyclyl-1-aryloxyalcolyl-4-pipéridinamines de formule I

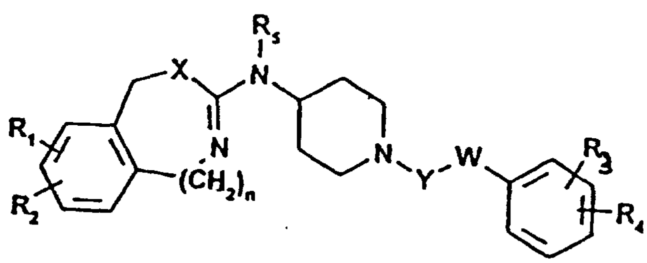 I

dans laquelle les substituants sont définis comme suit :

$R_1$ à $R_4$ égaux ou différents représentent :

- un hydrogène,
- un alcoyle ramifié ou non renfermant de 1 à 4 atomes de carbone,
- un alcoyloxy ramifié ou non renfermant de 1 à 4 atomes de carbone,
- un groupement halogéno,
- un groupement nitro,
- un groupement hydroxy,
- un groupement trifluorométhyle ou trifluorométhoxyle,

$R_5$ représente :

- un hydrogène,
- un alcoyle ramifié ou non renfermant de 1 à 6 atomes de carbone,
- un phenylalcoyle ramifié ou non renfermant de 7 à 12 atomes de carbone, pouvant être substitué sur l'aromatique par un ou deux radicaux définis comme $R_1$,

W et X représentent :

- un oxygène ou un soufre,

Y représente:

- $(CH_2)_m$ avec m est un entier compris entre 2 et 6 ou
- le radical $CH_2$-CH(OH)-$CH_2$-

n peut prendre les valeurs 0 ou 1,

lorsque les composés de formule I renferment un carbone asymétrique, leurs mélanges racémiques, leurs différents énantiomètres purs ou leurs mélanges, ainsi que
les sels minéraux ou organiques thérapeutiquement acceptables des composés de formule I et leurs hydrates éventuels.

**2.** Composé selon la revendication 1 **caractérisé en ce qu'**il est choisi parmi les composés suivants :

- Hydrogénomaléate de la N-méthyl-N-[1-[4-(4-fluorophénoxy)butyl]pipéridin-4-yl]-4H-3,1-benzothiazin-2-amine.
- Hydrogénomaléate de la N-méthyl-N-[1-[3-(4-fluorophénoxy)propyl] pipéridin-4-yl]-4H-3,1-benzothiazin-2-amine.
- Hydrogénomaléate de la N-méthyl-N-[1-[2-(4-fluorophénoxy)éthyl]pipéridin-4-yl]-4H-3,1-benzothiazin-2-amine.
- Hydrogénomaléate de la N-[1-[4-(4-fluorophénoxy)butyl]pipéridin-4-yl]-4H-3,1-benzothiazin-2-amine.
- Monohydrate de l'hydrogénomaléate de la N-[1-[3-(4-fluorophénoxy)propyl]pipéridin-4-yl]-4H-3,1-benzothiazin-2-amine.
- Hydrogénofumarate de la N-éthyl-N-[1-[3-(4-fluoro-phénoxy)propyl]pipéridin-4-yl]-4H -3,1-benzothiazin-2-amine.
- Hydrogénomaléate de la N-benzyl-N-[1-[3-(4-fluorophénoxy)propyl]pipéridin-4-yl]-4H-3,1-benzothiazin-2-amine.
- Hydrogénomaléate de la 6-fluoro-N-méthyl-N-[1-[4-(4-fluorophénoxy)butyl]pipéridin-4-yl]-4H-3,1-benzothiazin-2-amine.
- Hydrogénomaléate de la 6-chloro-N-méthyl-N-[1-[3-(4-fluorophénoxy)propyl]pipéridin-4-yl]-4H-3,1-benzothiazin-9-amine.
- Dihydrogénomaléate de la 6-méthoxy-N-méthyl-N-[1-[3-(4-fluorophénoxy)propyl]pipé- ridin-4-yl]-4H-3,1-benzothiazin-2-amine.
- Hydrogénomaléate de la N-méthyl-N-[1-[4-(3,4-difluorophénoxy)butyl]pipéridin-4yl]-4H-3,1-benzothiazin-2-amine.
- Hydrogéno maléate de la N-[1-[4-(3,4-difluorophénoxy)butyl]pipéridin-4-yl]-4H-3,1-benzothiazin-2-amine.
- Hydrogénomaléate de la N-[1-[4-(4-chlorophénoxy)butyl]pipéridin-4-yl]-4H-3,1-benzothiazin-2-amine.
- Hémihydrate du dichlorhydrate de la N-[1-[2-hydroxy-3-(4-fluorophénoxy)propyl] pipéridin-4-yl]-4H-3,1-benzothiazin-2-amine.
- N-[1-[2-hydroxy-3-(4-fluorophénoxy) propyl] pipéridin-4-yl]-4H-3,1-benzothiazin-2-amine.
- Dichlorhydrate de la N-méthyl-N-[1-[2-hydroxy-3-(4-fluorophénoxy)propyl]pipéridin-4-yl]-4H-3,1-benzothiazin-2-amine.
- Hydrogénomaléate de la N-méthyl-N-[1-(4-phénoxybutyl)pipéridin-4-yl]-4H-3,1-benzo thiazin-2-amine.
- N-[1-[4-(4-fluorophenoxy)butyl]pipéridin-4-yl]-4H-3,1-benzothiazin-2-amine.
- Dihydrogénomaléate de la 6,7-diméthoxy-N-[1-[3-(3,4-difluorophénoxy)propyl]pipéridin -4-yl]-4H-3,1-benzothiazin-2-amine.
- Dihydrogénomaléate de la 6,7-diméthoxy-N-méthyl-N-[1-[3-(3,4-difluorophénoxy)propyl]pipéridin-4-yl]-4H-3,1-benzothiazin-2-amine.
- Hémifumarate de la 6-méthyl-N-[1-[3-(3,4-difluorophénoxy)propyl]pipéridin-4-yl-3,1-benzothiazin-2-amine.
- Hydrogénomaléate de la N-méthyl-N-[1-[4-(4-méthylphénoxy)butyl]pipéridin-4-yl]-4H-3,1-benzothiazin-2-amine.
- Hydrogénomaléate de la N-méthyl-N-[1-[4-(4-méthoxyphénoxy)butyl]pipéridin-4-yl]-4H-3,1-benzothiazin-2-amine.
- Dichlorhydrate de la N-[1-[2-hydroxy-3-(3,4-difluorophénoxy)propyl]pipéridin-4-yl]-4H-3,1-benzothiazin-2-amine.

- N-[1-[2-hydroxy-3-(3,4-difluorophénoxy) propyl] pipéridin-4-yl]-4H-3,1-benzothiazin-2-amine.
- Hydrate du dihydrogénofumarate de la N-méthyl-N-[1-[4-(4-fluorophénoxy)butyl]pipéridin-4-yl]-1,5-dihydro-2,4-benzothiazepin-3-amine.
- Hydrate de dihydrogénofumarate de la N-méthyl-N-[1-[4-(3,4-difluorophénoxy) butyl] pipéridin-4-yl]-1,5-dihydro-2,4-benzothiazépin-3-amine.
- Hémifumarate de la 6,7-diméthoxy-N-[1-[3-(4-fluorophénoxy)propyl]pipéridin-4-yl]-4H-3,1-benzothiazin-2-amine.
- Dichlorhydrate de la N-[1-[4-(4-fluorophénoxy)butyl]pipéridin-4-yl]-4H-3,1-benzoxazin -2-amine.
- Hémifumarate de la 6,7-diméthoxy-N-[1-[4-(4-fluorophénoxy)butyl]pipéridin-4-yl]-4H-3,1-benzothiazin-2-amine.
- Hémihydrate du dichlorhydrate de la 6-méthyl-N-[1-[4-(4-fluorophénoxy)butyl]pipéridin-4-yl]-4H-3,1-benzothiazin-2-amine.
- Hydrogénomaléate de la N,6-diméthyl-N-[1-[4-(4-fluorophénoxy)butyl]pipéridin-4-yl]-4H-3,1-benzothiazin-2-amine.
- Dichlorhydrate de la (dl) N-méthyl-N-[1-[2-hydroxy-3-(3,4-difluorophénoxy)propyl] pipéridin-4-yl-4H-3,1-benzothiazin-3-amine.
- Hydrogénomaléate de la (S)-N-méthyl-N-[1-[2-hydroxy-3-(3,4-difluorophénoxy) propyl]piperidin-4-yl]-4H-3,1-benzothiazin-2-amine.
- Hydrogénomaléate de la N-méthyl-N-[1-[4-fluorophénylthio) butyl] pipéridin-4-yl]-4H-3,1-benzothiazin-2-amine.
- Hydrogénofumarate de la N-méthyl-N-[1-[5-(4-fluorophénoxyl) pentyl] pipéridin-4-yl]-4H-3,1-benzothiazin-2-amine.
- Hydrogénofumarate de la N-méthyl-N-[1-[3-(4-fluorophénoxy) propyl] pipéridin-4-yl]-4H-3,1-benzothiazin-2-amine.
- Hydrogénofumarate de la N-méthyl-N-[1-[6-(4-fluorophénoxy) hexyl] pipéridin-4-yl]-4H-3,1-benzothiazin-2-amine.
- Hydrogénofumarate de la N-méthyl-N-[1-[4-(3,4-diméthoxyphénoxy) butyl] pipéridin-4-yl]-4H-3,1-benzothiazin-2-amine.
- Hydrogénofumarate de la N-méthyl-N-[1-[4-(2-méthoxyphénoxy) butyl)] pipéridin-4-yl]-4H-3,1-benzothiazin-2-amine.
- Hydrogénofumarate. de la N-méthyl-N-[1-[4-(2,3-diméthoxyphénoxy) butyl)] pipéridin- 4-yl]-4H-3,1-benzothiazin-2-amine.
- Hydrogénofumarate de la N-méthyl-N-[1[4-(3,5-diméthoxyphénoxy) butyl] pipéridin-4-yl]-4H-3,1-benzothiazin-2-amine.
- Hydrogénofumarate de la N-méthyl-N-[1-[4-(2,6-diméthoxyphénoxy) butyl] pipéridin-4-yl]-4H-3,1-benzothiazin-2-amine.
- Hydrogénofumarate de la N-méthyl-N-[1-[4-(2,6-difluorophénoxy) butyl] pipéridin-4-yl]-4H-3,1-benzothiazin-2-amine.
- Hydrogénofumarate de la N-méthyl-N-[1-[5-(3,4-difluorophénoxy) pentyl] pipéridin-4-yl]-4H-3,1-benzothiazin-2-amine.
- Hydrogénofumarate de la N-méthyl-N-[1-[4-(3,5-difluorophénoxy) butyl] pipéridin-4-yl]-4H-3,1-benzothiazin-2-amine.
- Hydrogénofumarate de la N-méthyl-N-[1-[4-(2,4-difluorophénoxy) butyl] pipéridin-4-yl]-4H-3,1-benzothiazin-2-amine.
- Hydrogénofumarate de la N-méthyl-N-[1-[4-(2,5-difluorophénoxy) butyl] pipéridin-4-yl]-4H-3,1-benzothiazin-2-amine.
- Hydrogénofumarate de la N-méthyl-N-[1-[4-(3-fluoro-4-chlorophénoxy)butyl] pipéridin -4-yl]-4H-3,1-benzothiazin-2-amine.
- Hydrogénofumarate de la N-methyl-N-[1-[4-(2,3-difluorophénoxy)butyl] pipéridin-4-yl]-4H-3,1-benzothiazin-2-amine.
- Hydrogénofumarate de la N-méthyl-N-[1-[4(3-chloro-4-fluorophénoxy) butyl] pipéri-din-4-yl]-4H-3,1-benzothiazin-2-amine.
- Hydrogénofumarate de la N-méthyl-N-[1-[4-(3-fluorophénoxy) butyl] pipéridin-4-yl]-4H-3,1-benzothiazin-2-amine.
- Hydrogénofumarate de la N-méthyl-N-[1-[4-(3-méthoxyphénoxy) butyl] pipéridin-4-yl]-4H-3,1-benzothiazin-2-amine.
- Hydrogénofumarate de la N-méthyl-N-[1-[4-(2-fluorophénoxy) butyl] pipéridin-4-yl]-4H-3,1-benzothiazin-2-amine.

- Hydrogénofumarate de la N-méthyl-N-[1-[4-(2-méthoxy-4-chlorophénoxy) butyl] pipéridin-4-yl]-4H-3,1-benzothiazin-2-amine.
- Hydrogénofumarate de la N-méthyl-N-[1-[4-(4-chlorophénoxy) butyl] pipéridin-4-yl]-4H-3,1-benzothiazin-2-amine.
- Hydrogénofumarate de la N-méthyl-N-[1-[5-(4-méthoxyphénoxy) pentyl] pipéridin-4-yl]-4H-3,1-benzothiazin-2-amine.
- Dihydrogénofumarate de la N-[1-[5-(3,4-difluorophénoxy) pentyl] pipéridin-4-yl]-4H-3,1-benzothiazin-2-amine.
- Dihydrogénofumarate de la N-[1-[5-(4-fluorophénoxy) pentyl] pipéridin-4-yl]-4H-3,1-benzothiazin-2-amine.
- Hydrogénofumarate de la (R)-N-méthyl-N-[1-[2-hydroxy-3-(3,4-difluorophénoxy) propyl] pipéridin-4-yl]-4H-3,1-benzothiazin-2-amine.
- Hydrogénofumarate de la N-méthyl-N-[1-[5-(2-métoxyphénoxy)pentyl] pipéridin-4-yl]-4H-3,1-benzothiazin-2-amine.
- Hydrogénofumarate de la N-méthyl-N-[1-[2-(2-méthoxyphénoxy) éthyl] pipéridin-4-yl]-4H-3,1-benzothiazin-2-amine.

**3.** Procédé de préparation des composés chimiques de formule Ia, c'est-à-dire les composés chimiques de formule I selon la revendication 1 ou 2 pour laquelle n = 0, $R_5$ = H et Y = $(CH_2)_m$,

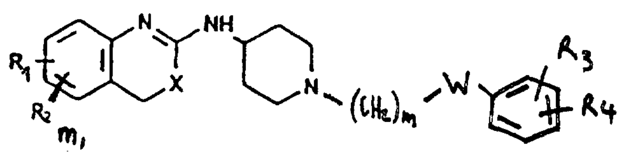

Ia

$R_1$, $R_2$, $R_3$, $R_4$, m, X et W sont tels que définis à la revendication 1,
**caractérisé en ce que** l'on fait réagir un orthoaminobenzylalcool de formule VIII

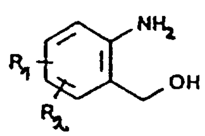

VIII

avec un 4-piperidinyl iso(thio)cyanate de formule IXa

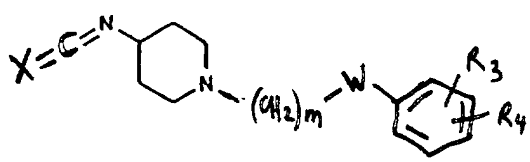

IXa

dans du THF ou du dioxane comme solvant à une température comprise entre 20 et 80° pour donner l'hydroxyméthyl(thio)urée intermédiaire Xa

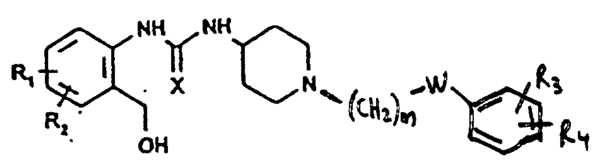

Xa

Xa est récupéré par filtration et cyclisé directement dans l'acide chlorhydrique concentré à une température comprise entre 20 et 80° pour donner le composé de formule générale Ia.

**4.** Procédé de préparation des composés chimiques de formule Ib

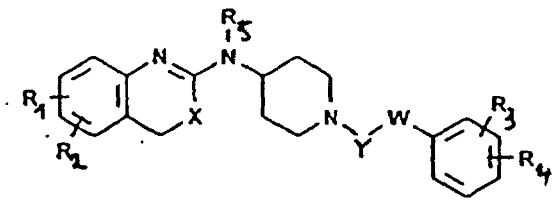

Ib

c'est-à-dire des composés chimiques de formule I selon les revendications 1 et 2 pour laquelle $R_1$ à $R_5$, W, X, Y sont tels que définis à la revendication 1 et n=0,
**caractérisé en ce que** l'on fait réagir un orthoaminoalcool de formule (VIII) avec un iso(thiocyanate) de formule (IXb)

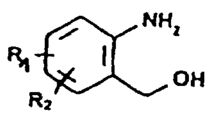

VIII

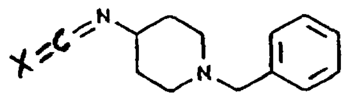

IXb

dans du THF ou du dioxane comme solvant à une température comprise entre 20 et 80° pour donner une hydroxyméthyl(thio)urée intermédiaire (Xb)

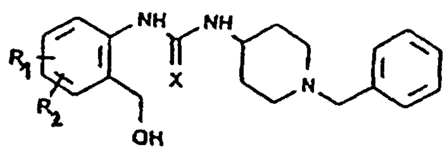

Xb

Xb qui peut ensuite être récupéré par filtration et qui est cyclisé directement par chauffage entre 20 et 70° dans l'acide chlorhydrique concentré pour donner le composé de formule XIb

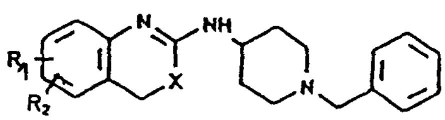

XIb

qui peut être alcoylé par un halogénure ($R_5$ - Br ou $R_5$ - I) ou un sulfate $(R_5)_2SO_4$ en présence de carbonate alcalin ou d'hydrure de sodium dans le DMF comme solvant pour donner le composé de formule (IVb),

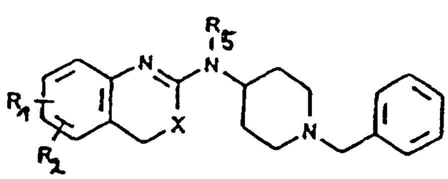

IVb

dont la N-débenzylation en présence de chloroformiate d'α-chloroéthyle dans le chlorure de méthylène suivi d'une hydrolyse au reflux du méthanol conduit à l'intermédiaire de formule (V)

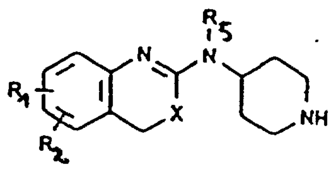

V

qui par N-alcoylation finale avec un halogénure de formule (VI) où Z représente un atome de brome, de chlore on d'iode,

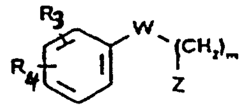

VI

dans le DMF en présence de $K_2CO_3$/KI 98/02 ou un glycidyéther de formule (VII) dans le méthanol donne le composé de formule générale Ib

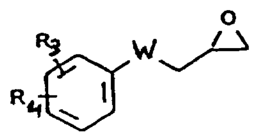

VII

**5.** Procédé de préparation des composés chimiques de formule I selon les revendications 1 et 2 où $R_5$ est différent de H **caractérisé en ce que** l'on fait agir un composé de formule I où $R_5$=H avec un halogénure d'(aryl)alcoyle $R_5$-Z ,$R_5$ étant tel que défini à la revendication 1 et Z tel que défini à la revendication 4, ou un sulfate d'alcoyle $(R_5)_2SO_4$, $R_5$ étant tel que défini à la revendication 1, en présence d'une base comme NaH ou $Na_2CO_3$ pour donner un composé de formule I pour lequel $R_5 \neq H$ est tel que défini à la revendication 1.

**6.** A titre de médicament les composés définis selon l'une des revendications 1 et 2.

**7.** Composition pharmaceutique **caractérisée en ce qu'**elle contient comme principe actif au moins un composé selon l'une des revendications 1, 2 ou 6 associé à un support pharmaceutique inerte ou autres véhicules pharmaceutiquement acceptables et pouvant ou non être associé à un autre médicament.

**8.** Composition pharmaceutique selon la revendication 7 utile dans le traitement prophylactique de l'ischémie myocardique comme les crises d'angor stable chronique, l'angor instable et de Prinzmetal, l'ischémie silencieuse, le réinfarctus, la réocclusion et la resténose.

**9.** Composition pharmaceutique selon la revendication 7 utile dans l'ischémie cérébrale, l'accident vasculaire cérébral, l'attaque ischémique transitoire et les maladies neurodégénératives.

**10.** Composition pharmaceutique selon la revendication 7 **caractérisée en ce qu'**elle permet de prévenir et de traiter l'athérosclérose.

## Patentansprüche

**1.** N-Heterocyclyl-1-aryloxyalcolyl-4-piperidinamine der Formel I

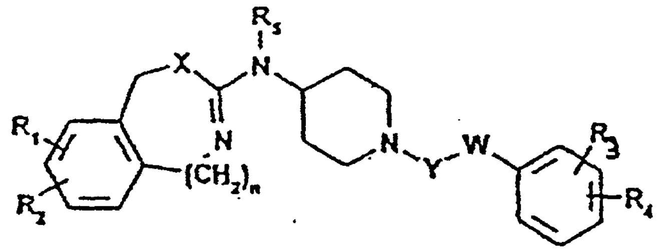

I

worin die Substituenten folgendermaßen definiert sind:

$R_1$ bis $R_4$, gleich oder verschieden, stehen für

- einen Wasserstoff,
- ein verzweigtes oder nicht verzweigtes Alkyl mit 1 bis 4 Kohlenstoffatomen,
- ein verzweigtes oder nicht verzweigtes Alkyloxy mit 1 bis 4 Kohlenstoffatomen,
- eine Halogengruppe,
- eine Nitrogruppe,
- eine Hydroxygruppe,
- eine Trifluormethyl- oder Trifluormethoxylgruppe,

$R_5$ steht für

- einen Wasserstoff,
- ein verzweigtes oder nicht verzweigtes Alkyl mit 1 bis 6 Kohlenstoffatomen,
- ein verzweigtes oder nicht verzweigtes Phenylalkyl mit 7 bis 12 Kohlenstoffatomen, welches am aromatischen Ring mit einem oder zwei Radikalen, die wie $R_1$ definiert sind, substituiert sein kann,

W und X stehen für

- einen Sauerstoff oder einen Schwefel,

Y steht für

- $(CH_2)_m$, wobei m eine ganze Zahl zwischen 2 und 6 ist, oder
- das Radikal $CH_2$-CH(OH)-$CH_2$-,

und n die Werte 0 oder 1 annehmen kann,
wenn die Verbindungen der Formel I einen asymmetrischen Kohlenstoff enthalten, ihre razemischen Mischungen, ihre verschiedenen reinen Enantiomeren oder deren Mischungen, sowie die therapeutisch geeigneten mineralischen oder organischen Salze der Verbindungen der Formel I und ihre möglichen Hydrate.

2. Verbindung gemäß Anspruch 1, **dadurch gekennzeichnet, dass** es sich um eine der folgenden Verbindungen handelt:

- Hydrogenmaleat von N-Methyl-N-[1-[4-(4-fluorphenoxy)butyl]-piperidin-4-yl]-4H-3,1-benzothiazin-2-amin,
- Hydrogenmaleat von N-Methyl-N-[1-[3-(4-fluorphenoxy)-propyl]piperidin-4-yl]-4H-3,1-benzothiazin-2-amin
- Hydrogenmaleat von N-Methyl-N-[1-[2-(4-fluorphenoxy)ethyl]-piperidin-4-yl]-4H-3,1-benzothiazin-2-amin,
- Hydrogenmaleat von N-[1-[4-(4-fluorphenoxy)butyl]-piperidin-4-yl]-4H-3,1-benzothiazin-2-amin,
- Hydrogenmaleatmonohydrat von N- [1- [3- (4-fluorphenoxy)-propyl]piperidin-4-yl]-4H-3,1-benzothiazin-2-amin,
- Hydrogenfumerat von N-Ethyl-N- [1- [3- (4-fluorphenoxy)-propyl]piperidin-4-yl]-4H-3;1-benzothiazin-2-amin,
- Hydrogenmaleat von N-Benzyl-N-[1-[3-(4-fluorphenoxy)-propyl]piperidin-4-yl]-4H-3,1-benzothiazin-2-amin,
- Hydrogenmaleat von 6-Fluor-N-methyl-N-[1-[4-(4-fluorphenoxy)butyl]piperidin-4-yl]-4H-3,1-benzothiazin-2-amin,
- Hydrogenmaleat von 6-Chlor-N-methyl-N-[1-[3-(4-fluorphenoxy)propyl]piperidin-4-yl]-4H-3,1-benzothiazin-2-amin,

- Dihydrogenmaleat von 6-Methoxy-N-methyl-N-[1-[3-(4-fluorphenoxy)propyl]piperidin-4-yl]-4H-3,1-benzothiazin-2-amin,
- Hydrogenmaleat von N-Methyl-N-[1-[4-(3,4-difluorphenoxy)-butyl]piperidin-4-yl]-4H-3,1-benzothiazin-2-amin,
- Hydrogenmaleat von N-[1-[4-(3,4-Difluorphenoxy)butyl]-piperidin-4-yl]-4H-3,1-benzothiazin-2-amin,
- Hydrogenmaleat von N- [1- [4- (4-Chlorphenoxy) butyl]-piperidin-4-yl]-4H-3,1-benzothiazin-2-amin,
- Dichlorhydrathemihydrat von N-[1-[2-Hydroxy-3-(4-fluorphenoxy)propyl]piperidin-4-yl]-4H-3,1-benzothiazin-2-amin,
- N-[1-[2-Hydroxy-3-(4-fluorphenoxy)propyl]piperidin-4-yl]-4H-3,1-benzothiazin-2-amin,
- Dichlorhydrat von N-Methyl-N-[1-[2-hydroxy-3-(4-fluorphenoxy)propyl]piperidin-4-yl]-4H-3,1-benzothiazin-2-amin,
- Hydrogenmaleat von N-Methyl-N-[1-(4-phenoxybutyl)piperidin-4-yl] -4H-3,1-benzothiazin-2-amin,
- N-[1-[4-[4-Fluorphenoxy]butyl]piperidin-4-yl]-4H-3,1-benzothiazin-2-amin,
- Dihydrogenmaleat von 6,7-Dimethoxy-N-[1-[3-(3,4-difluorphenoxy)propyl]piperidin-4-yl]-4H-3,1-benzothiazin-2-amin,
- Dihydrogenmaleat von 6,7-Dimethoxy-N-methyl-N-[1-[3-(3,4-difluorphenoxy)propyl]piperidin-4-yl]-4H-3,1-benzothiazin-2-amin,
- Hemifumarat von 6-Methyl-N-[1-[3-(3,4-difluorphenoxy)-propyl]piperidin-4-yl]-3,1-benzothiazin-2-amin,
- Hydrogenmaleat von N-Methyl-N-[1-[4-(4-methylphenoxy)-butyl]piperidin-4-yl]-4H-3,1-benzothiazin-2-amin,
- Hydrogenmaleat von N-Methyl-N-[1-[4-(4-methoxyphenoxy)-butyl]piperidin-4-yl]-4H-3,1-benzothiazin-2-amin,
- Dichlorhydrat von N-[1-[2-Hydroxy-3-(3,4-difluorphenoxy)-propyl]piperidin-4-yl]-4H-3,1-benzothiazin-2-amin,
- N-[1-[2-Hydroxy-3-(3,4-difluorphenoxy)propyl]piperidin-4-yl] -4H-3,1-benzothiazin-2-amin,
- Dihydrogenfumarathydrat von N-Methyl-N-[1-[4-(4-fluorphenoxy)butyl]piperidin-4-yl]-1,5-dihydro-2,4-benzothiazepin-3-amin,
- Dihydrogenfumarathydrat von N-Methyl-N-[1-[4-(3,4-difluorphenoxy)butyl]piperidin-4-yl]-1,5-dihydro-2,4-benzothiazepin-3-amin,
- Hemifumarat von 6,7-Dimethoxy-N-[1-[3-(4-fluorphenoxy)-propyl]piperidin-4-yl]-4H-3,1-benzothiazin-2-amin,
- Dichlorhydrat von N-[1-[4-(4-Fluorphenoxy)butyl]piperidin-4-yl] -4H-3,1-benzoxyzin-2-amin,
- Hemifumarat von 6,7-Dimethoxy-N-[1-[4-(4-fluorphenoxy)-butyl]piperidin-4-yl]-4H-3,1-benzothiazin-2-amin,
- Dichlorhydrathemihydrat von 6-Methyl-N-[1-[4-(4-fluorphenoxy)butyl]piperidin-4-yl]-4H-3,1-benzothiazin-2-amin,
- Hydrogenmaleat von N,6-Dimethyl-N-[1-[4-(4-fluorphenoxy)-butyl]piperidin-4-yl] -4H-3,1-benzothiazin-2-amin,
- Dichlorhydrat von (dl) N-Methyl-N-[1-[2-hydroxy-3-(3,4-difluorphenoxy)propyl]piperidin-4-yl]-4H-3,1-benzothiazin-3-amin,
- Hydrogenmaleat von (S)-N-Methyl-N-[1-[2-hydroxy-3-(3,4-difluorphenoxy)propyl]piperidin-4-yl]-4H-3,1-benzothiazin-2-amin,
- Hydrogenmaleat von N-Methyl-N-[1-(4-fluorphenylthio)-butyl]piperidin-4-yl]-4H-3,1-benzothiazin-2-amin,
- Hydrogenfumarat von N-Methyl-N-[1-[5-(4-fluorphenoxy)-pentyl]piperidin-4-yl]-4H-3,1-benzothiazin-2-amin,
- Hydrogenfumarat von N-Methyl-N-[1-[3- (4-fluorphenoxy)-propyl]piperidin-4-yl]-4H-3,1-benzothiazin-2-amin,
- Hydrogenfumarat von N-Methyl-N- [1- [6- (4-fluorphenoxy)-hexyl]piperidin-4-yl] -4H-3,1-benzothiazin-2-amin,
- Hydrogenfumarat von N-Methyl-N-[1-[4-(3,4-dimethoxyphenoxy) butyl]piperidin-4-yl]-4H-3,1-benzothiazin-2-amin,
- Hydrogenfumarat von N-Methyl-N-[1-[4-(2-methoxyphenoxy) butyl]piperidin-4-yl]-4H-3,1-benzothiazin-2-amin,
- Hydrogenfumarat von N-Methyl-N-[1-[4-(2,3-dimethoxyphenoxy)butyl]piperidin-4-yl]-4H-3,1-benzothiazin-2-amin,
- Hydrogenfumarat von N-Methyl-N-[1-[4-(3,5-dimethoxyphenoxy)butyl]piperidin-4-yl]-4H-3,1-benzothiazin-2-amin,
- Hydrogenfumarat von N-Methyl-N-[1-[4-(2,6-dimethoxyphenoxy)butyl]piperidin-4-yl]-4H-3,1-benzothiazin-2-amin,
- Hydrogenfumarat von N-Methyl-N-[1-[4-(2,6-difluorphenoxy)-butyl]piperidin-4-yl]-4H-3,1-benzothiazin-2-amin,
- Hydrogenfumarat von N-Methyl-N-[1-[5-(3,4-difluorphenoxy)-pentyl]piperidin-4-yl] -4H-3,1-benzothiazin-2-amin,
- Hydrogenfumarat von N-Methyl-N-[1-[4-(3,5-difluorphenoxy)-butyl]piperidin-4-yl]-4H-3,1-benzothiazin-2-amin,
- Hydrogenfumarat von N-Methyl-N-[1-[4-(2,4-difluorphenoxy)-butyl]piperidin-4-yl]-4H-3,1-benzothiazin-2-amin,

- Hydrogenfumarat von N-Methyl-N-[1-[4-(2,5-difluorphenoxy)-butyl]piperidin-4-yl]-4H-3,1-benzothiazin-2-amin,
- Hydrogenfumarat von N-Methyl-N-[1-[4-(3-fluor-4-chlorphenoxy)butyl]piperidin-4-yl]-4H-3,1-benzothiazin-2-amin,
- Hydrogenfumarat von N-Methyl-N- [1- [4-(2,3-difluorphenoxy)-butyl]piperidin-4-yl]-4H-3,1-benzothiazin-2-amin,
- Hydrogenfumarat von N-Methyl-N-[1-[4-(3-chlor-4-fluorphenoxy)butyl]piperidin-4-yl]-4H-3,1-benzothiazin-2-amin,
- Hydrogenfumarat von N-Methyl-N-[1-[4- (3-fluorphenoxy)-butyl]piperidin-4-yl]-4H-3,1-benzothiazin-2-amin,
- Hydrogenfumarat von N-Methyl-N-[1-[4-(3-methoxyphenoxy)-butyl]piperidin-4-yl]-4H-3,1-benzothiazin-2-amin,
- Hydrogenfumarat von N-Methyl-N-[1-[4-(2-fluorphenoxy)-butyl]piperidin-4-yl]-4H-3,1-benzothiazin-2-amin,
- Hydrogenfumarat von N-Methyl-N-[1-[4-(2-methoxy-4-chlorphenoxy)butyl]piperidin-4-yl]-4H-3,1-benzothiazin-2-amin,
- Hydrogenfumarat von N-Methyl-N-[1-[4-(4-chlorphenoxy)-butyl]piperidin-4-yl]-4H-3,1-benzothiazin-2-amin,
- Hydrogenfumarat von N-Methyl-N-[1-[5-(4-methoxyphenoxy)-pentyl]piperidin-4-yl]-4H-3,1-benzothiazin-2-amin,
- Dihydrogenfumarat von N-[1-[5-(3,4-Difluorphenoxy)-pentyl]piperidin-4-yl]-4H-3,1-benzothiazin-2-amin,
- Dihydrogenfumarat von N- [1- [5- (4-Fluorphenoxy)-pentyl]piperidin-4-yl]-4H-3,1-benzothiazin-2-amin,
- Hydrogenfumarat von (R)-N-Methyl-N-[1-[2-hydroxy-3-(3,4-difluorphenoxy)propyl]piperidin-4-yl]-4H-3,1-benzothiazin-2-amin,
- Hydrogenfumarat von N-Methyl-N-[1-[5-(2-methoxyphenoxy)-pentyl]piperidin-4-yl]-4H-3,1-benzothiazin-2-amin,
- Hydrogenfumarat von N-Methyl-N-[1-[2-(2-methoxyphenoxy)-ethyl]piperidin-4-yl]-4H-3,1-benzothiazin-2-amin.

**3.** Verfahren zur Herstellung der chemischen Verbindungen der Formel Ia, dass heißt, der chemischen Verbindungen der Formel I gemäß Anspruch 1 oder 2, worin n = 0, $R_5$ = H und Y = $(CH_2)_m$ sind,

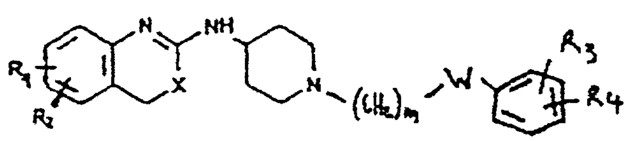

Ia

und $R_1$, $R_2$, $R_3$, $R_4$, m, X und W die in Anspruch 1 definierte Bedeutung haben, **dadurch gekennzeichnet, dass** man einen Orthoaminobenzylalkohol der Formel VIII

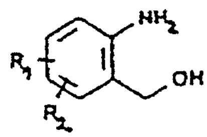

VIII

mit einem 4-Piperidinyliso(thio)cyanat der Formel IXa

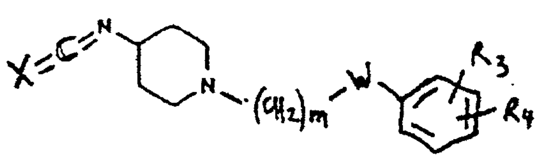

IXa

in THF oder Dioxan als Lösungsmittel bei einer Temperatur zwischen 20 und 80° umsetzt, wodurch das Zwischenprodukt Xa Hydroxymethyl(thio)harnstoff erhalten wird,

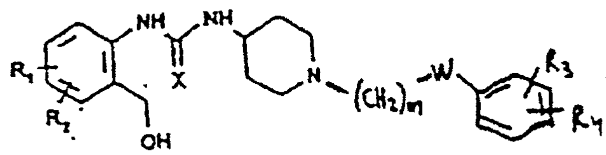 Xa

Xa durch Filtration gewinnt und direkt in konzentrierter Salzsäure bei der Temperatur zwischen 20 und 80° zyklisiert, wodurch die Verbindung der allgemeinen Formel Ia erhalten wird.

**4.** Verfahren zur Herstellung der chemischen Verbindungen der Formel Ib,

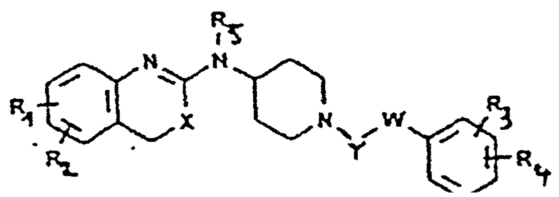 Ib

dass heißt, der chemischen Verbindungen der Formel I gemäß den Ansprüchen 1 und 2, worin $R_1$ bis $R_5$, W, X, Y die in Anspruch 1 definierte Bedeutung haben und n = 0 ist, **dadurch gekennzeichnet, dass** man einen Orthoaminoalkohol der Formel (VIII) mit einem Isothiocyanat der Formel (IXb)

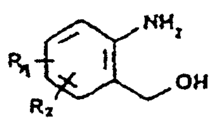 VIII 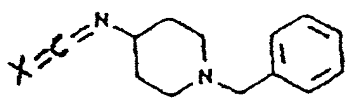 IXb

in THF oder Dioxan als Lösungsmittel bei einer Temperatur zwischen 20 und 80° umsetzt, wodurch das Zwischenprodukt (Xb) Hydroxymethyl(thio)harnstoff erhalten wird,

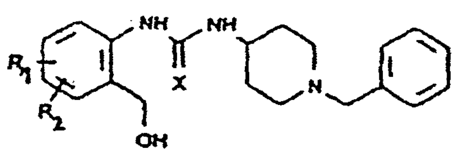 Xb

welches anschließend durch Filtration gewonnen und direkt durch Erhitzen auf 20 bis 70° in konzentrierter Salzsäure zyklisiert werden kann, wodurch die Verbindung der Formel XIb erhalten wird,

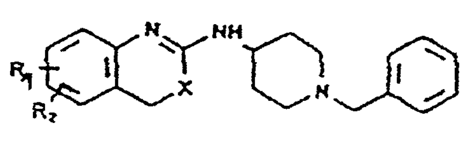 XIb

welche durch ein Halogenid ($R_5$-Br oder $R_5$-I) oder ein Sulfat $(R_5)_2SO_4$ in Gegenwart von Alkalicarbonat oder Natriumhydrid in DMF als Lösungsmittel alkyliert werden kann, wodurch die Verbindung der Formel (IVb) erhalten wird,

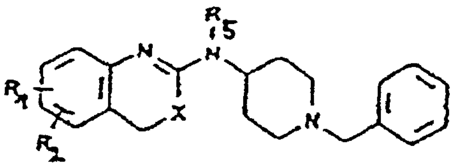

IVb

deren N-Debenzylierung in Gegenwart von α-Chlorethylchlorformiat in Methylenchlorid und anschließende Hydrolyse im Methanolrückfluss das Zwischenprodukt der Formel (V) ergibt,

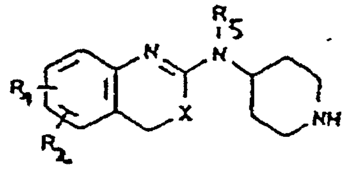

V

welches durch N-Alkylierung mit einem Halogenid der Formel (VI), worin Z ein Brom-, Chlor- oder Iodatom darstellt,

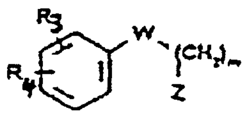

VI

in DMF in Gegenwart von $K_2CO_3$/KI 98/02 oder einem Glycidether der Formel (VII) in Methanol die Verbindung der allgemeinen Formel Ib ergibt.

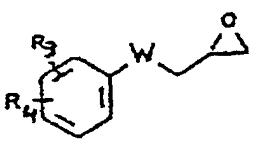

VII

**5.** Verfahren zur Herstellung der chemischen Verbindungen der Formel I gemäß den Ansprüchen 1 und 2, worin $R_5$ von H verschieden ist, **dadurch gekennzeichnet, dass** man eine Verbindung der Formel I, worin $R_5$ = H ist, mit einem (Aryl)alkylhalogenid $R_5$-Z, wobei $R_5$ die in Anspruch 1 definierte und Z die in Anspruch 4 definierte Bedeutung haben, oder einem Alkylsulfat $(R_5)_2SO_4$, wobei $R_5$ die in Anspruch 1 definierte Bedeutung hat, in Gegenwart einer Base, wie NaH oder $Na_2CO_3$, umsetzt, wodurch eine Verbindung der Formel I, worin $R_5$ ungleich H ist und die in Anspruch 1 definierte Bedeutung hat, erhalten wird.

**6.** Verbindungen gemäß einem der Ansprüche 1 und 2 als Arzneimittel.

**7.** Pharmazeutische Zusammensetzung, **dadurch gekennzeichnet, dass** sie als Wirkstoff wenigstens eine Verbindung gemäß einem der Ansprüche 1, 2 oder 6 in Verbindung mit einem inerten pharmazeutischen Träger oder anderen pharmazeutisch geeigneten Arzneistoffträgern enthält, wobei diese mit einem anderen Arzneimittel verbunden sein kann.

**8.** Pharmazeutische Zusammensetzung gemäß Anspruch 7 zur Verwendung bei der prophylaktischen Behandlung von myokardischer Ischämie, wie den chronischen stabilen Angina-Erkrankungen, den instabilen Angina-Erkrankungen und der Prinzmetal-Angina, stiller Ischämie, Reinfarkt, Reocclusio und Restenose.

**9.** Pharmazeutische Zusammensetzung gemäß Anspruch 7 zur Behandlung von zerebraler Ischämie, dem zerebralen vaskulären Unfall, dem vorübergehenden ischämischen Anfall und neurodegenerativen Erkrankungen.

**10.** Pharmazeutische Zusammensetzung gemäß Anspruch 7, **dadurch gekennzeichnet, dass** sie die Vorbeugung vor und Behandlung von Atherosklerose ermöglicht.

## Claims

**1.** N-Heterocyclyl-l-aryloxyalkyl-4-piperidinamines of formula I

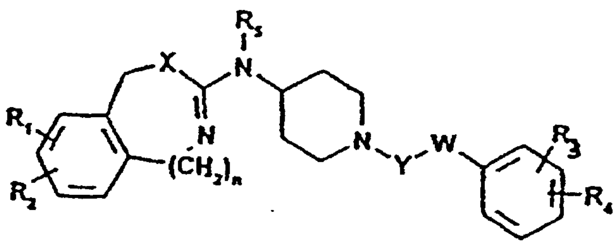

I

in which the substituents are defined as follows:

$R_1$ to $R_4$, which may be identical or different, represent:

- a hydrogen,
- a branched or unbranched alkyl containing from 1 to 4 carbon atoms,
- a branched or unbranched alkyloxy containing from 1 to 4 carbon atoms,
- a halogen group,
- a nitro group,
- a hydroxyl group,
- a trifluoromethyl or trifluoromethoxy group,

R5 represents:

- a hydrogen,
- a branched or unbranched alkyl containing from 1 to 6 carbon atoms,
- a branched or unbranched phenylalkyl containing from 7 to 12 carbon atoms, which can be substituted on the aromatic with one or two radicals defined as $R_1$,

W and X represent:

- an oxygen or a sulphur,

Y represents:

- $(CH_2)_m$ where m is an integer between 2 and 6 or
- the radical $-CH_2-CH(OH)-CH_2-$,

n can take the values 0 or 1,
when the compounds of formula I contain an asymmetric carbon, racemic mixtures thereof, various pure enantiomers thereof, or mixtures thereof, as well as the therapeutically acceptable inorganic or organic salts of the compounds of formula I and possible hydrates thereof.

**2.** Compound according to Claim 1, **characterized in that** it is chosen from the following compounds:

- N-methyl-N-[1-[4-(4-fluorophenoxy)butyl]piperid-4-yl]-4H-3,1-benzothiazin-2-amine hydrogen maleate,
- N-methyl-N-[1-[3-(4-fluorophenoxy)propyl]piperid-4-yl]-4H-3,1-benzothiazin-2-amine hydrogen maleate,
- N-methyl-N-[1-[2-(4-fluorophenoxy)ethyl]piperid-4-yl]-4H-3,1-benzothiazin-2-amine hydrogen maleate,
- N-[1-[4-(4-fluorophenoxy)butyl]piperid-4-yl]-4H-3,1-benzothiazin-2-amine hydrogen maleate,
- N-[1-[3-(4-fluorophenoxy)propyl]piperid-4-yl]-4H-3,1-benzothiazin-2-amine hydrogen maleate monohydrate,

- N-ethyl-N-[1-[3-(4-fluorophenoxy)propyl]piperid-4-yl]-4H-3,1-benzothiazin-2-amine hydrogen fumarate,
- N-benzyl-N-[1-[3-(4-fluorophenoxy)propyl]piperid-4-yl]-4H-3,1-benzothiazin-2-amine hydrogen maleate,
- 6-fluoro-N-methyl-N-[1-[4-(4-fluorophenoxy)butyl]-piperid-4-yl]-4H-3,1-benzothiazin-2-amine hydrogen maleate,
- 6-chloro-N-methyl-N-[1-[3-(4-fluorophenoxy)propyl]-piperid-4-yl]-4H-3,1-benzothiazin-2-amine hydrogen maleate,
- 6-methoxy-N-methyl-N-[1-[3-(4-fluorophenoxy)propyl]-piperid-4-yl]-4H-3,1-benzothiazin-2-amine dihydrogen maleate,
- N-methyl-N-[1-[4-(3,4-difluorophenoxy)butyl]piperid-4-yl]-4H-3,1-benzothiazin-2-amine hydrogen maleate,
- N-[1-[4-(3,4-difluorophenoxy)butyl]piperid-4-yl]-4H-3,1-benzothiazin-2-amine hydrogen maleate,
- N-[1-[4-(4-chlorophenoxy)butyl]piperid-4-yl]-4H-3,1-benzothiazin-2-amine hydrogen maleate,
- N-[1-[2-hydroxy-3-(4-fluorophenoxy)propyl]piperid-4-yl]-4H-3,1-benzothiazin-2-amine dihydrochloride hemihydrate,
- N-[1-[2-hydroxy-3-(4-fluorophenoxy)propyl]piperid-4-yl]-4H-3,1-benzothiazin-2-amine,
- N-methyl-N-[1-[2-hydroxy-3-(4-fluorophenoxy)propyl]-piperid-4-yl]-4H-3,1-benzothiazin-2-amine dihydrochloride,
- N-methyl-N-[1-(4-phenoxybutyl)piperid-4-yl]-4H-3,1-benzothiazin-2-amine hydrogen maleate,
- N-[1-[4-(4-fluorophenoxy)butyl]piperid-4-yl]-4H-3,1-benzothiazin-2-amine,
- 6,7-dimethoxy-N-[1-[3-(3,4-difluorophenoxy)propyl]-piperid-4-yl]-4H-3,1-benzothiazin-2-amine dihydrogen maleate,
- 6,7-dimethoxy-N-methyl-N-[1-[3-(3,4-difluorophenoxy)-propyl]piperid-4-yl]-4H-3,1-benzothiazin-2-amine dihydrogen maleate,
- 6-methyl-N-[1-[3-(3,4-difluorophenoxy)propyl]piperid-4-yl-3,1-benzothiazin-2-amine hemifumarate,
- N-methyl-N-[1-[4-(4-methylphenoxy)butyl]piperid-4-yl]-4H-3,1-benzothiazin-2-amine hydrogen maleate,
- N-methyl-N-[1-[4-(4-methoxyphenoxy)butyl]piperid-4-yl]-4H-3,1-benzothiazin-2-amine hydrogen maleate,
- N-[1-[2-hydroxy-3-(3,4-difluorophenoxy)propyl]-piperid-4-yl]-4H-3,1-benzothiazin-2-amine dihydrochloride,
- N-[1-[2-hydroxy-3-(3,4-difluorophenoxy)propyl]piperid-4-yl]-4H-3,1-benzothiazin-2-amine,
- N-methyl-N-[1-[4-(4-fluorophenoxy)butyl]piperid-4-yl]-1,5-dihydro-2,4-benzothiazepin-3-amine dihydrogen fumarate hydrate,
- N-methyl-N-[1-[4-(3,4-difluorophenoxy)butyl]piperid-4-yl] -1,5-dihydro-2,4-benzothiazepin-3-amine dihydrogen fumarate hydrate,
- 6,7-dimethoxy-N-[1-[3-(4-fluorophenoxy)propyl]piperid-4-yl]-4H-3,1-benzothiazin-2-amine hemifumarate,
- N-[1-[4-(4-fluorophenoxy)butyl]piperid-4-yl]-4H-3,1-benzoxazin-2-amine dihydrochloride,
- 6,7-dimethoxy-N-[1-[4-(4-fluorophenoxy)butyl]piperid-4-yl]-4H-3,1-benzothiazin-2-amine hemifumarate,
- 6-methyl-N-[1-[4-(4-fluorophenoxy)butyl]piperid-4-yl]-4H-3,1-benzothiazin-2-amine dihydrochloride hemihydrate,
- N-6-dimethyl-N-[1-[4-(4-fluorophenoxy)butyl]piperid-4-yl]-4H-3,1-benzothiazin-2-amine hydrogen maleate,
- (dl)-N-methyl-N-[1-[2-hydroxy-3-(3,4-difluorophenoxy)propyl]piperid-4-yl]-4H-3,1-benzothiazin-3-amine dihydrochloride,
- (S)-N-methyl-N-[1-[2-hydroxy-3-(3,4-difluorophenoxy)-propyl]piperid-4-yl]-4H-3,1-benzothiazin-2-amine hydrogen maleate,
- N-methyl-N-[1-[4-fluorophenylthio)butyl]piperid-4-yl]-4H-3,1-benzothiazin-2-amine hydrogen maleate,
- N-methyl-N-[1-[5-(4-fluorophenoxy)pentyl]piperid-4-yl]-4H-3,1-benzothiazin-2-amine hydrogen fumarate,
- N-methyl-N-[1-[3-(4-fluorophenoxy)propyl]piperid-4-yl]-4H-3,1-benzothiazin-2-amine hydrogen fumarate,
- N-methyl-N-[1-[6-(4-fluorophenoxy)hexyl]piperid-4-yl]-4H-3,1-benzothiazin-2-amine hydrogen fumarate,
- N-methyl-N-[1-[4-(3,4-dimethoxyphenoxy)butyl]piperid-, 4-yl]-4H-3,1-benzothiazin-2-amine hydrogen fumarate,
- N-methyl-N-[1-[4-(2-methoxyphenoxy)butyl]piperid-4-yl]-4H-3,1-benzothiazin-2-amine hydrogen fumarate,
- N-methyl-N-[1-[4-(2,3-dimethoxyphenoxy)butyl]piperid-4-yl]-4H-3,1-benzothiazin-2-amine hydrogen fumarate,
- N-methyl-N-[1-[4-(3,5-dimethoxyphenoxy)butyl]piperid-4-yl]-4H-3,1-benzothiazin-2-amine hydrogen fumarate,
- N-methyl-N-[1-[4-(2,6-dimethoxyphenoxy)butyl]piperid-4-yl]-4H-3,1-benzothiazin-2-amine hydrogen fumarate,
- N-methyl-N-[1-[4-(2,6-difluorophenoxy)butyl]piperid-4-yl]-4H-3,1-benzothiazin-2-amine hydrogen fumarate,
- N-methyl-N-[1-[5-(3,4-difluorophenoxy)pentyl]piperid-4-yl]-4H-3,1-benzothiazin-2-amine hydrogen fumarate,
- N-methyl-N-[1-[4-(3,5-difluorophenoxy)butyl]piperid-4-yl]-4H-3,1-benzothiazin-2-amine hydrogen fumarate,
- N-methyl-N-[1-[4-(2,4-difluorophenoxy)butyl]piperid-4-yl]-4H-3,1-benzothiazin-2-amine hydrogen fumarate,

- N-methyl-N-[1-[4-(2,5-difluorophenoxy)butyl]piperid-4-yl]-4H-3,1-benzothiazin-2-amine hydrogen fumarate,
- N-methyl-N-[1-[4-(3-fluoro-4-chlorophenoxy)butyl]-piperid-4-yl]-4H-3,1-benzothiazin-2-amine hydrogen fumarate,
- N-methyl-N-[1-[4-(2,3-difluorophenoxy)butyl]piperid-4-yl]-4H-3,1-benzothiazin-2-amine hydrogen fumarate,
- N-methyl-N-[1-[4-(3-chloro-4-fluorophenoxy)butyl]-piperid-4-yl]-4H-3,1-benzothiazin-2-amine hydrogen fumarate,
- N-methyl-N-[1-[4-(3-fluorophenoxy)butyl]piperid-4-yl]-4H-3,1-benzothiazin-2-amine hydrogen fumarate,
- N-methyl-N-[1-[4-(3-methoxyphenoxy)butyl]piperid-4-yl]-4H-3,1-benzothiazin-2-amine hydrogen fumarate,
- N-methyl-N-[1-[4-(2-fluorophenoxy)butyl]piperid-4-yl]-4H-3,1-benzothiazin-2-amine hydrogen fumarate,
- N-methyl-N-[1-[4-(2-methoxy-4-chlorophenoxy)butyl]-piperid-4-yl]-4H-3,1-benzothiazin-2-amine hydrogen fumarate,
- N-methyl-N-[1-[4-(4-chlorophenoxy)butyl]piperid-4-yl]-4H-3,1-benzothiazin-2-amine hydrogen fumarate,
- N-methyl-N- [1- [5-(4-methoxyphenoxy)pentyl]piperid-4-yl]-4H-3,1-benzothiazin-2-amine hydrogen fumarate,
- N-[1-[5-(3,4-difluorophenoxy)pentyl]piperid-4-yl]-4H-3,1-benzothiazin-2-amine dihydrogen fumarate,
- N-[1-[5-(4-fluorophenoxy)pentyl]piperid-4-yl]-4H-3,1-benzothiazin-2-amine dihydrogen fumarate,
- (R)-N-methyl-N-[1-[2-hydroxy-3-(3,4-difluorophenoxy)-propyl]piperid-4-yl]-4H-3,1-benzothiazin-2-amine hydrogen fumarate,
- N-methyl-N-[1-[5-(2-methoxyphenoxy)pentyl]piperid-4-yl]-4H-3,1-benzothiazin-2-amine hydrogen fumarate,
- N-methyl-N-[1-[2-(2-methoxyphenoxy)ethyl]piperid-4-yl]-4H-3,1-benzothiazin-2-amine hydrogen fumarate,

**3.** Process for the preparation of the chemical compounds of formula Ia, i.e. the chemical compounds of formula I according to Claim 1 or 2 for which
n = 0, $R_5$ = H and Y = $(CH_2)_m$,

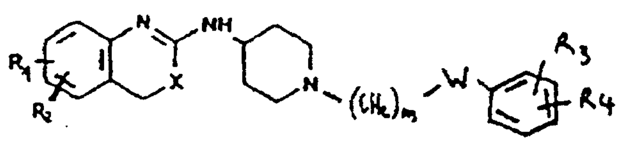

Ia

in which $R_1$, $R_2$, $R_3$, $R_4$, m, X and W are as defined in Claim 1,
**characterized in that** an ortho-aminobenzyl alcohol of formula VIII

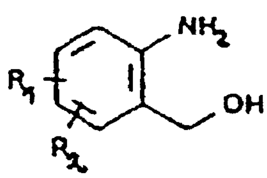

VIII

is reacted with a 4-piperidyl iso(thio)cyanate of formula IXa

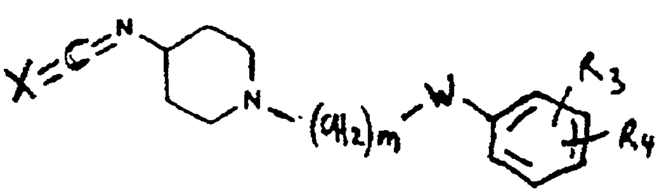

IXa

in THF or dioxane as solvent, at a temperature of between 20 and 80°, to give the intermediate hydroxymethyl (thio)urea Xa

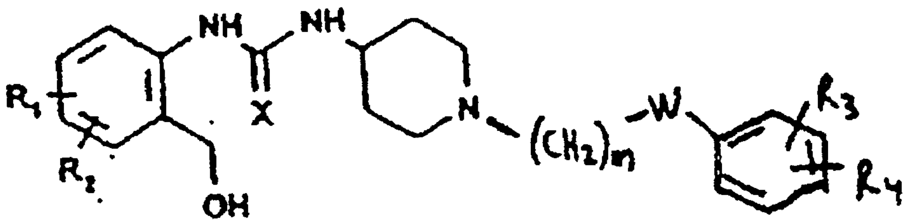

Xa

Xa being recovered by filtration and cyclized directly in concentrated hydrochloric acid at a temperature of between 20 and 80° to give the compound of general formula Ia.

**4.** Process for the preparation of the chemical compounds of formula Ib

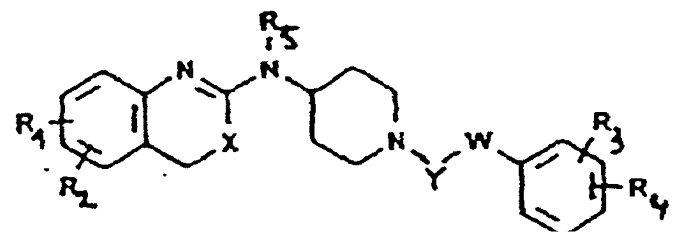

Ib

i.e. of the chemical compounds of formula I according to Claims 1 and 2, for which $R_1$ to $R_5$, W, X and Y are as defined in Claim 1 and n=0,
**characterized in that** an ortho-amino alcohol of formula (VIII) is reacted with an iso(thiocyanate) of formula (IXb)

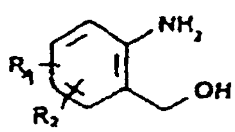

VIII

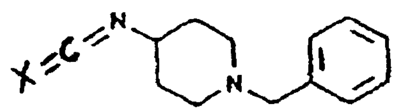

IXb

in THF or dioxane as solvent, at a temperature between 20 and 80° to give an intermediate hydroxymethyl-(thio) urea (Xb)

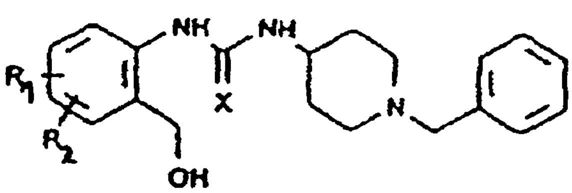

Xb

which product Xb can then be recovered by filtration and is cyclized directly by heating between 20 and 70° in concentrated hydrochloric acid to give the compound of formula XIb

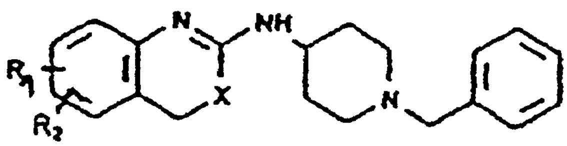

XIb

which can be alkylated with a halide ($R_5$-Br or $R_5$-I) or a sulphate $(R_5)_2SO_4$ in the presence of an alkali metal carbonate or sodium hydride in DMF as solvent, to give the compound of formula (IVb),

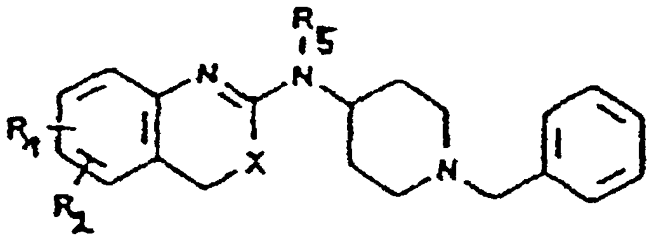

IVb

N-debenzylation of which in the presence of α-chloroethyl chloroformate in methylene chloride followed by hydrolysis in refluxing methanol leads to the intermediate of formula (V)

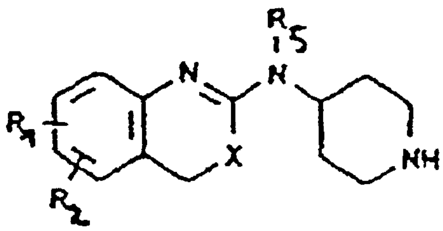

V

which compound, on final N-alkylation with a halide of formula (VI) in which Z represents a bromine, chlorine or iodine atom,

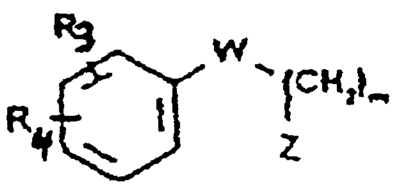

VI

in DMF in the presence of 98/02 $K_2CO_3$/KI or a glycidyl ether of formula (VII) in methanol, gives the compound of general formula Ib

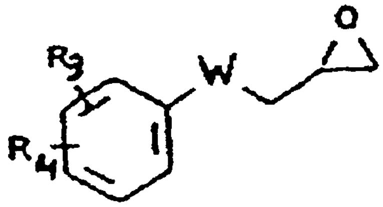

VII

**5.** Process for the preparation of the chemical compounds of formula I according to Claims 1 and 2 where $R_5$ is other than H, **characterized in that** a compound of formula I where $R_5$ = H is reacted with an (aryl)alkyl halide $R_5$-Z, $R_5$ being as defined in Claim 1, and Z as defined in Claim 4, or an alkyl sulphate $(R_5)_2SO_4$, $R_5$ being as defined in Claim 1, in the presence of a base such as NaH or $Na_2CO_3$ to give a compound of formula I for which $R_5 \neq H$ is as defined in Claim 1.

**6.** The compounds defined according to either of Claims 1 and 2, as drugs.

**7.** Pharmaceutical composition, **characterized in that** it contains, as active principle, at least one compound according to one of Claims 1, 2 and 6 combined with an inert pharmaceutical support or other pharmaceutically acceptable vehicles and which may or may not be combined with another drug.

**8.** Pharmaceutical composition according to Claim 7, which is useful in the prophylactic treatment of myocardial ischaemia, such as attacks of chronic stable angina, unstable angina and Prinzmetal's angina, silent ischaemia, reinfarction, reocclusion and restenosis.

**9.** Pharmaceutical composition according to Claim 7, which is useful in cerebral ischaemia, strokes, transitory ischemic attacks and neurodegenerative diseases.

**10.** Pharmaceutical composition according to Claim 7, **characterized in that** it allows atherosclerosis to be prevented and treated.